# EUROPEAN PATENT APPLICATION

(11) **EP 4 671 239 A1**
(43) Date of publication of application: **31.12.2025**
(21) Application number: 24759739.6
(22) Date of filing: 22.02.2024
(51) Int. Cl.: C07D 401/14, A61K 31/496, A61K 31/506, A61P 35/00

(54) **ARYL SUBSTITUENT-CONTAINING DEGRADATION AGENT FOR CDK12/13, PREPARATION METHOD THEREFOR, AND PHARMACEUTICAL COMPOSITION AND USE THEREOF**

(30) Priority: 24.02.2023 CN 202310175306; 24.11.2023 CN 202311588550
(71) Applicant: Shanghai Institute of Organic Chemistry, Chinese Academy of Sciences, Shanghai 200032 (CN); The Regents of The University of Michigan, Ann Arbor, Michigan 48109 (US); Livzon Pharmaceutical Group Inc., Guangdong 519090 (CN)
(72) Inventor: DING, Ke, Shanghai 200032 (CN); CHINNAIYAN, Arul M., Ann Arbor Michigan 48109 (US); YANG, Jianzhang, Shanghai 200032 (CN); CHANG, Yu, Ann Arbor Michigan 48109 (US); ZHOU, Licheng, Shanghai 200032 (CN); ZHOU, Kaijie, Shanghai 200032 (CN); WANG, George Xiaoju, Ann Arbor Michigan 48109 (US); WANG, Zhen, Shanghai 200032 (CN); HUANG, Weixue, Shanghai 200032 (CN); ZHOU, Fengtao, Shanghai 200032 (CN)
(74) Representative: J A Kemp LLP
(86) International application number: PCT/CN2024/078146
(87) International publication number: WO 2024/175065

(57) **Abstract**

The present invention relates to an aryl substituent-containing degradation agent for cyclin-dependent kinase 12/13 (CDK12/13), a preparation method therefor, and a pharmaceutical composition and use thereof. The degradation agent for CDK12/13 of the present invention has a structure represented by formula (I). The compound can be used as a protein kinase degradation agent, and can effectively and highly selectively degrade a CDK12/13 protein and inhibit proliferation, migration, and invasion of various tumor cells.

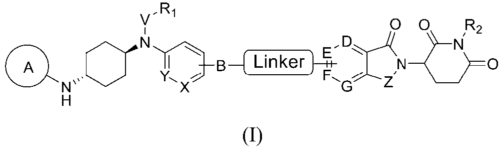

## Description

### TECHNICAL FIELD

The present invention belongs to the field of chemical medicine, and in particular relates to a degradation agent for cyclin-dependent kinase 12/13 (CDK12/13) and a pharmaceutical composition and use thereof.

### BACKGROUND ART

Protein kinases are key regulators of cellular function and constitute one of the largest and most functionally diverse gene families. Protein kinases direct the activity, localization and overall function of many proteins through the phosphorylation of substrate proteins, and are involved in almost all cellular activities. Abnormalities in the expression, activation, and localization of protein kinases are closely related to the occurrence and development of a variety of diseases, and are important driving factors for a variety of diseases such as tumors and inflammation. As of December 31, 2020, the FDA has approved a total of 62 kinase inhibitor drugs for marketing. Studies have shown that the non-kinase functions of proteins play an important role in diseases such as tumors. The literature has reported the non-kinase functions of kinases such as CDK6, FAK, EGFR, PIPK3, PDK1, BRAF, CRAF, CHK2, ZAP70, AKT, and Aurora A. Therefore, simply inhibiting the activity of kinases cannot completely inhibit the non-kinase functions of kinases, resulting in potential adverse effects such as low efficacy and drug resistance. Therefore, the development of protein degradation agents is expected to comprehensively inhibit the enzymatic activity and non-kinase functions of kinases and exert a potent therapeutic effect. Protein degradation agents based on the PROTAC principle are currently the most successful and mature protein degradation strategy, and have been successfully used in the development of degradation agents for multiple kinases and other targets.

PROTAC refers to a class of small molecule compounds that can specifically recognize and induce degradation of target proteins; the molecular structure consists of three parts: target protein recognition ligand, Linker and E3 recognition ligand. The advantage of PROTAC is that it not only effectively inhibits the kinase activity of the target protein, but also can quickly degrade and eliminate the target protein. Theoretically, only a catalytic amount of drugs is needed to degrade almost all proteins (including membrane proteins) in cells, so it has high safety, drug resistance and broad application prospects. At present, degradation agents for target proteins such as ERR, ABL, BET, CDK4/6 have been successfully developed. The results show that they can not only treat the proliferation of gene-driven tumors, but also overcome the drug resistance of inhibitors. In 2018, Pfizer announced an investment of US$830 million in the research and development of protein degrader drugs based on PROTAC technology. In March 2019, WuXi AppTec's partner Arinas announced that its protein degrader ARV-110 targeting the androgen receptor had entered clinical research; this is the world's first protein degrader to enter the clinical research stage.

CDK12/13 (cyclin-dependent kinase 12/13) is a member of a cyclin-dependent kinase family (CDKs) of serine/threonine protein kinases, and forms a complex with Cyclin K to realize biological functions. CDK12/13 comprises 1490, 1512 amino acids, respectively, and shares 46% homology. The kinase domain consists of 300 amino acids with as high as 92% homology. The CDK12/13 forms a complex together with the Cyclin K by phosphorylating a C-terminal domain (CTD) of RNA polymerase II (RNA Pol II). The CTD is a highly repeated sequence composed of seven amino acids (YSPTSPS). A human CTD comprises a 52-repeat unit. The CDK12/13 mainly phosphorylates Ser2 to regulate transcription and post-transcriptional mRNA processing. Genetic studies show that the CDK12 promotes the transcription of a full-length gene product by inhibiting the cleavage of an intronic polyadenylation site, and many homologous recombination repair genes (such as: BRCA1/2, ATM, ATR, FANCD2, and FANCI) comprise more intronic polyadenylation sites. Therefore, the expression of these genes is more sensitive to the loss or inhibition of the CDK12. Compared to other transcribed CDKs, the CDK12/13 contains an additional N-terminal arginine/serine-rich (RS) motif, which is commonly found in proteins involved in pre-mRNA splicing. A motif of a proline-rich motif (PRIM) is also found at the N- and C-terminal, and is likely to serve as a binding site for SH3, WW, or a protein containing an actin-binding protein (profilin) domain. These structures suggest that CDK12/13 may have non-kinase functions (such as protein-protein interaction) and play an important role in mRNA splicing and mRNA 3' end processing. Therefore, it is far from enough to use only CDK12/13 kinase inhibitors as probe tool molecules to study the biological functions of CDK12/13. The use of proteolysis targeting chimeras (PROTACs) to induce CDK12/13 degradation and simultaneously inhibit the kinase and non-kinase functions of CDK12/13 has great potential in studying the biological functions and disease treatment of CDK12/13, and has attracted increasing interest among scientists in recent years.

### SUMMARY OF THE INVENTION

Based on this, the present invention provides a class of trans-1,4-cyclohexanediamine compounds and use thereof as CDK12/13 degradation agents. Such compounds can effectively and highly selectively degrade CDK12/13 protein kinases and inhibit the proliferation, migration and invasion of various tumor cells.

In a first aspect of the present invention, provided is a compound having a structure of formula (I), or a pharmaceutically acceptable salt thereof, or a pharmaceutical composition thereof, or a prodrug molecule thereof: 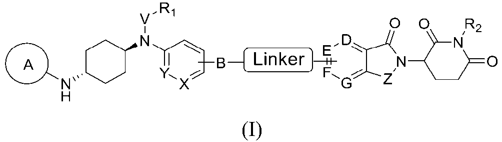 wherein Z is CH₂ or CO;
V is selected from the group consisting of: 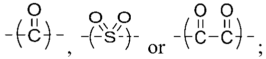
X and Y are each independently selected from the group consisting of: N, CH or CR₃; wherein R₃ is selected from the group consisting of: halogen, cyano, hydroxyl, amino, C₁-C₃ alkyl, halogenated C₁-C₃ alkyl, C₁-C₃ alkoxy, halogenated C₁-C₃ alkoxy, C₃-C₈ cycloalkyl or 3- to 8-membered heterocyclyl;
B is selected from the group consisting of: 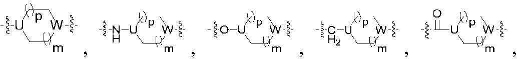 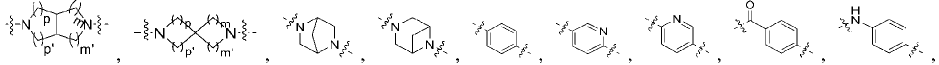 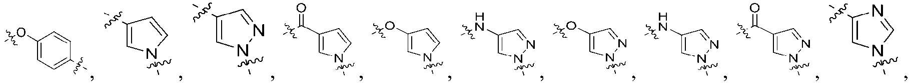 NH, O, CO, or CH₂; wherein each of U and W is independently selected from the group consisting of: N or CH; each of m, p, m' and p' is independently: 0, 1, 2 or 3;
A ring is selected from the group consisting of: 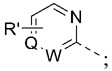
wherein Q and W are each independently selected from: CH or N;
R' is independently selected from: hydrogen, halogen, cyano, hydroxy, substituted hydroxy, amino, substituted amino, C₁-C₅ alkyl, halogenated C₁-C₅ alkyl, C₁-C₅ alkoxy, halogenated C₁-C₃ alkoxy, or C₃-C₈ cycloalkyl, and when Q and W are independently selected from CH, R' may be a substituent on Q or W;
D, E, F, and G are each independently selected from the group consisting of: CH, N, or CR₆; wherein R₆ is selected from the group consisting of: halogen, trifluoromethyl, hydroxy, cyano, amino, methyl, methoxy, or trifluoromethoxy;
R₁ is selected from the group consisting of: H, -NHR₇, -OR₇, or -(C(R₉)R₈)R₇;
wherein R₇ is -R₁₀, -CH₂R₁₀ or -(CH₂)₂R₁₀;
R₈ and R₉ are each independently selected from the group consisting of: hydrogen, halogen, cyano, methyl, halomethyl, methoxy, halomethoxy, ethyl, haloethyl, ethoxy, haloethoxy, hydroxy, amino, or 3- to 8-membered heterocyclic ring containing 1, 2 or 3 heteroatoms, and the heteroatoms are selected from O, S or N;
or R₈ and R₉, together with the C atom to which they are attached, form a 3- to 7-membered heterocyclic ring;
R₁₀ is selected from the group consisting of:
   1) cyano, C₁-C₃ alkyl, halogenated C₁-C₄ alkyl, C₁-C₄ alkoxy, C₃-C₁₀ cycloalkyl, substituted or unsubstituted 5-to 12-membered aromatic ring, or substituted or unsubstituted 3- to 12-membered heterocyclic ring; or
   2) 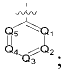
      wherein Q₁, Q₂, Q₃, Q₄ and Q₅ are each independently selected from: CH, N or CR₁₁;
      each R₁₁ is independently selected from the group consisting of: halogen, cyano, hydroxy, amino, nitro, C₁-C₃ alkyl, halogenated C₁-C₃ alkyl, C₁-C₄ alkoxy, halogenated C₁-C₄ alkoxy, or C₃-C₈ cycloalkyl;
      R₂ is selected from the group consisting of: H, C₁-C₃ alkyl, 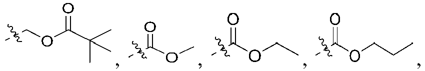 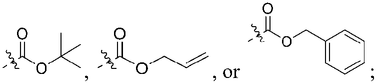
      when B is selected from the group consisting of: 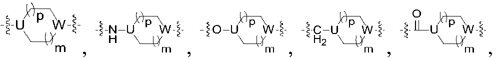 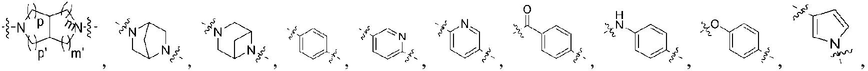 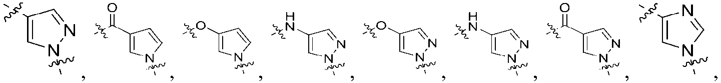 NH, O, CO, or CH₂; wherein each of U and W is independently selected from the group consisting of: N or CH; each of m, p, m' and p' is independently: 0, 1, 2 or 3;
      Linker is: 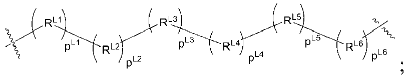
      wherein,
      R^{L1}, R^{L2}, R^{L3}, R^{L4} and R^{L5} are the same or different and are each independently selected from the substituted or unsubstituted groups in the group consisting of: a chemical bond, CH₂, CHD, CD₂, C=O, O, NH, SO, SO₂, P=O, NHCO, NHSO₂, OCH₂, OCH₂CH₂, CH₂OCH₂, NHCH₂, NMeCH₂, NHCH₂CH₂, NMeCH₂CH₂, CH₂NHCO, NHCOCH₂, 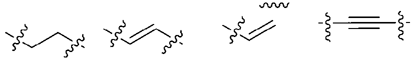 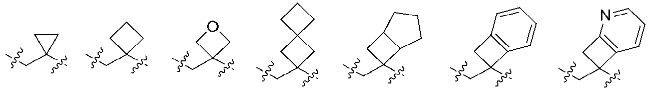 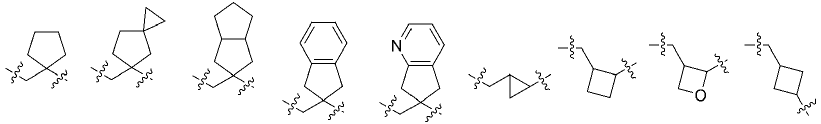 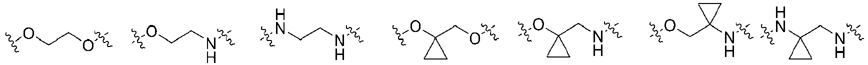 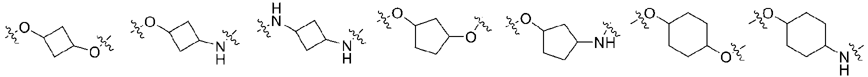 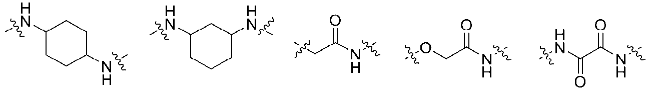
      R^{L6} is a ring and optionally selected from the following structure: 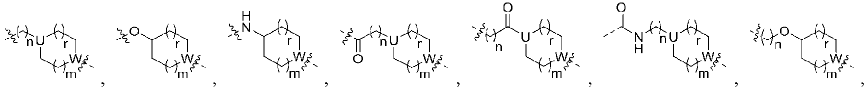 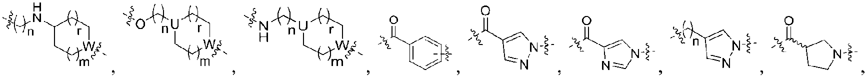 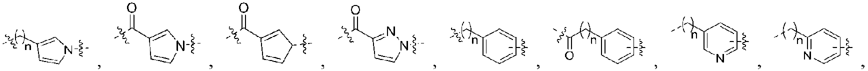 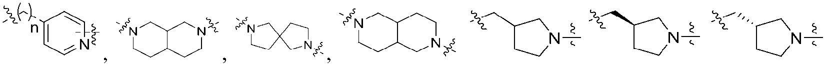 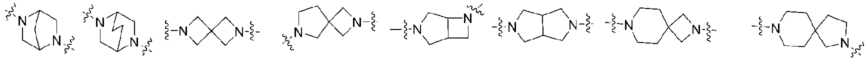 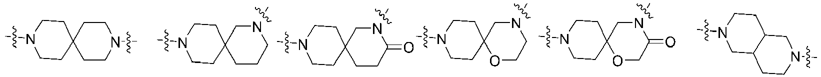 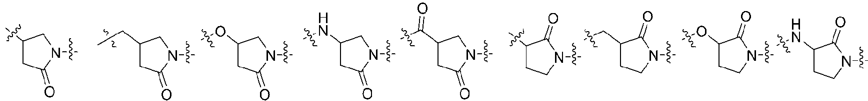 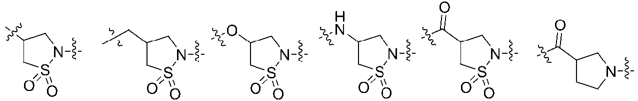 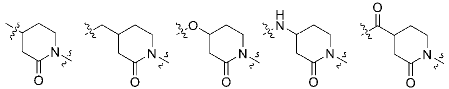 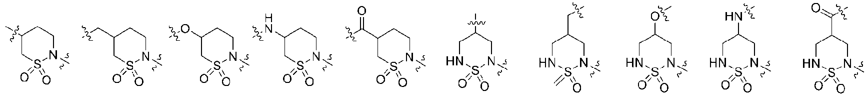 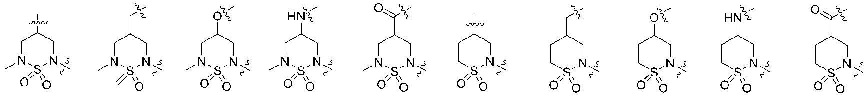 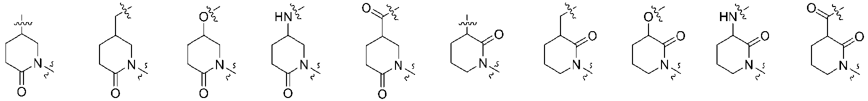 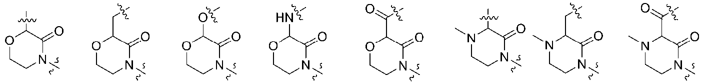 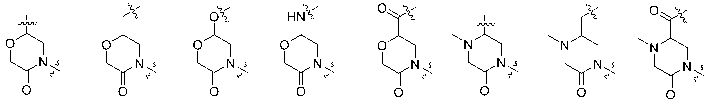 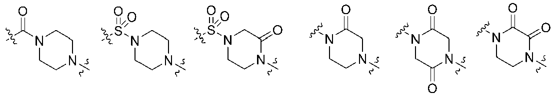 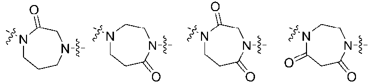 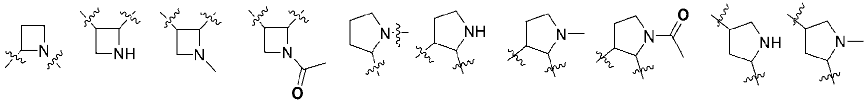 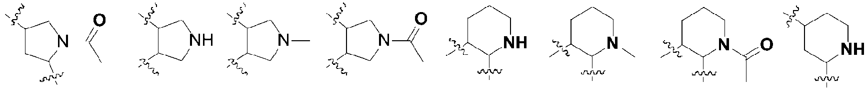 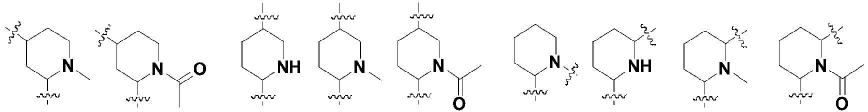 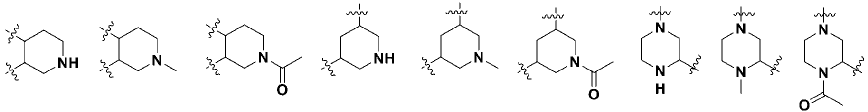 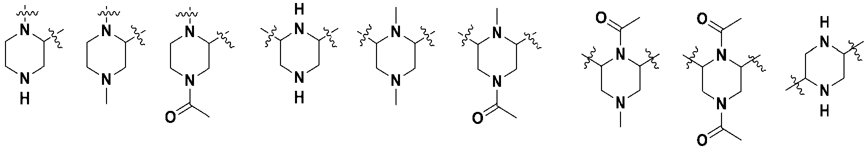 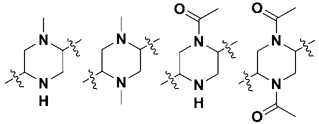 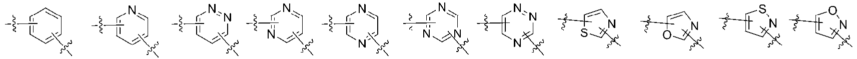 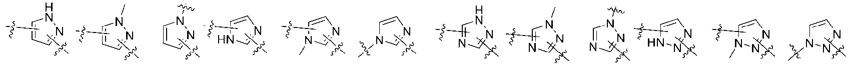 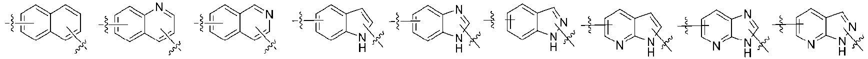 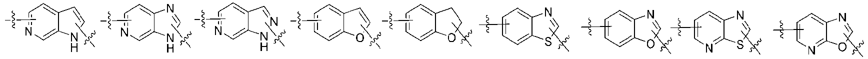 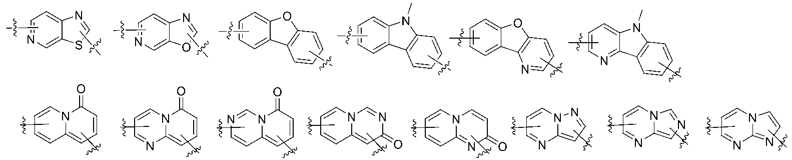 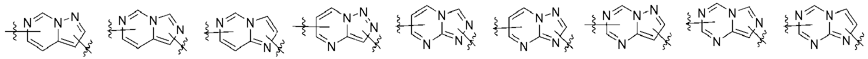 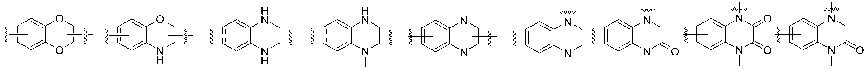 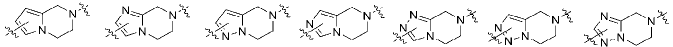 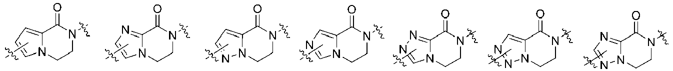 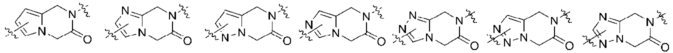
      wherein each n is independently 0, 1, 2, 3, 4, 5 or 6; each of r and m is independently 0, 1 or 2; each of U and W is independently selected from the group consisting of: N or CH
      p^{L1}, p^{L2}, p^{L3}, p^{L4}, p^{L5} and p^{L6} are independently selected from 0, 1, 2, 3, 4, 5 or 6.
      when B is 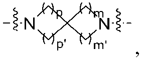 where each of m, p, m' and p' is independently: 0, 1, 2 or 3;
      Linker is: 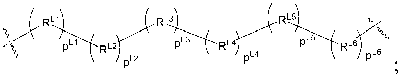
      wherein,
      R^{L1}, R^{L2}, R^{L3}, R^{L4}, R^{L5} and R^{L6} are the same or different and are each independently selected from the substituted or unsubstituted groups in the group consisting of: a chemical bond, CH₂, CHD, CD₂, C=O, O, NH, SO, SO₂, P=O, NHCO, NHSO₂, OCH₂, OCH₂CH₂, CH₂OCH₂, NHCH₂, NMeCH₂, NHCH₂CH₂, NMeCH₂CH₂, CH₂NHCO, NHCOCH₂, 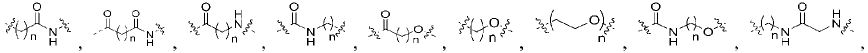 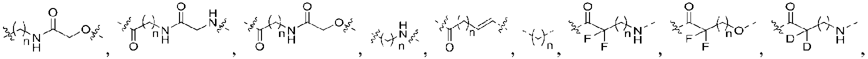 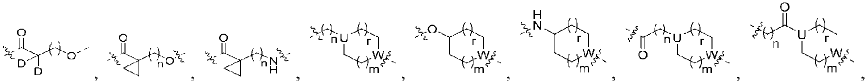 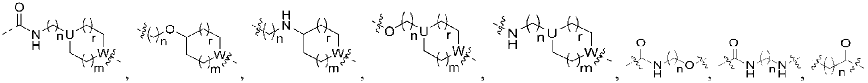 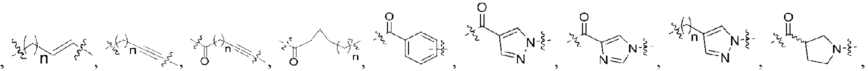 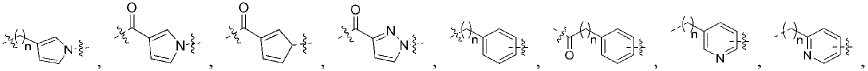 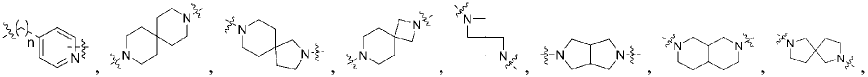 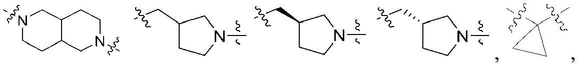 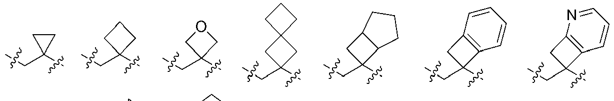 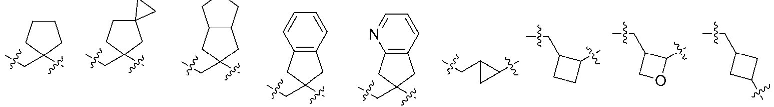 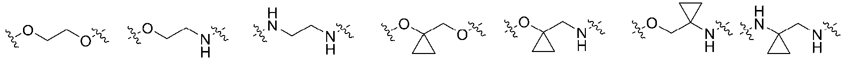 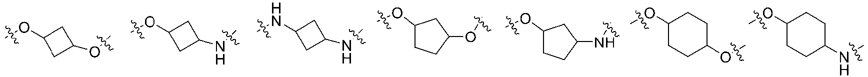 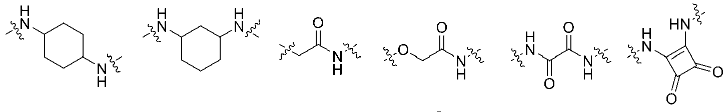 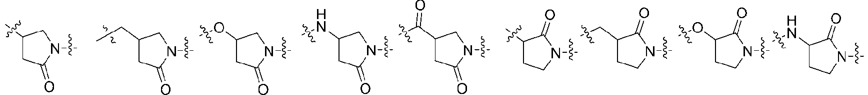 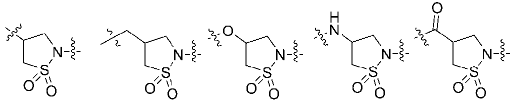 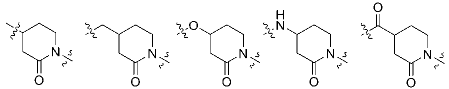 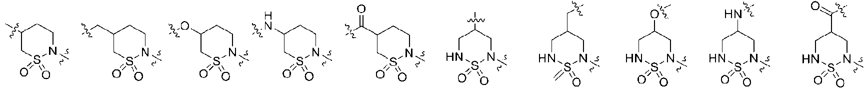 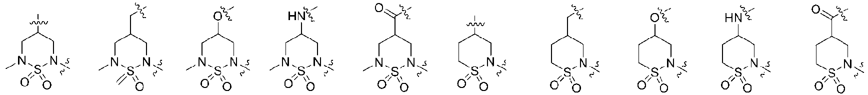 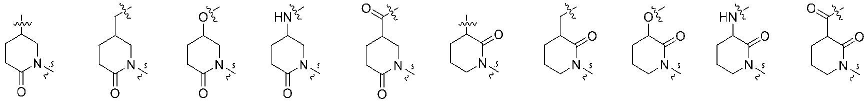 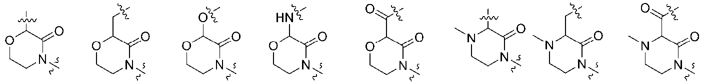 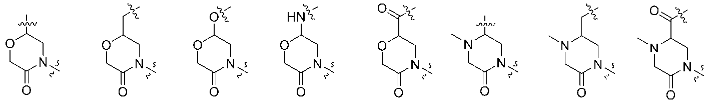 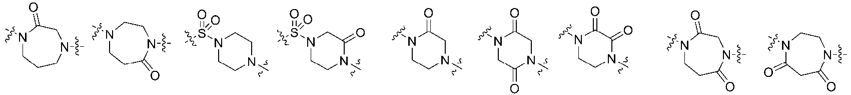 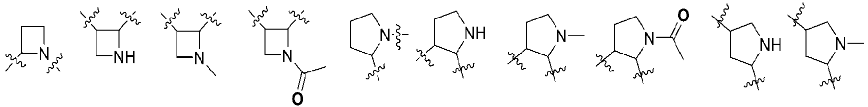 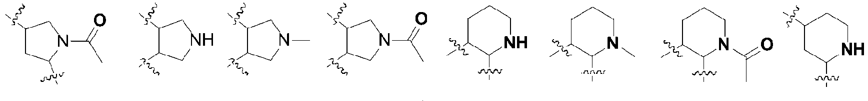 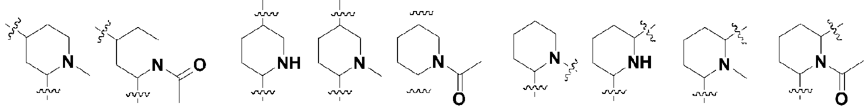 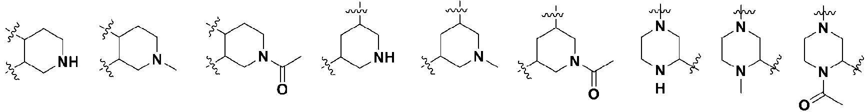 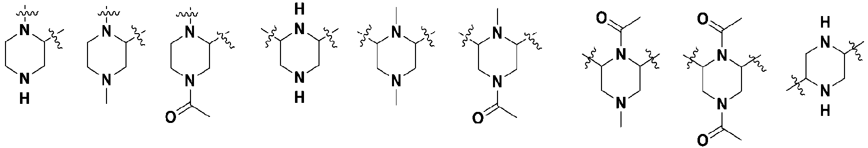 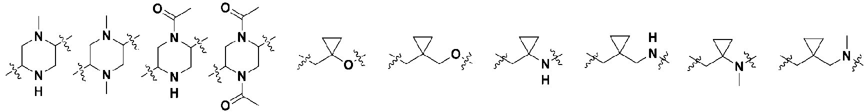 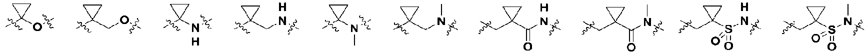 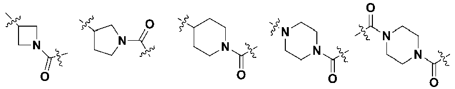 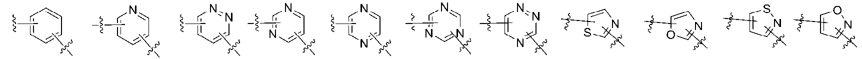 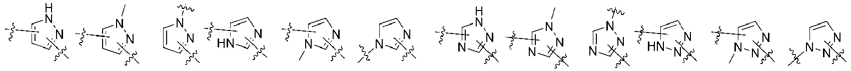 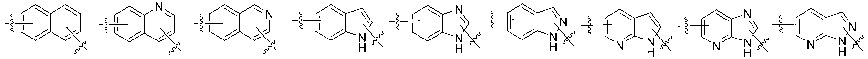 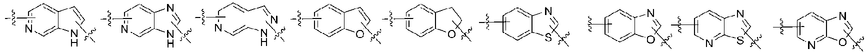 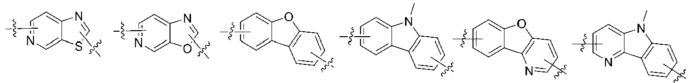 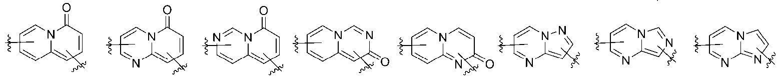 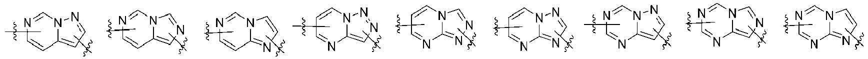 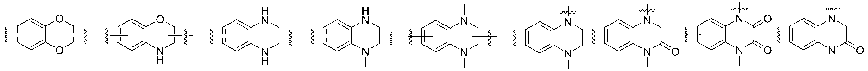 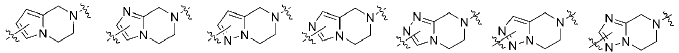 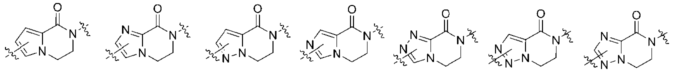 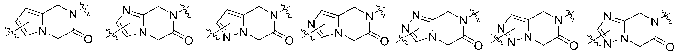
      wherein each n is independently 0, 1, 2, 3, 4, 5 or 6; each of r and m is independently 0, 1 or 2; each of U and W is independently selected from the group consisting of: N or CH;
      p^{L1}, p^{L2}, p^{L3}, p^{L4}, p^{L5} and p^{L6} are independently selected from 0, 1, 2, 3, 4, 5 or 6.

In some embodiments, V is selected from the group consisting of:

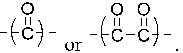

In some embodiments, X and Y are each independently selected from the group consisting of: N, CH or CR₃;
wherein R₃ is selected from the group consisting of: halogen, cyano, hydroxy, amino, C₁-C₃ alkyl, halogenated C₁-C₃ alkyl, C₁-C₃ alkoxy, or halogenated C₁-C₃ alkoxy.

In some embodiments, B is selected from the group consisting of: 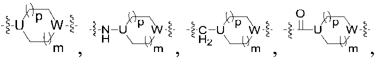 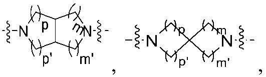 NH, O, or CO;
wherein each of U and W is independently selected from the group consisting of: N or CH; each of m, p, m' and p' is independently 0, 1, 2 or 3.

In some embodiments, A ring is selected from: 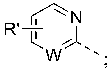
wherein W is selected from the group consisting of: CH or N.
R' is optionally selected from: hydrogen, halogen, cyano, hydroxy, amino, C₁-C₃ alkyl, halogenated C₁-C₃ alkyl, C₁-C₅ alkoxy, halogenated C₁-C₃ alkoxy, or C₃-C₈ cycloalkyl.

In some embodiments, the D, E, F and G are CH or CR₆; wherein R₆ is selected from the group consisting of: halogen.

In some embodiments, the R₁ is -NHR₇;
wherein R₇ is -CH₂R₁₀; R₁₀ is defined as described above.

In another preferred embodiment, the R₁₀ is selected from the group consisting of: cyano, C₁-C₅ alkyl, halogenated C₁-C₄ alkyl, C₁-C₄ alkoxy, C₃-C₁₀ cycloalkyl, 4- to 7-membered heterocyclyl, or C₆-C₁₀ aryl; wherein the aryl is substituted with one or more CR₁₁; R₁₁ is defined as described above.

In some embodiments,
when B is selected from the group consisting of: 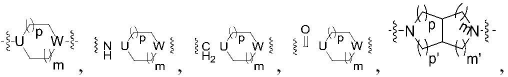 NH, O, or CO;
the Linker is selected from the group consisting of: 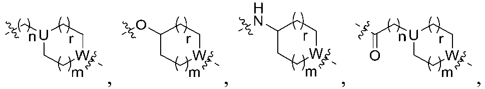 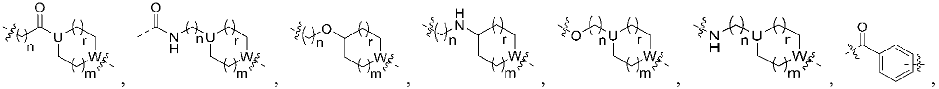 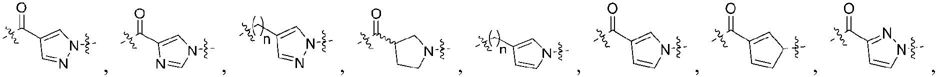 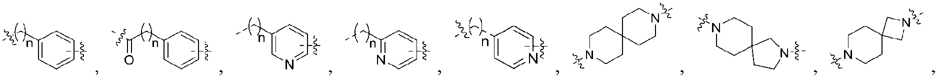 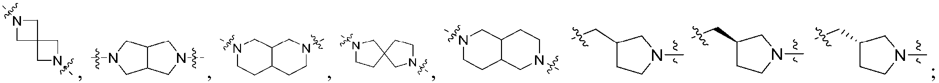
and when B is 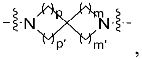 the Linker is selected from the group consisting of: a chemical bond, 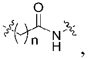 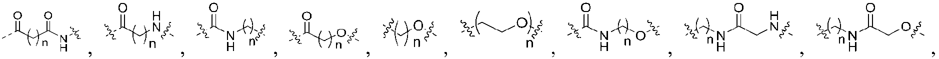 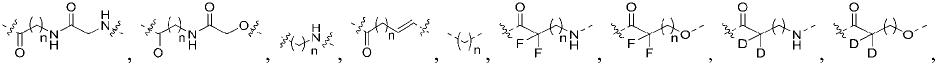 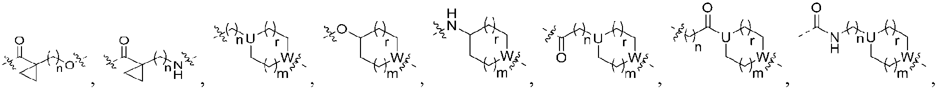 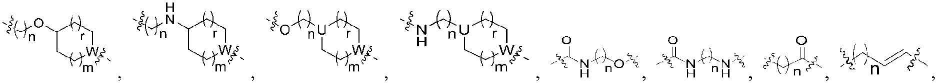 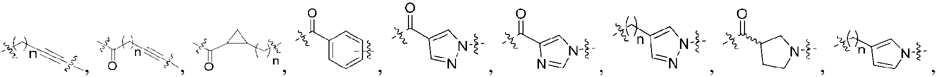 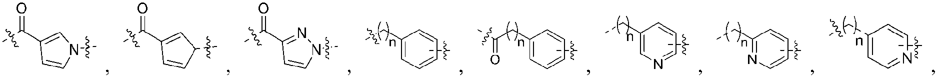 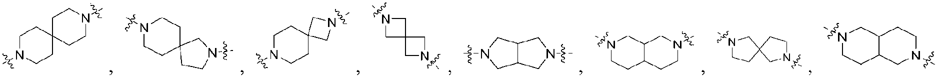 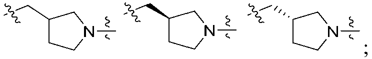 wherein each n is independently 0, 1, 2, 3, 4, 5 or 6; each of r and m is independently 0, 1 or 2; each of U and W is independently selected from the group consisting of: N or CH.

In some embodiments, the compound has a structure represented by formula (II): 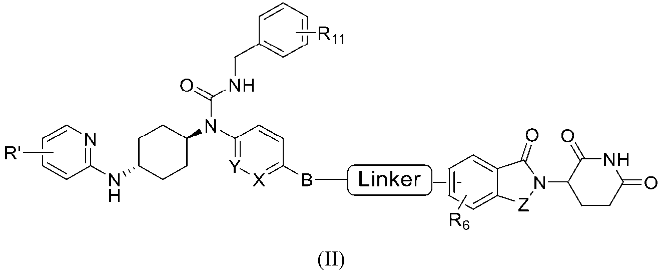
wherein B is selected from: 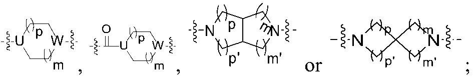 wherein each of U and W is independently selected from the group consisting of: N or CH; each of m, p, m' and p' is independently 0, 1, 2 or 3;
R' is optionally selected from: hydrogen, halogen, cyano, hydroxy or amino;
R₁₁ and R₆ are optionally selected from: hydrogen or halogen;
X and Y are independently selected from: CH, N or CR₃; wherein R₃ is selected from: halogen, cyano, hydroxy or amino;
Z is optionally selected from: CH₂ or CO;

In some embodiments, the compound is selected from the group consisting of:

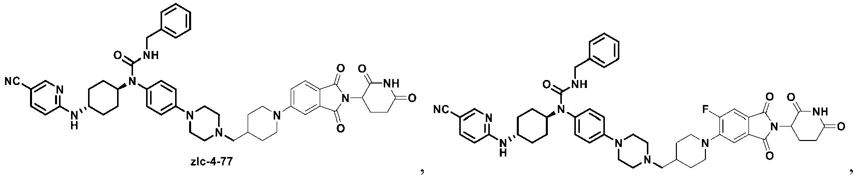

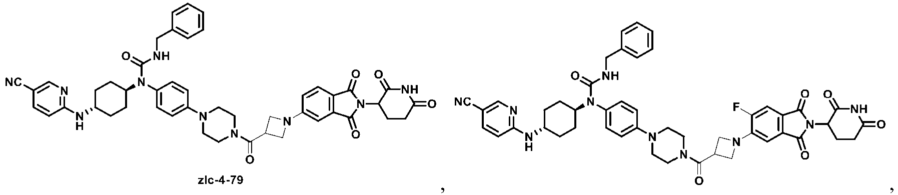

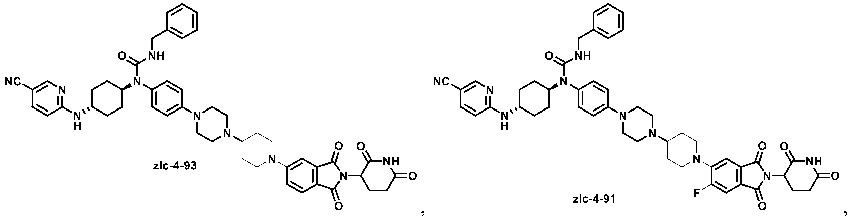

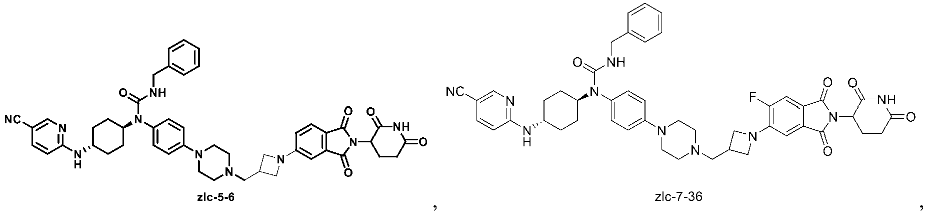

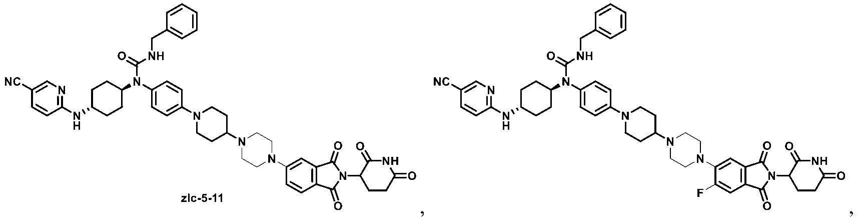

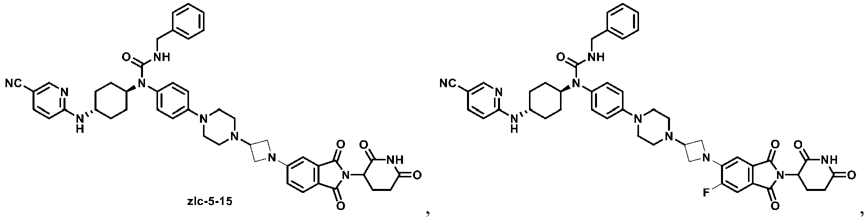

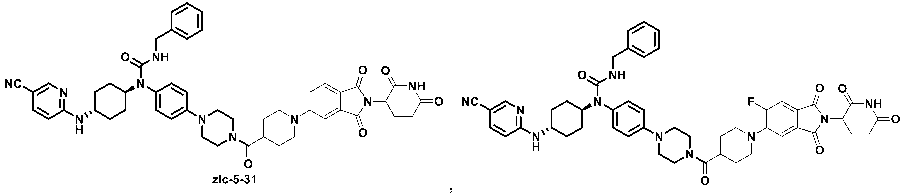

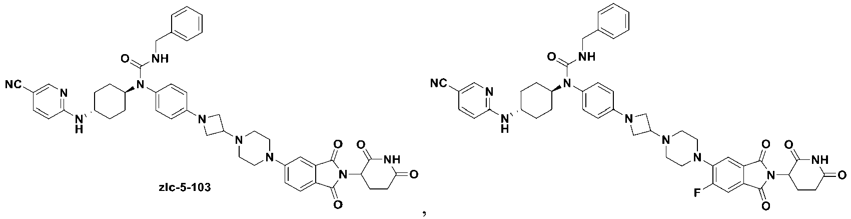

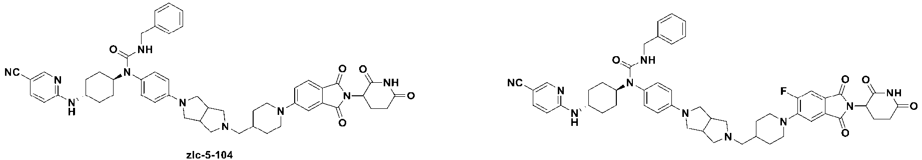

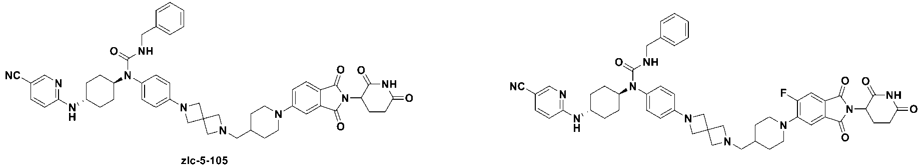

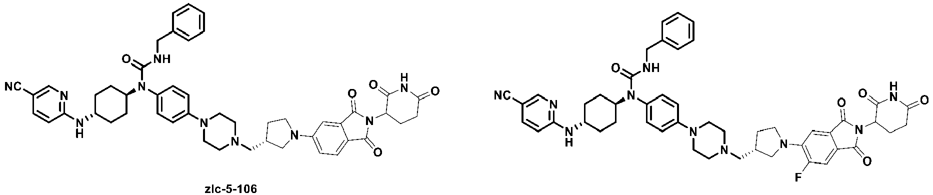

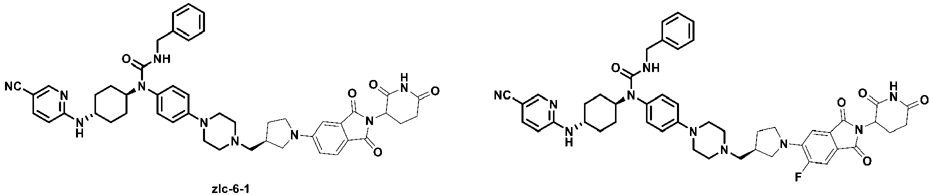

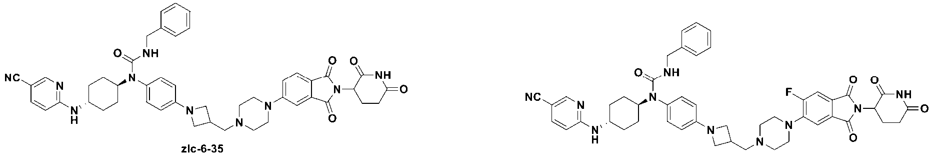

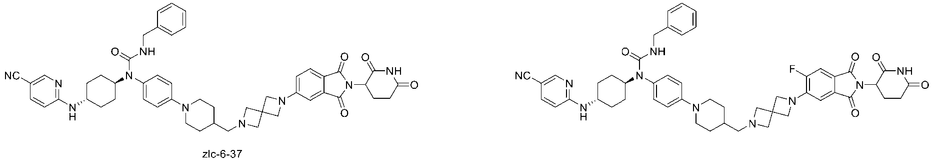

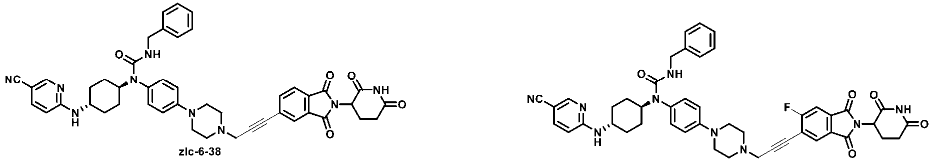

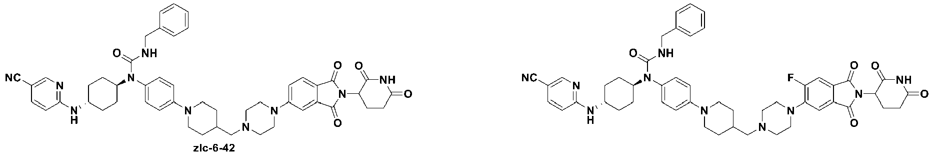

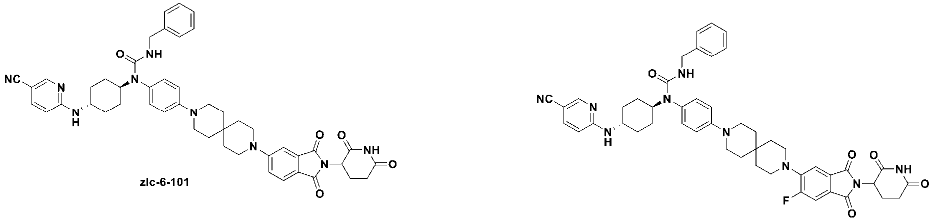

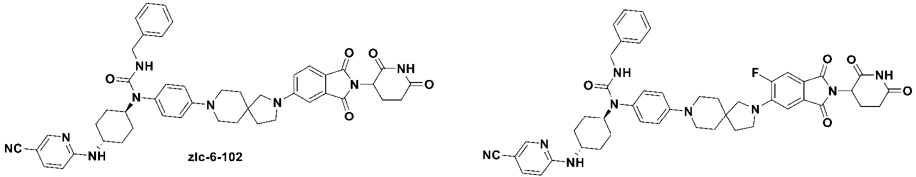

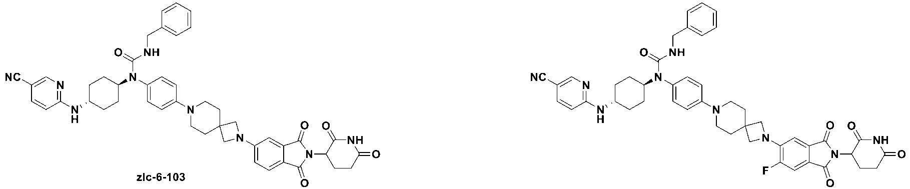

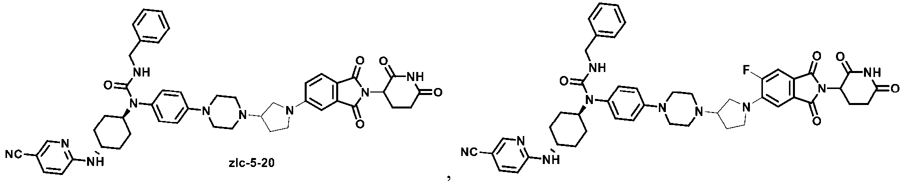

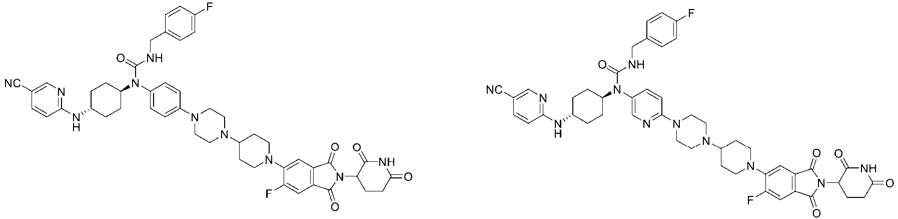

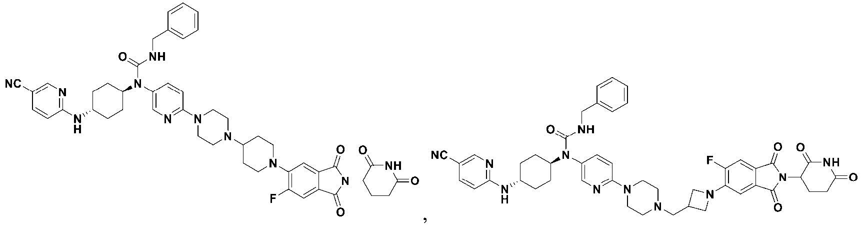

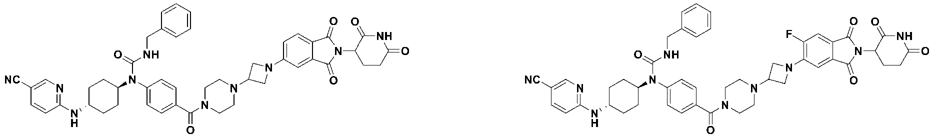

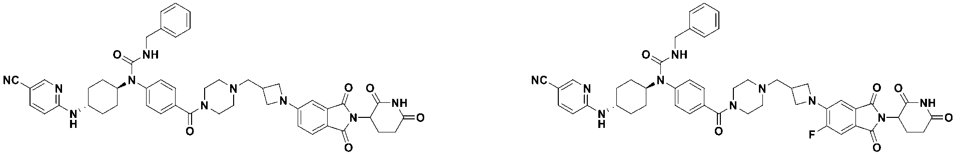

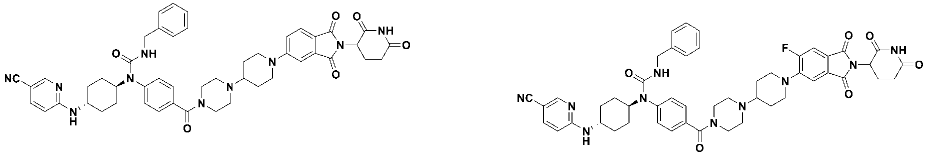

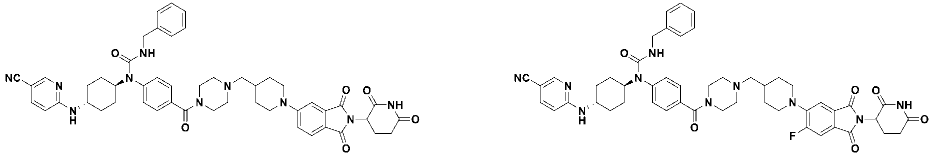

In a second aspect of the present invention, provided is a compound having a structure of formula (I), or a pharmaceutically acceptable salt thereof, or a pharmaceutical composition thereof, or a prodrug molecule thereof: 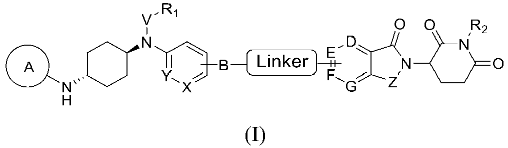 wherein Z is selected from the group consisting of: CH₂ or CO;
V is selected from the group consisting of: 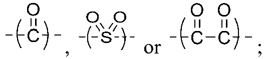
X and Y are selected from the group consisting of: N, CH or CR₃; wherein R₃ is selected from the group consisting of: halogen, cyano, hydroxyl, amino, C₁-C₃ alkyl, halogenated C₁-C₃ alkyl, C₁-C₃ alkoxy, halogenated C₁-C₃ alkoxy, C₃-C₈ cycloalkyl, or 3- to 8-membered heterocyclyl;
B is selected from the group consisting of: 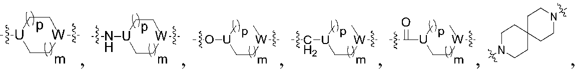 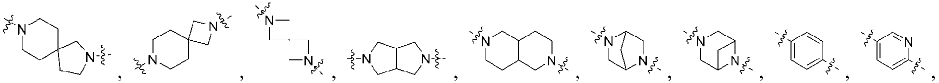 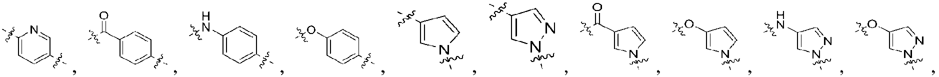 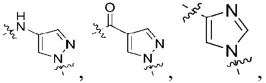 NH, O, CO, or CH₂; wherein each of U and W is independently selected from the group consisting of: N or CH; each of m and p is independently: 0, 1 or 2;
A ring is selected from the group consisting of: 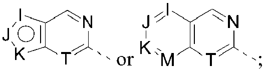
M and T are each independently selected from the group consisting of: N or CR₄;
I, J and K are each independently selected from the group consisting of: N, O, S, CR₄ and NR₄;
R₄ is selected from the group consisting of: hydrogen, halogen, cyano, hydroxy, amino, C₁-C₅ alkyl, halogenated C₁-C₅ alkyl, C₁-C₃ alkoxy, halogenated C₁-C₃ alkoxy, C₃-C₈ cycloalkyl, -CH₂R₅, -(CH₂)₂R₅, -(CH₂)₃R₅, or 3- to 8-membered heterocyclyl;
R₅ is selected from the group consisting of: cyano, hydroxy, amino, C₃-C₈cycloalkyl, or 3- to 8-membered heterocyclyl;
D, E, F, and G are each independently selected from the group consisting of: CH, N, or CR₆; wherein R₆ is selected from the group consisting of: halogen, trifluoromethyl, hydroxy, cyano, amino, methyl, methoxy, or trifluoromethoxy;
R₁ is selected from the group consisting of: H, -NHR₇, -OR₇, or -(C(R₉)R₈)R₇;
wherein R₇ is -R₁₀, -CH₂R₁₀ or -(CH₂)₂R₁₀;
R₈ and R₉ are each independently selected from the group consisting of: hydrogen, halogen, cyano, methyl, halomethyl, methoxy, halomethoxy, ethyl, haloethyl, ethoxy, haloethoxy, hydroxy, amino, or 3- to 8-membered heterocyclic ring containing 1, 2 or 3 heteroatoms, and the heteroatoms are selected from O, S or N;
or R₈ and R₉, together with the C atom to which they are attached, form a 3- to 7-membered heterocyclic ring;
R₁₀ is selected from the group consisting of:
   1) cyano, C₁-C₃ alkyl, halogenated C₁-C₄ alkyl, C₁-C₄ alkoxy, C₃-C₁₀ cycloalkyl, substituted or unsubstituted 5-to 12-membered aromatic ring, or substituted or unsubstituted 3- to 12-membered heterocyclic ring; or
   2) 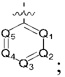
      wherein Q₁, Q₂, Q₃, Q₄ and Q₅ are each independently selected from: CH, N or CR₁₁;
      each R₁₁ is independently selected from the group consisting of: halogen, cyano, hydroxy, amino, nitro, C₁-C₃ alkyl, halogenated C₁-C₃ alkyl, C₁-C₄ alkoxy, halogenated C₁-C₄ alkoxy, or C₃-C₈ cycloalkyl;
      R₂ is selected from the group consisting of: H, C₁-C₃ alkyl, 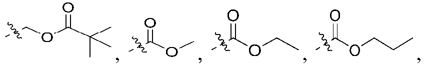 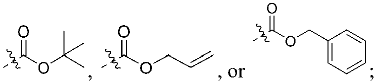
      Linker is: 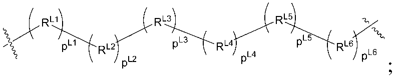
      wherein R^{L1} - R^{L6} are the same or different, and are each independently selected from the substituted or unsubstituted groups in the group consisting of: a bond, CH₂, CHD, CD₂, C=O, O, NH, SO, SO₂, P=O, NHCO, NHSO₂, OCH₂, OCH₂CH₂, CH₂OCH₂, NHCH₂, NMeCH₂, NHCH₂CH₂, NMeCH₂CH₂, CH₂NHCO, NHCOCH₂, 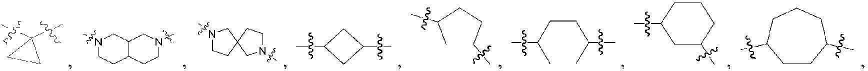 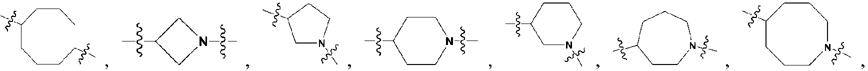 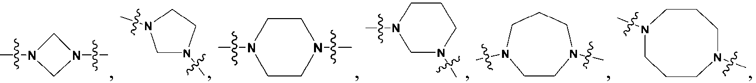 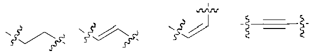 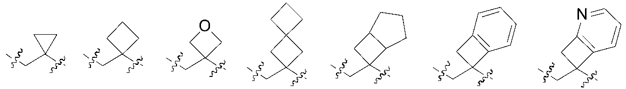 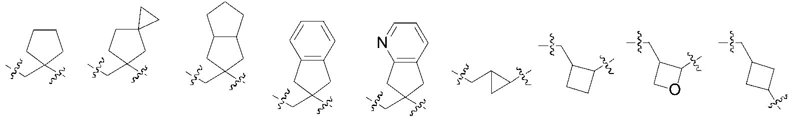 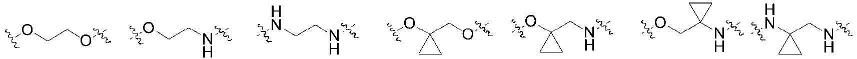 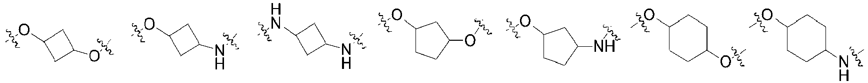 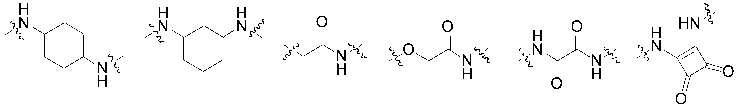 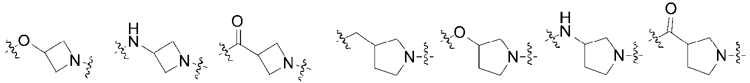 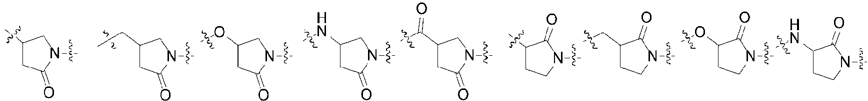 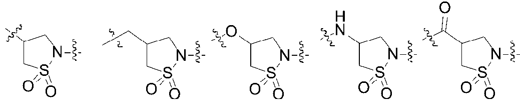 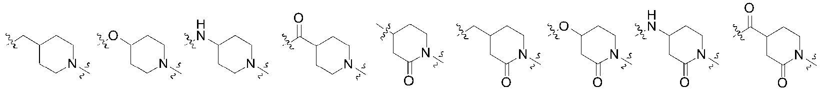 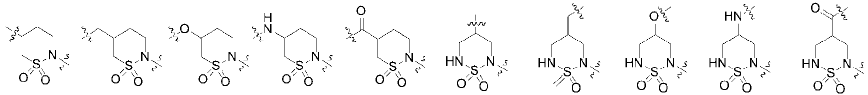 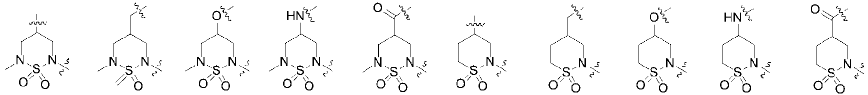 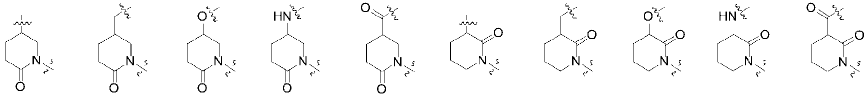 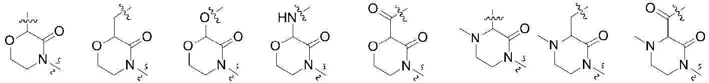 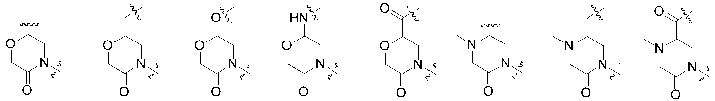 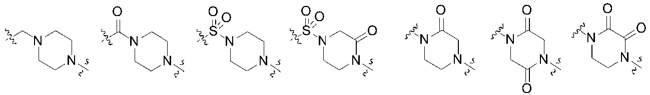 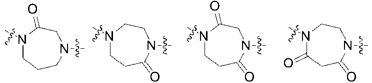 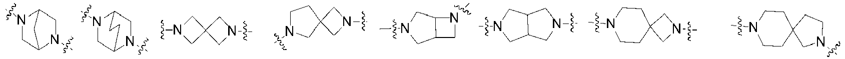 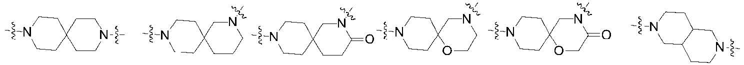 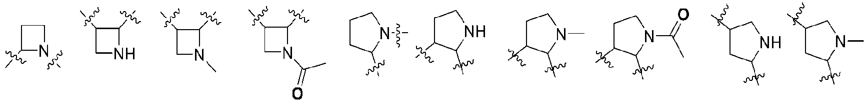 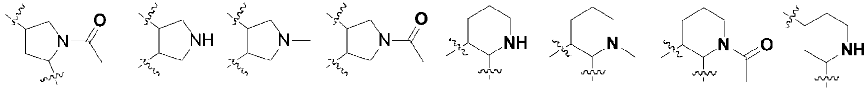 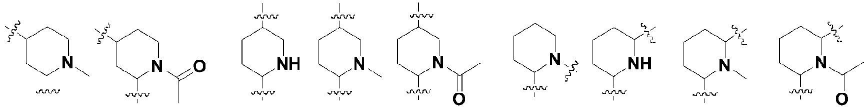 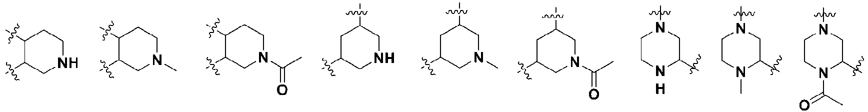 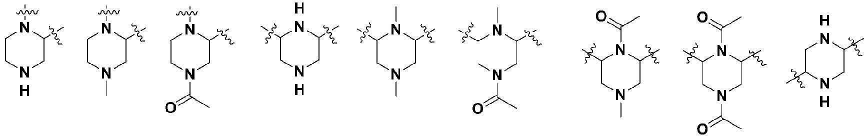 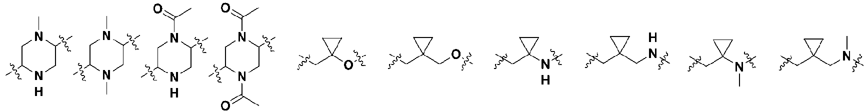 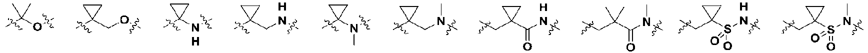 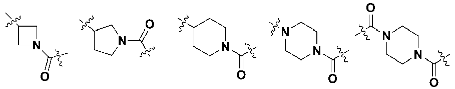 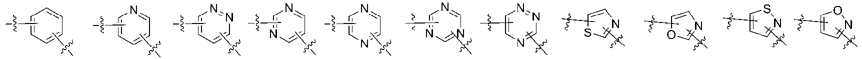 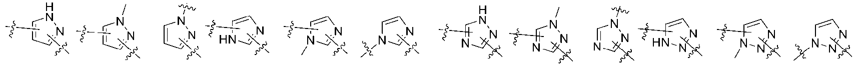 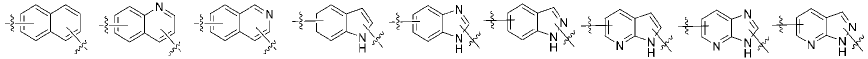 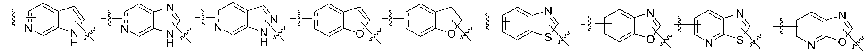 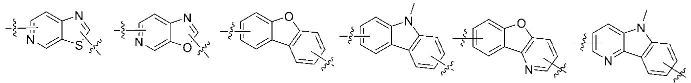 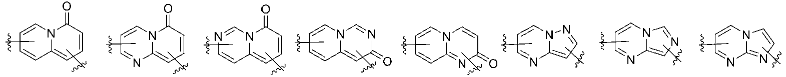 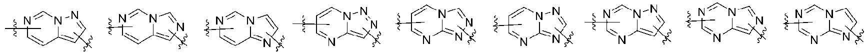 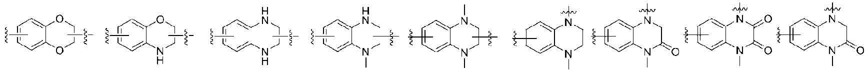 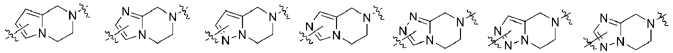 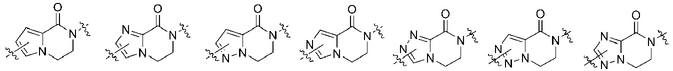 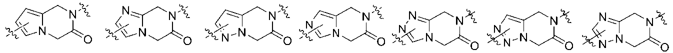
      wherein each n is independently 0, 1, 2, 3, 4, 5 or 6; each of r and m is independently 0, 1 or 2; each of U and W is independently selected from the group consisting of: N or CH
      the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester, amino, amido, sulfonamido, or ureido;
      each of p^{L1} - p^{L6} is independently selected from 0, 1, 2, 3, 4, 5 or 6.

In some embodiments, V is selected from the group consisting of:

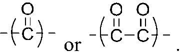

In some embodiments, X and Y are each independently selected from the group consisting of: N, CH or CR₃;

wherein R₃ is selected from the group consisting of: halogen, cyano, hydroxy, amino, C₁-C₃ alkyl, halogenated C₁-C₃ alkyl, C₁-C₃ alkoxy, or halogenated C₁-C₃ alkoxy.

In some embodiments, B is selected from the group consisting of: 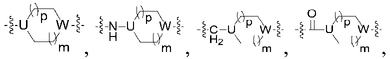 NH, O or CO;
wherein each of U and W is independently selected from the group consisting of: N or CH; each of m and p is independently 0 or 1.

In some embodiments, A ring is selected from the group consisting of: 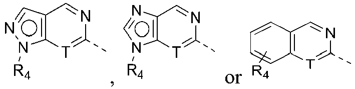 wherein T is selected from the group consisting of: CH or N.

In some embodiments, R₄ is selected from the group consisting of: hydrogen, halogen, cyano, hydroxy, amino, C₁-C₅ alkyl, halogenated C₁-C₃ alkyl, C₁-C₅ alkoxy, halogenated C₁-C₃ alkoxy, C₃-C₈ cycloalkyl, -CH₂R₅, -(CH₂)₂R₅, -(CH₂)₃R₅, or 3- to 8-membered heterocyclyl.

In some embodiments, the D, E, F and G are CH.

In some embodiments, the R₁ is -NHR₇;
wherein R₇ is -CH₂R₁₀; R₁₀ is defined as described above.

In another preferred embodiment, the R₁₀ is selected from the group consisting of: cyano, C₁-C₅ alkyl, halogenated C₁-C₄ alkyl, C₁-C₄ alkoxy, C₃-C₁₀ cycloalkyl, 4- to 7-membered heterocyclyl, or C₆-C₁₀ aryl; wherein the aryl is substituted with one or more CR₁₁; R₁₁ is defined as described above.

In some embodiments, the Linker is selected from the group consisting of: 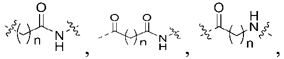 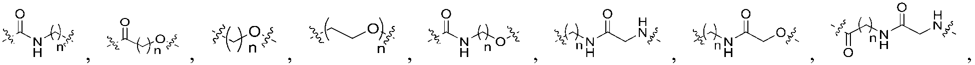 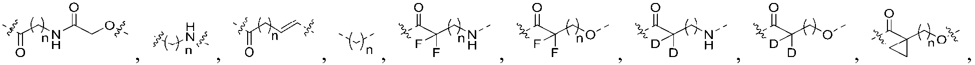 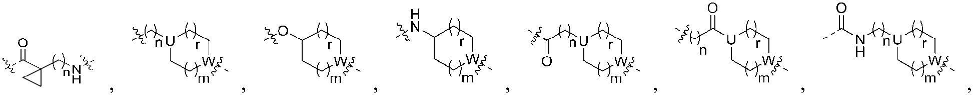 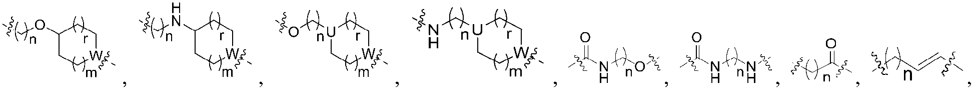 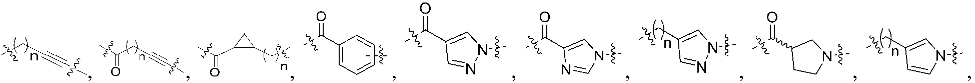 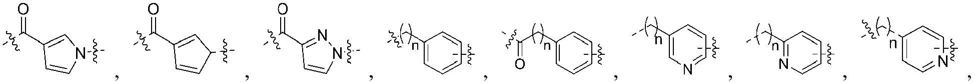 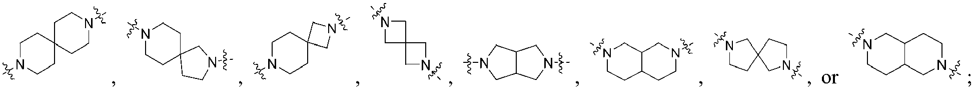 wherein each n is independently 0, 1, 2, 3, 4, 5 or 6; each of r and m is independently 0, 1 or 2; each of U and W is independently selected from the group consisting of: N or CH.

In another preferred embodiment, the Linker is selected from the group consisting of: 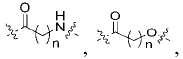 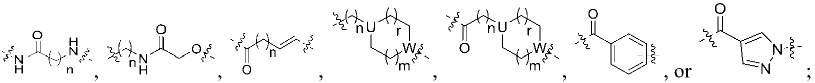 wherein each n is independently 0, 1, 2, 3, 4, 5 or 6; each of r and m is independently 0, 1 or 2; each of U and W is independently selected from the group consisting of: N or CH.

In some embodiments, in some embodiments, the compound has a structure represented by formula (II) or formula (III): 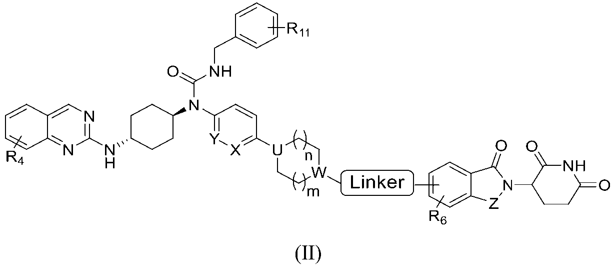 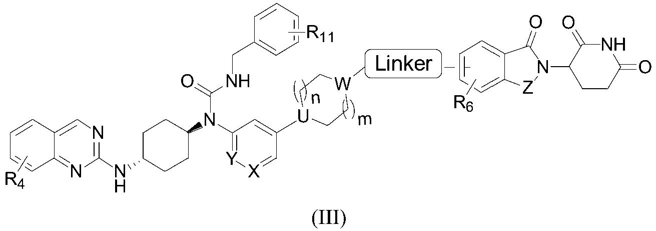 wherein U and W are independently selected from: CH or N;
X and Y are independently selected from: CH, N or CR₃;
Z is optionally selected from: CH₂ or CO;
n and m are independently selected from: 0 or 1.

In some embodiments, the compound is selected from the group consisting of:

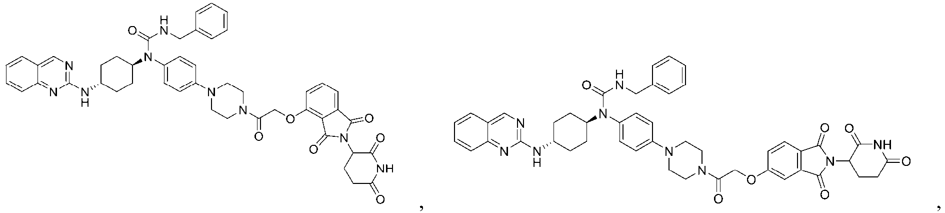

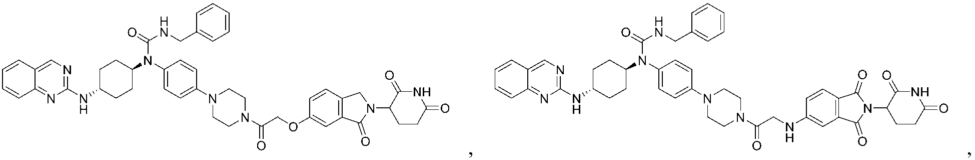

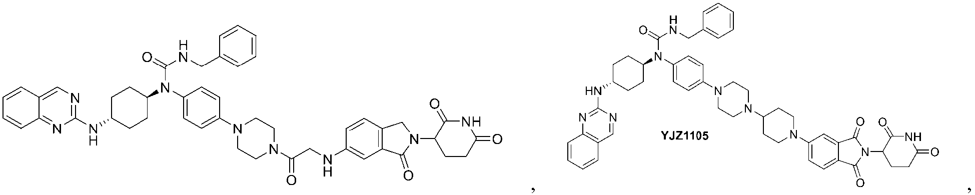

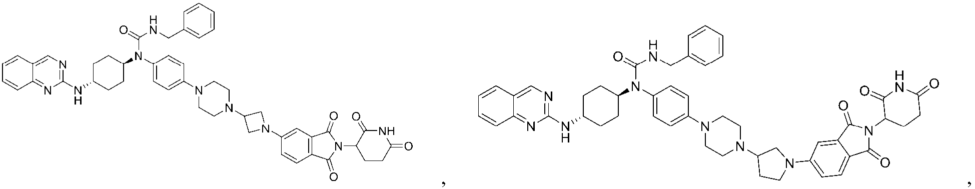

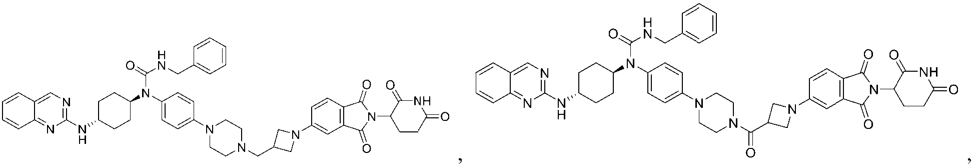

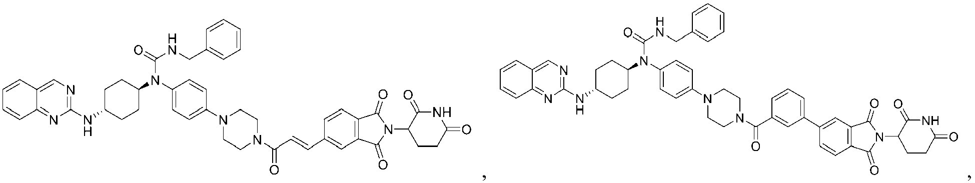

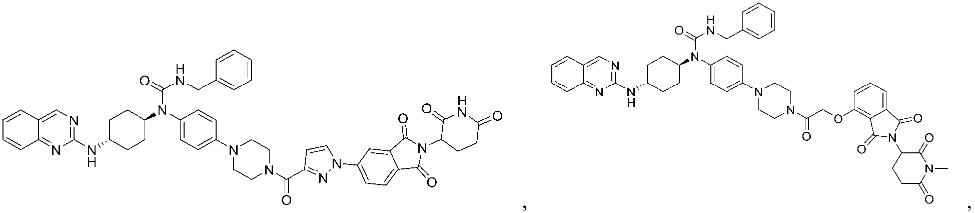

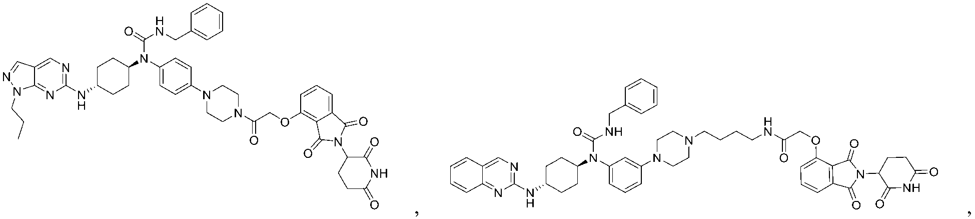

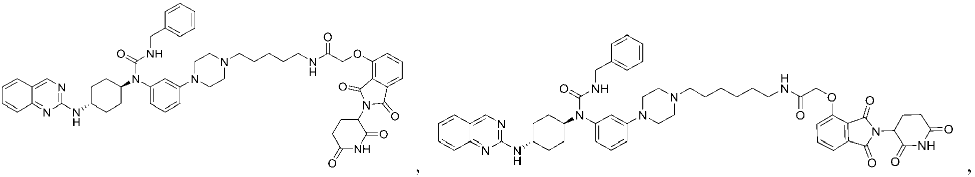

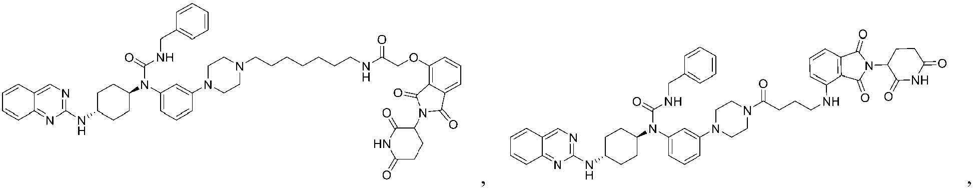

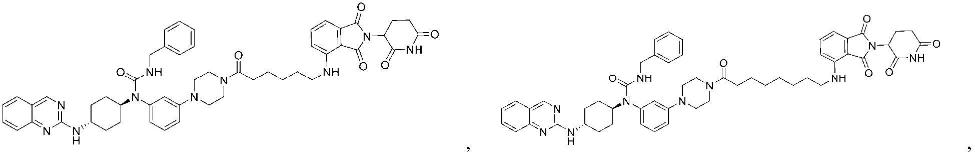

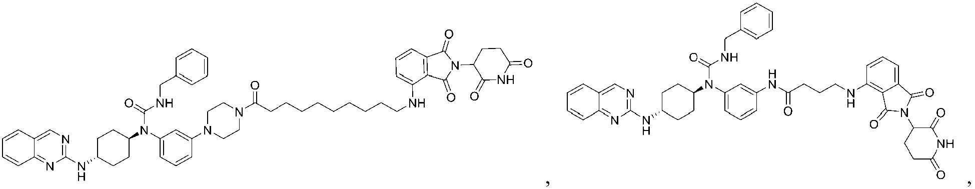

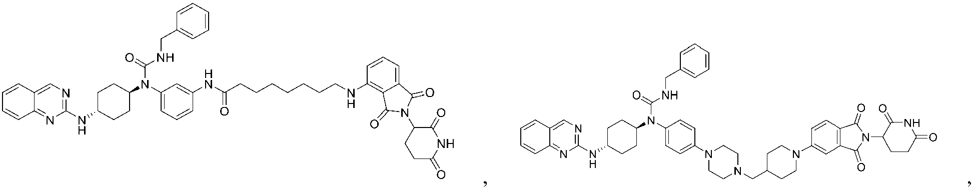

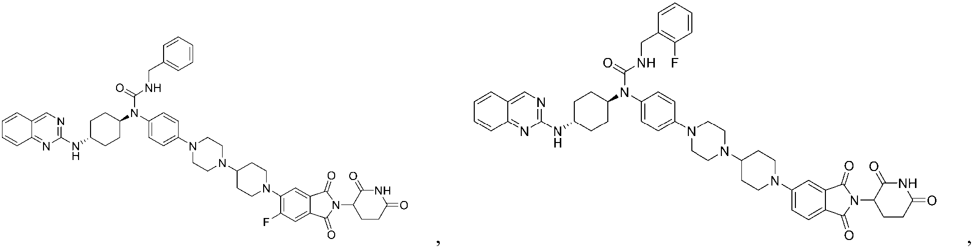

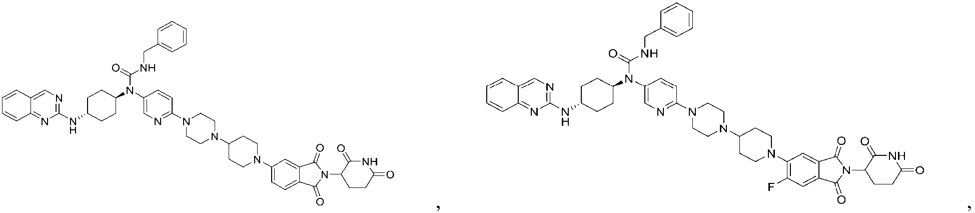

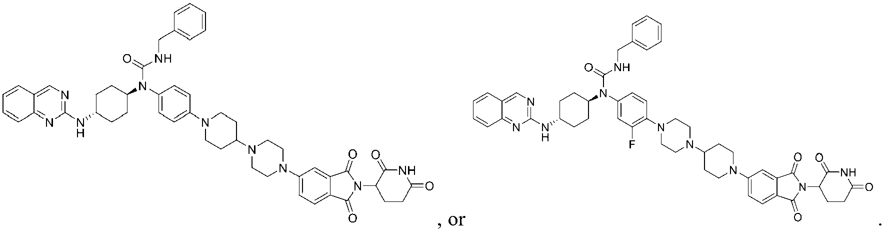

In a third aspect of the present invention, provided is a pharmaceutical composition, comprising
(1) as an active ingredient, a compound of the first or second aspect of the present invention, or a pharmaceutically acceptable salt thereof, a stereoisomer thereof, or a prodrug molecule thereof; and
optionally (2) a pharmaceutically acceptable carrier.

In a fourth aspect of the present invention, provided is use of the compound of the first or second aspect of the present invention, or the pharmaceutically acceptable salt thereof, or the stereoisomer thereof, or the prodrug molecule thereof, or the pharmaceutical composition of the third aspect of the present invention, for use in the preparation of a degradation agent for a CDK12/13 protein kinase.

In a fifth aspect of the present invention, provided is use of the compound of the first or second aspect of the present invention, or the pharmaceutically acceptable salt thereof, or the stereoisomer thereof, or the prodrug molecule thereof, or the pharmaceutical composition of the third aspect of the present invention, for use in the preparation of a drug for preventing and/or treating a disease mediated by a CDK12/13 serine/threonine protein kinase.

In some embodiments, the disease mediated by the CDK12/13 serine/threonine protein kinase is selected from the group consisting of: prostate cancer, breast cancer, uterine cancer, ovarian cancer, non-small cell lung cancer, small cell lung cancer, Ewing sarcoma, lung adenocarcinoma, squamous cell lung carcinoma, pancreatic cancer, liver cancer, skin cancer, epithelial cell carcinoma, gastrointestinal stromal tumor, leukemia, histiocytic lymphoma, nasopharyngeal carcinoma, head and neck tumor, colon cancer, rectal cancer, or glioma.

It should be understood that within the scope of the present invention, the above-mentioned technical features and the technical features specifically described below (e.g., in the examples) in the present invention may be combined with each other, thus forming novel or preferred technical solutions, which are not repeated one by one here due to limited space.

### BRIEF DESCRIPTION OF THE DRAWINGS

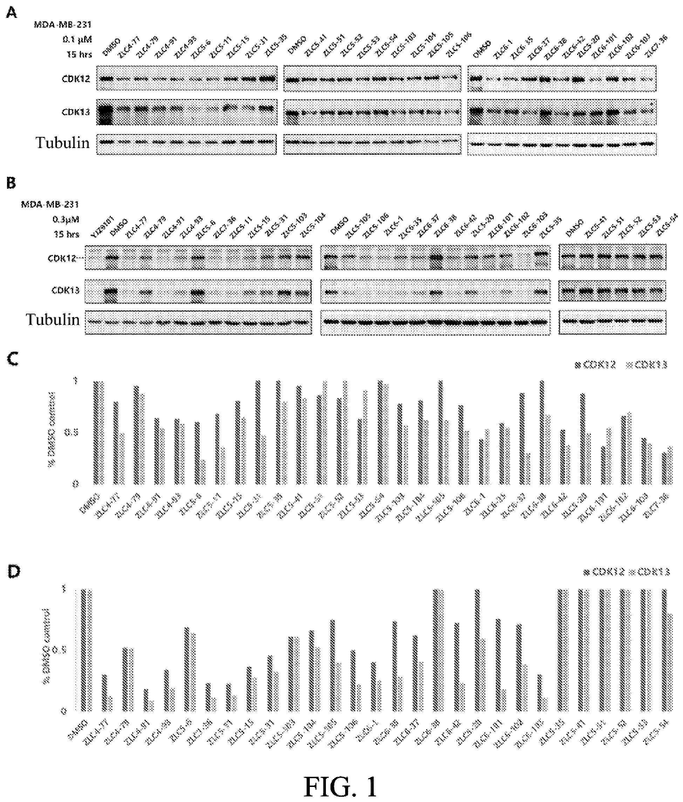

FIG. 1 is (A) WB results of degradation of CDK12 and CDK13 proteins after treatment of MDA-MB-231 cells by some compounds at 0.1µM for 15 h; (B) WB results of degradation of CDK12 and CDK13 proteins after treatment of MDA-MB-231 cells by some compounds at 0.3µM for 15 hours; (C)the CDK12/13 levels obtained by quantifying the gray value of the bands in the WB result image (FIG. A), with the control group DMSO as the reference standard; and (D) the CDK12/13 levels obtained by quantifying the gray value of the bands in the WB result image (FIG. B), with the control group DMSO as the reference standard.
FIG. 2 is (A) WB measurement results of time-dependent degradation of CDK12 and CDK13 proteins in MDA-MB-231 cells by representative compounds; and (B) WB measurement results of the intracellular CDK12/13 protein levels after treatment of MDA-MB-231 cells by representative compounds at different concentrations for 15 h.
FIG. 3 is measurement results of the inhibitory activity of compounds on triple negative breast cancer cell MDA-MB-231.

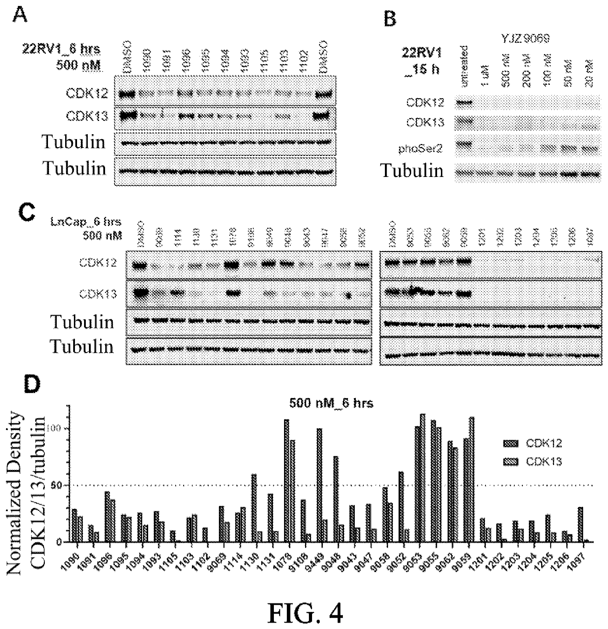

FIG. 4 is (A) WB results of degradation of CDK12 and CDK13 proteins after treatment of 22RV1 cells by some compounds at a concentration of 500 nM for 6 hours; (B) compound **YJZ9069** degrades CDK12 and CDK13 proteins in 22RV1 cells in a dose-dependent manner and inhibits phosphorylation of serine at position 2 (phoSer2) in the C-terminal domain of RNA polymerase II (RNA Pol II) in a dose-dependent manner; (C) WB results of degradation of CDK12 and CDK13 proteins after treatment of LnCap cells by some compounds at a concentration of 500 nM for 6 hours; (D) the protein levels by quantifying the gray value of the bands in the WB result images (FIGs. A and C) using the control group DMSO as the reference standard.
FIG. 5 is WB measurement results of the intracellular CDK12 and CDK13 protein levels after treatment of 22RV1 cells by the representative compounds **YJZ9069, YJZ1201, YJZ1202, YJZ1203, YJZ1204, YJZ1097, YJZ1205** and **YJZ1206** at different concentrations for 6 h.
FIG. 6 is measurement results of the proliferation-inhibiting activity of compounds on prostate cancer cell line VCap.

### DETAILED DESCRIPTION

After extensive and in-depth research, the inventors have unexpectedly discovered a class of trans-1,4-cyclohexanediamine compounds, and conducted a series of bioactivity tests on the compounds, thereby finding that the compounds have excellent CDK12/13 inhibition properties. On the basis of this, the present invention has been made.

The experimental methods without clear indication of specific conditions in the following examples of the present invention are carried out usually based on general conditions, or based on conditions recommended by manufacturers. Various common chemical reagents used in the examples are all commercially available products.

Unless otherwise defined, all technical terms and scientific terms used in the present invention have the same meaning as commonly understood by those skilled in the technical field of the present invention. The terms used in the specification of the present invention are only for the purpose of describing specific examples, and are not used to limit the present invention.

The terms "comprising" and "having" and any variations thereof in the present invention are intended to encompass non-exclusive inclusions. For example, a process, method, apparatus, product, or device that includes a series of steps is not limited to the listed steps or modules, but optionally further includes unlisted steps, or optionally further includes other steps that are inherent to such process, method, product, or device.

The "plurality of" mentioned in the present invention refers to two or more than two. The term "and/or" describes an association relationship between associated objects, and means that there may be three relationships. For example, A and/or B may mean three situations: A exists alone, both A and B exist, or B exists alone. The character "/" generally means that there is an "or" relationship between associated objects therebefore and thereafter.

In the compound of the present invention, when any variable (e.g., R₁₀ or R₁₁) occurs more than once in any component, the definition of the variable at each occurrence is independent of the definition of the variable at each of other occurrences. Likewise, a combination of substituents and variables is allowed, as long as such a combination makes a compound stable. A line drawn from a substituent into a ring system means that the indicated bond may be attached to any substitutable ring atom. If the ring system is polycyclic, it means that such a bond is only attached to any appropriate carbon atom on an adjacent ring. It should be understood that those of ordinary skills in the art may select a substituent and a substitution form for the compound of the present invention to provide a compound that is chemically stable and is readily synthesized from readily available starting materials in accordance with a technique in the art and a method set forth below. If a substituent itself is substituted with more than one group, it should be understood that these groups may be on the same carbon atom or on different carbon atoms, as long as the structure is stable.

As used herein, the term "alkyl" is meant to include a branched or linear saturated aliphatic hydrocarbon radical having a particular number of carbon atoms. For example, "C₁-C₈" in "C₁-C₈ alkyl" is defined to include a group having 1, 2, 3, 4, 5, 6, 7 or 8 carbon atoms in a linear or branched arrangement. The term "cycloalkyl" refers to a monocyclic saturated aliphatic hydrocarbon radical having a particular number of carbon atoms. For example, the "cycloalkyl" includes cyclopropyl, methyl-cyclopropyl, 2,2-dimethyl-cyclobutyl, 2-ethyl-cyclopentyl, cyclohexyl, and the like.

As used herein, the term "alkenyl" includes a linear or branched alkenyl group. For example, C₂-C₆ alkenyl refers to a linear or branched alkenyl group having 2 to 6 carbon atoms, such as vinyl, allyl, 1-propenyl, isopropenyl, 1-butenyl, 2-butenyl, or the like.

As used herein, the term "alkynyl" includes a linear or branched alkynyl group. For example, C₂-C₆ alkynyl refers to a linear or branched alkynyl group having 2 to 6 carbon atoms, such as ethynyl, propynyl, butynyl, or the like.

As used herein, the term "cycloalkyl" refers to a cyclic saturated aliphatic hydrocarbon radical having a particular number of carbon atoms. For example, C₃-C₁₀ alkenyl refers to a cyclic saturated aliphatic hydrocarbon radical having 3 to 10 carbon atoms. It may be a monocyclic ring, e.g., cyclopropyl, cyclobutyl, cyclopentyl, cyclohexyl, or the like. Bicyclic ring forms, such as bridged ring or spiro-ring forms, are also possible.

As used herein, the term "heterocyclyl" or "heterocycloalkyl" refers to a saturated or partially saturated cyclic group having a particular number of ring atoms (e.g., 3-10 ring atoms), wherein 1-3 of the atoms are heteroatoms selected from N, S and O. It may be a monocyclic ring, or may be a bicyclic or polycyclic ring, for example, a fused ring, a bridged ring or a spiro ring. Specific examples may be oxetanyl, azetanyl, tetrahydro-2H-pyranyl, piperidinyl, tetrahydrofuryl, morpholinyl, pyrrolidinyl, etc.

As used herein, the term "alkylamino" refers to an amino group substituted with an alkyl group. For example, "C₁-C₆ alkylamino" refers to an amino group that is substituted with a C₁-C₆ alkyl group, which may be monosubstituted or disubstituted; for example, methylamino, ethylamino, propylamino, isopropylamino, butylamino, isobutylamino, tert-butylamino, dimethylamino, diethylamino, dipropylamino, diisopropylamino, dibutylamino, diisobutylamino, ditert-butylamino, etc.

As used herein, the term "alkyloxy" refers to a group having an alkyl-oxy structure. For example, "C₁-C₆ alkoxy" refers to a linear or branched alkoxy group having 1 to 6 carbon atoms, including methoxy, ethoxy, propoxy, isopropoxy, butoxy, isobutoxy, tert-butoxy, etc.

As used herein, the term "haloalkyl" represents an alkyl group in which one or more hydrogen atoms are substituted with a halogen, wherein the alkyl group is defined as described above.

As used herein, the term "haloalkoxy" represents an alkyloxy group in which one or more hydrogen atoms are substituted with a halogen, wherein the alkyloxy is defined as described above.

As will be understood by those skilled in the art, the "halogen" used herein is intended to include F, Cl, Br and I. Preferably, the halogen or halogen atom is selected from F, Cl and Br. "Halogenated" refers to substitution with an atom selected from F, Cl, Br and I.

Unless otherwise specified as "substituted or unsubstituted", the groups in the present invention may be substituted with substituents selected from the group consisting of: halogen, cyano, nitro, hydroxy, amino, C₁-C₆ alkylamino, C₁-C₅ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, etc.

The present invention includes the free form of the compound of formula (I) (herein, formula (I) includes formula (II)), and also includes pharmaceutically acceptable salts, stereoisomers and prodrug molecules thereof. The term "free form" refers to a compound in a non-salt form. Pharmaceutically acceptable salts encompassed include not only the exemplary salts of the specific compounds described herein, but also all typical pharmaceutically acceptable salts of the free forms of the compounds of Formula (I) or Formula (II). The free forms of the particular salts of the compounds may be isolated using techniques known in the art. For example, the free form may be regenerated by treating the salt with an appropriate dilute aqueous base solution, such as a dilute aqueous NaOH solution, a dilute aqueous potassium carbonate solution, dilute aqueous ammonia, and a dilute aqueous sodium bicarbonate solution. The free forms differ somewhat from their respective salt forms in some physical properties such as solubility in a polar solvent, but for the purpose of the present invention, such acidic and basic salts are otherwise pharmaceutically equivalent to their respective free forms.

The pharmaceutically acceptable salt of the present invention may be synthesized from the compound of the present invention comprising a basic moiety or an acidic moiety in accordance with a conventional chemical method. Generally, a salt of an alkali compound is prepared by ion exchange chromatography or by reacting a free base with a stoichiometric amount or excess amount of an inorganic or organic acid in a desired salt form in a suitable solvent or a combination of a plurality of solvents. Similarly, a salt of an acidic compound is formed by a reaction with a suitable inorganic or organic base.

Accordingly, the pharmaceutically acceptable salt of the compound of the present invention includes a conventional non-toxic salt of the compound of the present invention formed by reacting an alkali compound of the present invention with an inorganic or organic acid. For example, the conventional non-toxic salt not only includes a salt prepared from an inorganic acid, such as hydrochloric acid, hydrobromic acid, sulfuric acid, sulfamic acid, phosphoric acid, or nitric acid, but also includes a salt prepared from an organic acid, such as acetic acid, propionic acid, succinic acid, glycolic acid, stearic acid, lactic acid, malic acid, tartaric acid, citric acid, ascorbic acid, pamoic acid, maleic acid, hydroxymaleic acid, phenylacetic acid, glutamic acid, benzoic acid, salicylic acid, p-aminobenzenesulfonic acid, 2-acetoxymonobenzoic acid, fumaric acid, benzenesulfonic acid, toluenesulfonic acid, methanesulfonic acid, ethanedisulfonic acid, oxalic acid, isethionic acid, or trifluoroacetic acid.

If the compound of the present invention is acidic, an appropriate "pharmaceutically acceptable salt" refers to a salt prepared from a pharmaceutically acceptable non-toxic base including an inorganic base and an organic base. A salt derived from an inorganic base includes an aluminum salt, an ammonium salt, a calcium salt, a copper salt, a ferric salt, a ferrous salt, a lithium salt, a magnesium salt, a manganese salt, a manganous salt, a potassium salt, a sodium salt, a zinc salt, and the like, and is particularly preferably an ammonium salt, a calcium salt, a magnesium salt, a potassium salt, and a sodium salt. A salt derived from a pharmaceutically acceptable organic non-toxic base includes a salt of a primary amine, a secondary amine, and a tertiary amine, and a substituted amine includes a naturally occurring substituted amine, a cyclic amine, and an basic ion exchange resin, such as arginine, betaine, caffeine, choline, N,N'-dibenzylethylenediamine, diethylamine, 2-diethylaminoethanol, 2-dimethylaminoethanol, aminoethanol, ethanolamine, ethyldiamine, N-ethylmorpholine, N-ethylpiperidine, glucosamine, glucosamine, histidine, hydroxocobalamin, isopropylamine, lysine, methylglucamine, morpholine, piperazine, piperidine, polyamine resin, procaine, purine, theobromine, triethylamine, trimethylamine, tripropylamine, and tromethamine.

Berg et al, "Pharmaceutical Salts" J. Pharm. Sci. '1977: 66: 1-19 describes in more detail the preparation of the pharmaceutically acceptable salt described above and other typical pharmaceutically acceptable salts.

Unless otherwise specified, the structural formulas described in the present invention are intended to include all isomeric forms (such as enantiomers, diastereomers and geometric isomers (or conformational isomers)): for example, R and S configurations containing asymmetric centers, (Z) and (E) isomers containing double bonds, etc. Therefore, individual stereochemical isomers of the present compounds or mixtures thereof as enantiomers, diastereomers, or geometric isomers (or conformational isomers) are within the scope of the present invention.

As used herein, the term "tautomer" means that structural isomers of different energies can interconvert across a low energy barrier. For example, proton tautomers (i.e., prototropic) include interconversions by proton migration, such as 1H-indazole and 2H-indazole. Valence tautomers include interconversions by reorganization of some of the bonding electrons.

As used herein, the term "solvate" refers to a complex in which the compound of the present invention is coordinated with solvent molecules with a specific ratio.

As used herein, the term "hydrate" refers to a complex in which the compound of the present invention is coordinated with water.

The compounds of the present invention may also be in prodrug form. As used herein, the term "prodrug" refers to a compound that produces an active compound during metabolism (e.g., in vivo or in vitro). In some embodiments, a prodrug may be inactive or may have a lower activity than a free drug, but may provide favorable treatment, administration, or metabolic characteristics. Exemplary prodrug moieties of the present invention may be linked to a free drug via hydroxy, amino, phosphate, or phosphorothioate backbone of a nucleotide, and may comprise an ester, carbamate, carbonyl, thioester, amide, isocyanate, urea, thiourea, or other physiologically acceptable metabolically unstable moieties. In some embodiments, the prodrug is activated by enzymatic hydrolysis.

The present disclosure also includes isotopically labeled compounds which are the same as the compound of formula (I) (including the compound of formula (II)), but in which one or more atoms are replaced by an atom having an atomic mass or mass number different from the atomic mass or mass number usually found in nature. Examples of isotopes suitable for inclusion in the compound of the present invention are hydrogen, carbon, nitrogen, oxygen, phosphorus, sulfur, fluorine and chlorine, such as, but not limited to ²H, ³H, ¹³C, ¹⁴C, ¹⁵N, ¹⁸O, ³¹P, ³⁵S, ¹⁸F and ³⁶Cl. Replacement with heavier isotopes (e.g., deuterium, i.e. ²H) may afford certain therapeutic advantages resulting from greater metabolic stability, for example, increased in vivo half-life or lower dosage requirements, and thus, heavier isotopes may be preferred in some circumstances. The compounds can be combined with positron-emitting isotopes for use in medical imaging and positron emission tomography (PET) studies to determine receptor distribution. Suitable positron-emitting isotopes that can be incorporated into the compound of formula (I) or formula (II) are ¹¹C, ¹³N, ¹⁵O and ¹⁸F. The isotopically labeled compound of formula (I) or formula (II) can generally be prepared by replacing a non-isotopically labeled reagent with a suitable isotopically labeled reagent according to a conventional technology known to those skilled in the art or a method similar to the methods described herein.

The compound disclosed herein may be present in solvated and non-solvated forms with a pharmaceutically acceptable solvent such as water or ethanol, and the present invention is intended to cover the solvated and non-solvated forms. In an embodiment, the compound is amorphous. In an embodiment, the compound is a single polymorph. In another embodiment, the compound is a mixture of polymorphs. In another embodiment, the compound is a crystalline form.

### Pharmaceutical composition and administration method

Since the compound of the present invention is a CDK12/13 protein degradation agent, the compound and a pharmaceutically acceptable salt thereof, as well as other compound forms disclosed herein, can be included in a pharmaceutical composition useful for treating, preventing, and alleviating a disease associated with CDK12/13 activity.

The pharmaceutical composition of the present invention comprises an effective amount, e.g., a safe and effective amount, of the compound of the present invention or a pharmacologically acceptable salt thereof and a pharmacologically acceptable excipient or carrier. The "effective amount" refers to an amount that is enough to trigger a desired biological response (e.g., treatment of a disorder). The "safe and effective amount" refers to: an amount of the compound that is enough to significantly ameliorate a condition without causing severe side effects. Generally, the pharmaceutical composition comprises from 1 to 3000 mg (active dose ranging from 3 to 30 mg/kg) of the compound/dose of the present disclosure, and more preferably comprises from 10 to 2000 mg of the compound/dose of the present invention. Preferably, "a dose" is a capsule or tablet.

The "pharmaceutically acceptable carrier" refers to: one or more compatible solid or liquid fillers or gel substances which are suitable for human use, and must be of sufficient purity and sufficiently low toxicity. The "compatibility" herein means that components of the composition can be mixed with the compound of the present disclosure and with each other without significantly reducing the efficacy of the compound. Examples of pharmaceutically acceptable carrier moieties include cellulose and derivatives thereof (such as sodium carboxymethylcellulose, sodium ethylcellulose, and cellulose acetate), gelatin, talc, solid lubricants (such as stearic acid and magnesium stearate), calcium sulfate, vegetable oils (such as soybean oil, sesame oil, peanut oil, and olive oil), polyol (such as propylene glycol, glycerol, mannitol, and sorbitol), emulsifiers (such as Tween^{®}), wetting agents (such as sodium lauryl sulfate), colorants, flavoring agents, stabilizers, antioxidants, preservatives, and pyrogen-free water.

The administration mode of the compound or the pharmaceutical composition of the present invention is not particularly limited, and the representative administration mode includes (but is not limited to): oral, intratumoral, rectal, parenteral (intravenous, intramuscular, or subcutaneous), or topical administration.

Solid dosage forms for oral administration include capsules, tablets, pills, powders, and granules. In these solid dosage forms, an active compound is mixed with at least one conventional inert excipient (or carrier), such as sodium citrate or dicalcium phosphate, or with the following ingredients: (a) a filler or a filling agent, such as starch, lactose, sucrose, glucose, mannitol, and silicic acid; (b) a binder, such as hydroxymethylcellulose, alginate, gelatin, polyvinylpyrrolidone, sucrose, and acabic gum; (c) a humectant, such as glycerin; (d) a disintegrant, such as agar, calcium carbonate, potato starch or tapioca starch, alginic acid, some complex silicates, and sodium carbonate; (e) a retarding solvent, such as paraffin; (f) an absorption enhancer, such as a quaternary amine compound; (g) a wetting agent, such as cetyl alcohol and glyceryl monostearate; (h) an adsorbent, such as kaolin; and (i) a lubricant, such as talc, calcium stearate, magnesium stearate, solid polyethylene glycol, sodium lauryl sulfate, or a mixture thereof. In the capsules, the tablets, and the pills, the dosage forms may further include a buffering agent.

The solid dosage forms, such as the tablets, the dragees, the capsules, the pills, and the granules, may be prepared from a coating and a shell material, such as an enteric coating and other materials well known in the art. They may comprise an opacifying agent and an active compound, or a compound in such compositions that it is released in a portion of the digestive tract in a delayed manner. Examples of useful embedding components are polymers and waxes. When necessary, the active compound may also be in micro-encapsulated form with one or more of the above-mentioned excipients.

Liquid dosage forms for oral administration include a pharmaceutically acceptable emulsion, solution, suspension, syrup, or elixir. In addition to the active compound, the liquid dosage forms may comprise an inert diluent commonly used in the art, such as water or other solvents, a solubilizing agent, and an emulsifier, such as ethanol, isopropanol, ethyl carbonate, ethyl acetate, propanediol, 1,3-butanediol, dimethylformamide, and an oil, in particular, cottonseed oil, peanut oil, maize germ oil, olive oil, castor oil, and sesame oil, or a mixture thereof.

Besides these inert diluents, the composition may further comprise an auxiliary agent, such as a wetting agent, an emulsifier and a suspending agent, a sweetening agent, a corrigent, and a perfume.

Besides the active compound, the suspension may further comprise a suspending agent, such as ethoxylated isooctadecanol, polyoxyethylene sorbitol and sorbitan ester, microcrystalline cellulose, aluminum methoxide, and agar, or a mixture thereof.

A composition for parenteral injection may comprise a physiologically acceptable sterile aqueous or anhydrous solution, dispersion, suspension or emulsion, and a sterile powder for redissolution into a sterile injectable solution or dispersion. An appropriate aqueous and non-aqueous carrier, diluent, solvent, or excipient includes water, ethanol, polyol, and an appropriate mixture thereof.

Dosage forms for topical administration of the compound of the present invention include an ointment, a powder, a patch, a spray, and an inhalant. An active ingredient is mixed under sterile conditions with a physiologically acceptable carrier and any preservative, buffering agent, or propellant, when necessary.

The compound of the present disclosure may be administered alone or may be administered in combination with other pharmaceutically acceptable compounds.

When the pharmaceutical composition is used, a safe and effective amount of the compound of the present invention is suitable for a mammal (such as human) in need of treatment, wherein the dose is a pharmaceutically considered effective dose when administered, and the daily administration dose for a person with a body weight of 60 kg is generally from 1 to 2000 mg, and preferably from 6 to 600 mg. Of course, a specific dose should also be determined by comprehensively considering factors, such as a route of administration and a health condition of a patient, which are all within the scope of skills of skilled physicians.

### Use and therapeutic method

As described above, the compound of the present invention is a CDK12/13 protein degradation agent, and as such the compound or the composition comprising the compound may be used to treat, prevent, and alleviate a disease associated with activity or aberrant expression of CDK12/13. In some embodiments, disclosed herein is use of the compound of the present invention in the preparation of a drug for preventing and/or treating a disease mediated by CDK12/13 serine/threonine protein kinase. In some embodiments, disclosed herein is the compound of the present invention for use in the prevention and/or treatment of a disease mediated by CDK12/13 serine/threonine protein kinase. In some embodiments, disclosed herein is a method for treating a disease mediated by CDK12/13 serine/threonine protein kinase in a subject in need thereof, including administering to the subject an effective amount of the compound of the present invention. In some embodiments, the disease mediated by the CDK12/13 serine/threonine protein kinase includes: prostate cancer, breast cancer, uterine cancer, ovarian cancer, non-small cell lung cancer, small cell lung cancer, Ewing sarcoma, lung adenocarcinoma, squamous cell lung carcinoma, pancreatic cancer, liver cancer, skin cancer, epithelial cell carcinoma, gastrointestinal stromal tumor, leukemia, histiocytic lymphoma, or nasopharyngeal carcinoma, head and neck tumor, colon cancer, rectal cancer, or glioma.

When being used for the use and method disclosed herein, the disclosed compound and composition can be used in combination with other known therapies. As used herein, "combined" administration refers to the delivery of two (or more) different treatments to a subject during a course when the subject has a disorder, e.g., delivery of the two or more treatments after the subject has been diagnosed with the disorder and before the disorder has been cured or eliminated or treatment has been ceased for other reasons. In some embodiments, delivery of one treatment is still ongoing when delivery of a second treatment begins, thereby resulting in overlap in terms of administration. This is sometimes referred to herein as "simultaneous" or "concurrent delivery". In other embodiments, the delivery of one treatment ends before the delivery of the other treatment begins. In some embodiments of either case, the treatment is more effective due to the combined administration. For example, the second treatment is more effective, e.g., compared to giving the second treatment without the first treatment, the same effect can be seen with fewer second treatments, or the second treatment provides a greater reduction in symptoms; or the similar conditions are observed with the first treatment. In some embodiments, delivery results in a reduction in symptoms or other parameters associated with a disorder that is greater than the reduction observed with one treatment delivered in the absence of the other treatment. The effects of the two treatments can be partially additive, fully additive, or greater than additive. Delivery can be such that the effect of the first treatment delivered is still detectable when the second treatment is delivered.

The compound or composition disclosed herein and at least one additional therapeutic agent can be administered simultaneously, in the same or separate compositions, or sequentially. For sequential administration, the compound described herein can be administered first and the additional agent can be administered subsequently, or the order of administration can be reversed.

In some embodiments, the compound described herein is administered in combination with other treatment modalities, including surgery, radiation, transplantation (e.g., stem cell transplantation or bone marrow transplantation), chemotherapy, immunotherapy, cryotherapy, and/or thermotherapy. Such combination therapy may allow for administration of agents and/or other agents at low doses, thereby avoiding possible toxicity or complications associated with the therapies.

In some embodiments, the compound described herein is administered together with at least one additional therapeutic agent, such as a chemotherapeutic agent. In certain embodiments, the compound described herein is administered in combination with one or more additional chemotherapeutic agents. The chemotherapeutic agents may be chemotherapeutic agents identified in the "A to Z List of Cancer Drugs" published by the National Cancer Institute.

### The main advantages of the present invention are that:

1. The degradation agent for cyclin-dependent kinase 12/13 (CDK12/13) provided by the present invention can effectively degrade CDK12 and CDK13 protein kinases and can be used in the preparation a drug for preventing or treating a disease mediated by CDK12 and/or CDK13 protein kinases, such as prostate cancer, breast cancer, uterine cancer, ovarian cancer, non-small cell lung cancer, small cell lung cancer, Ewing sarcoma, lung adenocarcinoma, squamous cell lung carcinoma, pancreatic cancer, liver cancer, skin cancer, epithelial cell carcinoma, gastrointestinal stromal tumor, leukemia, histiocytic lymphoma, nasopharyngeal carcinoma, head and neck tumor, colon cancer, rectal cancer, glioma, etc.
2. The degradation agent for cyclin-dependent kinase 12/13 (CDK12/13) provided by the present invention has strong degradation activity.
3. The degradation agent for cyclin-dependent kinase 12/13 (CDK12/13) provided by the present invention has high protein kinase degradation selectivity.

The present invention is further described in detail below with reference to specific examples.

### Example 1: Compound zlc-4-77

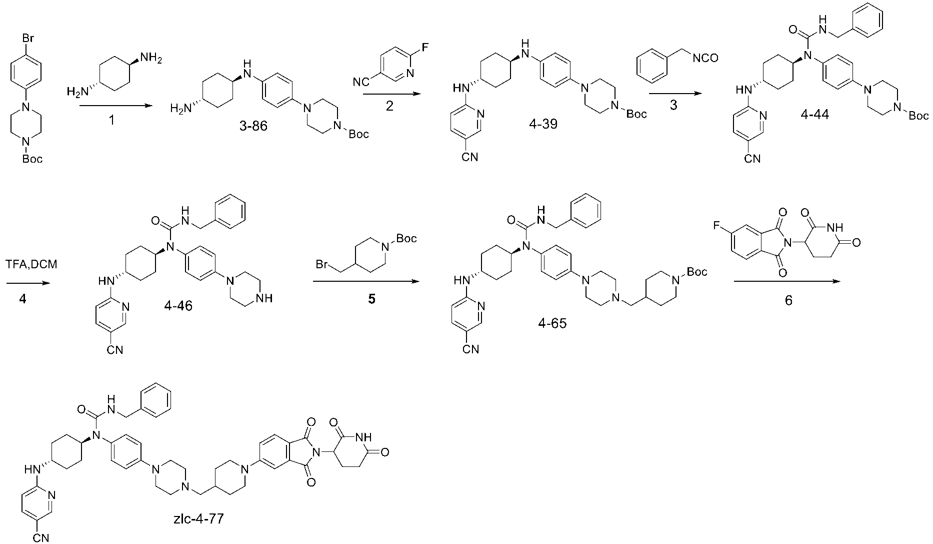

### Step 1: Preparation of intermediate 3-86

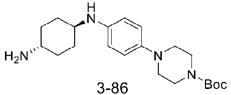

In a three-necked round-bottomed flask, to a system of tert-butyl 4-(4-bromophenyl)piperazin-1-carboxylate (1.02 g, 3mmol) in anhydrous DMSO (15 mL) were added trans-cyclohexane-1,4-diamine 2 (1.2 g, 10.5 mmol), potassium phosphate (1.3 g, 6 mmol), CuI (57 mg, 0.3 mmol), and D-proline (35 mg, 0.3 mmol) in sequence. After the completion, argon replacement was performed for three times, and the temperature was raised to 100°C. After the reaction was completed as monitored by TLC, the reaction was brought to room temperature, filtered through celite, and washed three times with a DCM/MeOH (10:1) system. The filtrate was concentrated under reduced pressure, wet-loaded, and purified by normal-phase silica gel column chromatography to obtain the target compound 3-86 (off-white solid, 480 mg, 43% yield). ¹H NMR (400 MHz, DMSO-*d*₆) δ 6.74 (d, *J =* 8.8 Hz, 2H), 6.49 (d, *J =* 8.9 Hz, 2H), 4.88 (d, *J =* 8.2 Hz, 1H), 3.42 (t, *J =* 5.1 Hz, 4H), 3.03 (s, 1H), 2.83 (t, *J =* 5.1 Hz, 4H), 2.76 (s, 1H), 1.95 (d, *J =* 12.8 Hz, 2H), 1.85 (d, *J =* 12.4 Hz, 2H), 1.41 (s, 9H), 1.26 (q, *J =* 10.9 Hz, 2H), 1.11 (q, *J =* 11.6 Hz, 2H). HRMS (ESI) for C₂₁H₃₄N₄O₂ [M+H]⁺, calcd: 375.2755, found: 375.2739.

### Step 2: Preparation of intermediate 4-39

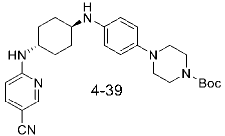

In a 100 mL round-bottomed flask, to a system of **3-86** (1.38 g, 3.7 mmol) in DMF (7.5 mL) were added 5-cyano-2-fluoropyridine 3 (451 mg, 3.7 mmol) and Cs₂CO₃ (1.45 g, 4.44 mol) in sequence. After the completion, the mixture was reacted overnight at room temperature. After the reaction was completed as monitored by TLC, the reaction was filtered, and the filtrate was concentrated under reduced pressure. Purification by normal-phase silica gel column chromatography obtained Intermediate 4-39 (white solid, 1.4 g, 80%). ¹H NMR (400 MHz, DMSO-*d*₆) δ 8.37 (s, 1H), 7.63 (d, *J =* 8.5 Hz, 1H), 7.51 (d, *J* = 1 Hz, 1H), 6.75 (d, *J =* 6.9 Hz, 2H), 6.52 (d, *J =* 8.3 Hz, 3H), 4.92 (s, 1H), 3.75 (s, 1H), 3.42 (s, 4H), 3.12 (s, 1H), 2.83 (s, 4H), 1.98 (s, 4H), 1.41 (s, 9H), 1.36 - 1.28 (m, 2H), 1.25 - 1.17 (m, 2H).

### Step 3: Preparation of intermediate 4-44

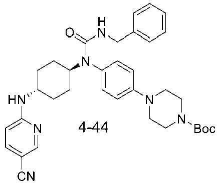

In a 25 mL round-bottomed flask, to a system of 4-39 (1.438 g, 3 mmol) in DMF (3 mL) were added benzyl isocyanate 4 (1.2 g, 9 mmol) and DIPEA (1.6 mL) in sequence. After the completion, the temperature was raised to 95°C. After 6 h, the reaction was completed as monitored by TLC. The reaction was brought to room temperature, concentrated under reduced pressure, and purified by normal-phase silica gel chromatography to obtain the target intermediate **4-44** (white solid, 1.3 g, 72% yield). ¹H NMR (400 MHz, DMSO-*d*₆) δ 8.29 (s, 1H), 7.60 (d, *J =* 9.0 Hz, 1H), 7.46 (d, *J =* 6.6 Hz, 1H), 7.28 - 7.24 (m, 2H), 7.18 - 7.14 (m, 3H), 7.03 - 6.98 (m, 4H), 6.46 (d, *J =* 8.7 Hz, 1H), 5.58-5.56 (m, 1H), 4.26 (t, *J* = 11.9 Hz, 1H), 4.14 (d, *J =* 5.4 Hz, 2H), 3.45 (s, 5H), 3.16 (s, 4H), 1.90 (d, *J* = 11.1 Hz, 2H), 1.76 (d, *J =* 11.6 Hz, 2H), 1.42 (d, *J =* 1.6 Hz, 9H), 1.27 (m, 2H), 1.14 - 1.04 (m, 2H).

### Step 4: Preparation of intermediate 4-46

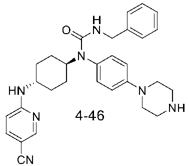

In a 25 mL round-bottomed flask, to a system of **4-44** (1.83 g, 3 mmol) in CH₂Cl₂ (2 mL) was added CF₃COOH (1 mL) dropwise. The mixture was stirred at room temperature overnight. After the reaction was completed, the reaction was concentrated under reduced pressure and extracted with a saturated sodium bicarbonate-CH₂Cl₂/MeOH (10:1) system. The resulting substance was dried over anhydrous sodium sulfate and purified by silica gel column chromatography to obtain the target intermediate **4-46** (white solid, 1.43 g, 94%). ¹H NMR (500 MHz, DMSO-*d*₆) δ 8.29 (s, 1H), 7.60 (d, *J =* 9.2 Hz, 1H), 7.48 (s, 1H), 7.28 - 7.25 (m, 2H), 7.18 - 7.15 (m, 3H), 7.00 - 6.95 (m, 4H), 6.47 (d, *J = 8.9* Hz, 1H), 5.56 (s, 1H), 4.26 (m, 1H), 4.14 (d, *J =* 5.9 Hz, 2H), 3.48 (s, 1H), 3.37 - 3.34 (m, 1H),3.11 - 3.09 (m, 4H), 2.85 - 2.83 (m, 4H), 1.90 (d, *J =* 11.4 Hz, 2H), 1.76 (d, *J =* 10.7 Hz, 2H), 1.33 - 1.27 (m, 2H), 1.13 - 1.06 (m, 2H).

### Step 5: Preparation of intermediate 4-65

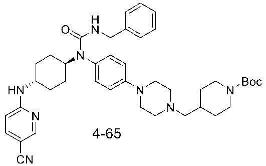

In a 25 mL round-bottomed flask, to a system of **4-46** (153 mg, 0.3 mmol) in DMF (2 mL) were added potassium carbonate (83 mg, 0.6 mmol) and 1-Boc-4-bromomethylpiperidine (167 mg, 0.6 mmol) in sequence. After the completion, the temperature was raised to 80°C, and the mixture was stirred overnight. After the reaction was completed, the reaction was concentrated under reduced pressure and purified by normal-phase silica gel column chromatography to obtain the target intermediate **4-65** (white solid, 110 mg, 52%). ¹H NMR (500 MHz, DMSO-*d*₆) δ 8.29 (d, *J* = 2.1 Hz, 1H), 7.60 (d, *J* = 8.7 Hz, 1H), 7.49 (d, *J* = 6.6 Hz, 1H), 7.28 - 7.26 (m, 2H), 7.18 - 7.14 (m, 3H), 7.03 - 6.96 (m, 4H), 6.46 (d, *J =* 8.8 Hz, 1H), 5.59 (*t, J =* 5.6 Hz, 1H), 4.28 - 4.23 (s, 1H), 4.14 - 4.13(d, *J =* 6.0 Hz, 2H), 3.92 (s, 2H), 3.50 (s, 1H), 3.17 (s, 4H), 2.47 (s, 4H), 2.17 (d, *J =* 6.6 Hz, 2H), 1.90 (d, *J =* 9.7 Hz, 2H), 1.77 - 1.68 (m, 5H), 1.39 (s, 9H), 1.33 - 1.26 (m, 2H), 1.13 - 1.08 (m, 2H), 0.98 - 0.94 (m, 2H), 0.84 - 0.81 (m, 2H). MS (ESI) for C₄₁H₅₅N₈O₃ [M+H]⁺, calcd: 707.4, found: 707.5.

### Step 6: Preparation of compound zlc-4-77

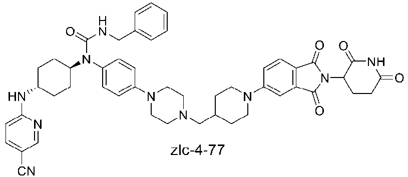

In a 25 mL round-bottomed flask, to a system of **4-65** (79 mg, 0.112 mmol) in CH₂Cl₂ (1 mL) was added CF₃COOH (0.3 mL) dropwise. The mixture was stirred at room temperature overnight. After the reaction was completed, and the solvent was concentrated under reduced pressure. DMF (2 mL), 2-(2,6-dioxo-piperidin-3-yl)-5-fluoro-isoindole-1,3-dione (31 mg, 0.112 mmol), and DIPEA (0.023 mL, 0.135 mmol) were added in sequence. The temperature was raised to 105°C. After the reaction was completed as monitored, the reaction was brought to room temperature, concentrated under reduced pressure, and purified by normal-phase silica gel chromatography to obtain the target product **zlc-4-77** (yellow solid, 30 mg, 32%). ¹H NMR (600 MHz, DMSO-*d*₆) δ 11.08 (s, 1H), 8.29 (d, *J =* 2.1 Hz, 1H), 7.65 (d, *J =* 8.5 Hz, 1H), 7.60 (d, *J =* 8.4 Hz, 1H), 7.47 (s, 1H), 7.31 (s, 1H), 7.28 - 7.25 (m, 2H), 7.23 (d, *J= 8.6* Hz, 1H), 7.18 - 7.15 (m, 3H), 7.01 - 6.97 (m, 4H), 6.51 - 6.44 (m, 1H), 5.57 (t, *J =* 5.2 Hz, 1H), 5.06 (dd, *J =* 12.8, 5.4 Hz, 1H), 4.26 (t, *J* = 11.8 Hz, 1H), 4.15 (d, *J =* 5.7 Hz, 2H), 4.05 (d, *J =* 12.7 Hz, 2H), 3.48 (s, 1H), 3.19 (s, 4H), 2.97 (t, *J =* 12.0 Hz, 2H), 2.93 - 2.84 (m, 1H), 2.63 - 2.53 (m, 2H), 2.52 - 2.50 (m, 4H), 2.20 (d, *J =* 6.1 Hz, 2H), 2.04 - 1.99 (m, 1H), 1.90 (d, *J =* 10.3 Hz, 3H), 1.82 (d, *J =* 12.5 Hz, 2H), 1.76 (d, *J =* 10.6 Hz, 2H), 1.35 - 1.28 (m, 2H), 1.17 - 1.05 (m, 4H). ¹³C NMR (151 MHz, DMSO) δ 172.81, 170.12, 167.64, 166.97, 159.25, 156.80, 155.01, 153.10, 150.15, 141.33, 134.05, 131.50, 128.15, 128.02, 126.69, 126.23, 125.01, 119.12, 117.58, 117.35, 115.34, 107.74, 93.99, 63.71, 53.21, 52.91, 48.73, 47.70, 47.27, 43.47, 32.49, 31.31, 30.98, 30.20, 29.64, 22.20, 14.09. HRMS (ESI) for C₄₉H₅₅N₁₀O₅ [M+H]⁺, calcd: 863.4351, found: 863.4360.

### Example 2: Preparation of compound zlc-4-79

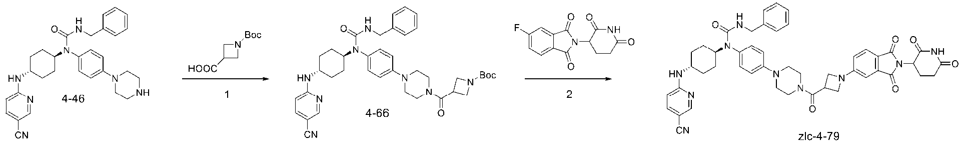

### Step 1: Preparation of intermediate 4-66

In a 25 mL round-bottomed flask, to a system of **4-46** (153 mg, 0.3 mmol) in DMF (2 mL) were added HATU (137 mg, 0.36 mmol), DIPEA (0.1 mL, 0.6 mmol), and 1-N-Boc-3-azetidinecarboxylic acid (67 mg, 0.33 mmol) were added in sequence . After the completion, the reaction was stirred at room temperature. After the reaction was completed as monitored, water was added to quench the reaction. The solid was precipitated and filtered, and the filter cake was washed (CH₂Cl₂/MeOH = 10:1), dried over anhydrous sodium sulfate, and purified by normal-phase silica gel chromatography to obtain the target intermediate **4-66** (white solid, 120 mg, 59%). ¹H NMR (500 MHz, DMSO-*d*₆) δ 8.29 (d, *J =* 2.1 Hz, 1H), 7.60 (d, *J =* 9.2 Hz, 1H), 7.48 (s, 1H), 7.28 - 7.25 (m, 2H), 7.18 - 7.14 (m, 3H), 7.03 - 6.99 (m, 4H), 6.46 (d, *J =* 8.9 Hz, 2H), 5.61 - 5.59 (m, 1H), 4.28 - 4.23 (m, 1H), 4.14 (d, *J =* 5.8 Hz, 2H), 3.72 - 3.68 (m, 1H), 3.63 - 3.60 (m, 4H), 3.40 (s, 2H), 3.19 - 3.17 (m, 3H), 3.16 - 3.12 (m, 4H), 1.90 (d, *J =* 10.9 Hz, 2H), 1.76 (d, *J =* 12.3 Hz, 2H), 1.37 (s, 9H), 1.32 - 1.27 (m, 2H), 1.13 - 1.06 (m, 2H). MS (ESI) for C₃₉H₄₉N₈O₄ [M+H]⁺, calcd: 693.4, found: 692.8.

### Step 2: Preparation of compound zlc-4-79

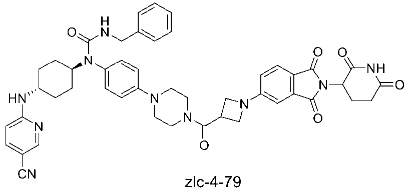

The synthesis method is similar to that of step f in Example 1.

¹H NMR (600 MHz, DMSO-*d*₆) δ 11.07 (s, 1H), 8.29 (d, *J =* 2.2 Hz, 1H), 7.66 (d, *J =* 8.3 Hz, 1H), 7.60 (d, *J =* 8.2 Hz, 1H), 7.51 - 7.44 (m, 1H), 7.28 - 7.26 (m, 2H), 7.19 - 7.15 (m, 3H), 7.05 - 7.01 (m, 4H), 6.84 (d, *J=* 1.8 Hz, 1H), 6.70 (dd, *J =* 8.4, 2.0 Hz, 1H), 6.47 (d, *J =* 8.9 Hz, 1H), 5.58 (t, *J =* 5.5 Hz, 1H), 5.06 (dd, *J =* 12.8, 5.4 Hz, 1H), 4.28 - 4.25 (m, 3H), 4.18 - 4.15 (m, 4H), 4.00 - 3.97 (m, 1H), 3.65 (s, 2H), 3.49 (s, 3H), 3.22 (m, 4H), 2.91 - 2.84 (m, 1H), 2.59 - 2.53 (m, 2H), 2.03 - 2.00 (m, 1H), 1.91 (d, *J =* 10.3 Hz, 2H), 1.77 (d, *J =* 10.6 Hz, 2H), 1.31 (m, 2H), 1.10 (m, 2H). ¹³C NMR (151 MHz, DMSO) δ 172.80, 170.10, 169.35, 167.43, 167.15, 159.24, 156.77, 154.98, 153.10, 149.92, 141.30, 133.80, 131.60, 128.76, 128.03, 126.71, 126.25, 124.83, 119.12, 117.24, 115.97, 114.30, 104.56, 94.00, 53.63, 52.92, 48.72, 48.07, 47.75, 44.42, 43.48, 41.30, 31.30, 31.21, 30.98, 30.20, 22.19, 14.09. HRMS (ESI) for C₄₇H₄₉N₁₀O₆ [M+H]⁺, calcd: 849.3831, found: 849.3839.

### Example 3: Preparation of compound zlc-4-91

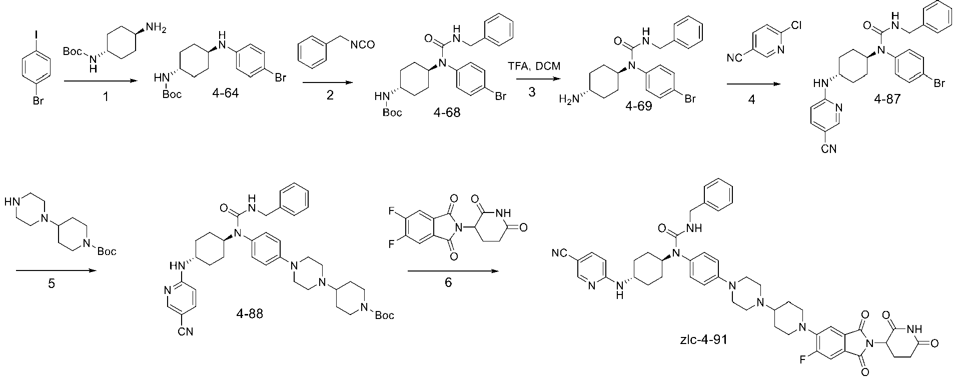

### Step 1: Preparation of intermediate 4-64

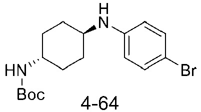

The compounds p-bromoiodobenzene (5.0 g, 17.7 mmol), N-BOC-trans-1,4-cyclohexanediamine (3.2 g, 14.7 mmol), Pd₂(dba)₃ (1.37 g, 1.5 mmol), Xantphos (1.7 g, 2.94 mmol) and tert-ButONa (2.8 g, 29.4 mmol) were mixed and dissolved in 150 mL toluene. After three times of argon replacement, the reaction was heated to 100°C overnight. After the reaction was completed, the reaction was filtered through celite and concentrated by rotary evaporation under reduced pressure. The residue was purified by column chromatography to obtain the target compound 4-64 (yellow solid, 3.9 g, 72% yield). ¹H NMR (500 MHz, DMSO-*d*₆) δ 7.16 - 7.13 (m, 2H), 6.79 (d, *J =* 7.9 Hz, 1H), 6.49 (dd, *J* = 8.9, 2.0 Hz, 2H), 5.63 (d, *J* = 8.0 Hz, 1H), 3.21 - 3.19 (m, 1H), 3.06 - 3.03 (m, 1H), 1.93 (d, *J* = 12.6 Hz, 2H), 1.77 (d, *J* = 12.5 Hz, 2H), 1.28 - 1.21 (m, 1H), 1.16 - 1.11 (m, 2H).

### Step 2: Preparation of intermediate 4-68

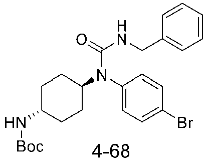

The intermediate **4-64** (3.9 g, 10.6 mmol) were dissolved in 4 mL DMF, and benzyl isocyanate (4.2 g, 31.7 mmol) and DIPEA (1.59 g, 12.3 mmol) were added. The mixture was reacted at 95°C for 5 h. The solvent was removed by rotary evaporation, and the residue was purified by column chromatography to obtain the target compound **4-68** (yellow solid, 3.7 g, 69% yield). ¹H NMR (500 MHz, DMSO-*d*₆) δ 7.64 - 7.62 (m, 2H), 7.27 - 7.25 (m, 2H), 7.19 - 7.15 (m, 3H), 7.12 - 7.10 (m, 2H), 6.69 (d, *J =* 7.9 Hz, 1H), 6.01 (t, *J =* 6.1 Hz, 1H), 4.24 - 4.16 (m, 1H), 4.13 (d, *J =* 6.1 Hz, 2H), 2.94 (m, 1H), 1.72 (d, *J =* 11.7 Hz, 4H), 1.34 (s, 9H), 1.24 - 1.21 (m, 2H), 1.03 - 0.96 (d, *J =* 12.1 Hz, 2H).

### Step 3: Preparation of intermediate 4-69

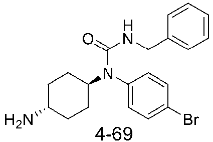

Intermediate **4-68** (3.7 g, 7.3 mmol) was dissolved in 5 mL DCM and 2.5 mL of trifluoroacetic acid (TFA) was added. After the mixture was heated to 55°C and refluxed for 6 h, the solvent was removed by rotary evaporation under reduced pressure and the residue was purified by column chromatography to obtain the target compound 4-69 (yellow solid, 2.5 g, yield 70%). ¹H NMR (500 MHz, DMSO-*d*₆) δ 7.65 - 7.63 (m, 2H), 7.28 - 7.25 (m, 2H), 7.19 - 7.11 (m, 5H), 6.04 (m, 1H), 4.23 - 4.18 (m, 1H), 4.12 (d, *J =* 5.3 Hz, 2H), 2.79 (m, 1H), 1.89 (d, *J =* 12.8 Hz, 2H), 1.79 (d, *J =* 12.2 Hz, 2H), 1.38 (m, 2H), 1.09 - 1.01 (m, 2H).

### Step 4: Preparation of intermediate 4-87

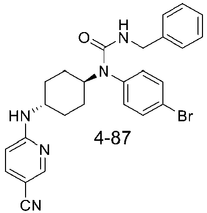

Compound **4-69** (2.5 g, 5.1 mmol) was dissolved in 15 mL DMF. 5-cyano-2-fluoropyridine (744 mg, 6.1 mol) and Cs₂CO₃ (2.0 g, 6.1 mol) were added, and the mixture was stirred for 15 min at room temperature and then heated to 60°C for 40 min. After the reaction was completed, the solvent was removed by rotary evaporation under reduced pressure and the residue was purified by column chromatography to obtain the target compound **4-87** (white solid, 1.9 g, yield 70%). ¹H NMR (500 MHz, DMSO-*d*₆) δ 8.30 (d, *J =* 2.2 Hz, 1H), 7.65 - 7.60 (m, 3H), 7.48 (d, *J =* 6.5 Hz, 1H), 7.29 - 7.26 (m, 2H), 7.19 - 7.13 (m, 5H), 6.47 (d, *J* = 8.9 Hz, 1H), 6.03 (t, *J =* 5.9 Hz, 1H), 4.30 - 4.23 (m, 1H), 4.14 (*d, J =* 6.0 Hz, 2H), 3.51 (s, 1H), 1.91 (*d, J =* 11.0 Hz, 1H), 1.78 (*d, J =* 11.4 Hz, 2H), 1.31 (m, 2H), 1.10 (m, 2H).

### Step 5: Preparation of intermediate 4-88

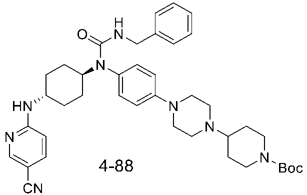

In a 25 mL Shrek flask, compound **4-87** (1.51 g, 3 mmol), tert-butyl 4-piperazin-1-ylpiperidin-1-carboxylate (986 mg, 3.6 mmol), Pd₂(dba)₃ (274 mg, 0.3 mmol), Xantphos (347 mg, 0.6 mmol) and tert-ButONa (576 g, 6 mmol) were added in sequence. Argon replacement was performed for three times, and toluene (6 mL) was added. The temperature was raised to 110°C, and the mixture was reacted overnight. After completion, the reaction was filtered through celite, concentrated under reduced pressure, and purified by column chromatography to obtain the target compound **4-88** (yellow solid, 1.27 g, 61%). ¹H NMR (500 MHz, DMSO-*d*₆) δ 8.29 (d, *J =* 1.7 Hz, 1H), 7.60 (d, *J =* 8.5 Hz, 1H), 7.48 (s, 1H), 7.28 - 7.25 (m, 2H), 7.18 - 7.14 (m, 3H), 7.00 - 6.95 (m, 5H), 6.46 (d, *J =* 9.0 Hz, 1H), 5.59 (s, 1H), 4.28 - 4.23 (m, 1H), 4.14 (d, *J =* 5.9 Hz, 2H), 3.95 (s, 2H), 3.16 (s, 4H), 2.73 (s, 1H), 2.61 (s, 4H), 2.40 - 2.37 (m, 1H), 1.90 (d, *J* = 10.8 Hz, 2H), 1.77 (m, 4H), 1.39 (s, 9H), 1.33 - 1.23 (m, 6H), 1.13 - 1.05 (m, 2H).

### Step 6: Preparation of compound zlc-4-91

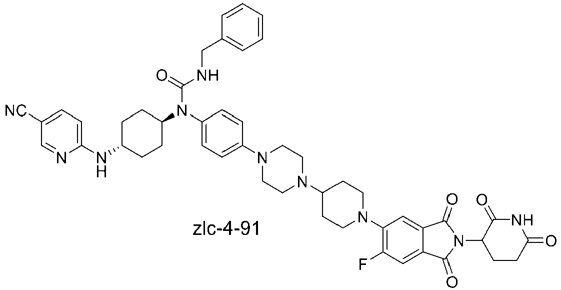

The synthesis method is similar to that of step f in Example 1.

¹H NMR (600 MHz, DMSO-*d*₆) δ 11.11 (s, 1H), 8.29 (d, *J* = 2.2 Hz, 1H), 7.71 (d, *J =* 11.4 Hz, 1H), 7.59 (d, *J =* 8.4 Hz, 1H), 7.45 (m, 2H), 7.28 - 7.25 (m, 2H), 7.18 - 7.15 (m, 3H), 7.01 - 6.96 (m, 4H), 6.46 (d, *J =* 8.9 Hz, 1H), 5.57 (m, 1H), 5.11 (dd, *J =* 12.9, 5.4 Hz, 2H), 4.28 - 4.26 (m, 1H), 4.15 (d, *J =* 5.7 Hz, 2H), 3.67 (d, *J* = 11.5 Hz, 2H), 3.49 (s, 1H), 3.19 (s, 4H), 2.93 - 2.86 (m, 3H), 2.66 (s, 4H), 2.61 - 2.58 (m, 1H), 2.54 - 2.52 (d, *J =* 13.2 Hz, 1H), 2.49 - 2.44 (d, *J =* 15.7 Hz, 1H), 2.04 - 2.02 (m, 1H), 1.93 - 1.90 (m, 4H), 1.76 (d, *J =* 10.4 Hz, 2H), 1.62 - 1.57 (m, 2H), 1.33 - 1.27 (m, 2H), 1.13 - 1.07 (d, 2H). ¹³C NMR (151 MHz, DMSO) δ 172.76, 169.91, 166.69, 166.21, 159.25, 158.09, 156.81, 156.41, 153.09, 150.15, 145.47, 141.34, 131.50, 128.77, 128.14, 128.01, 126.69, 126.22, 122.98, 119.11, 115.29, 113.72, 112.00, 111.83, 93.99, 59.76, 52.92, 49.33, 49.05, 48.77, 48.02, 43.48, 31.31, 30.96, 30.20, 27.88, 22.09, 14.09. HRMS (ESI) for C₄₈H₅₂N₁₀O₅ [M+H]⁺, calcd: 867.4101, found: 867.4104.

### Example 4: Preparation of compound zlc-4-93

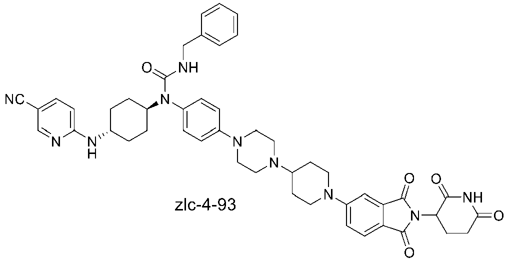

Refer to Example 1 for the synthesis method.

¹H NMR (600 MHz, DMSO-*d*₆) δ 11.08 (s, 1H), 8.29 (d, *J =* 2.2 Hz, 1H), 7.66 (d, *J =* 8.5 Hz, 1H), 7.60 (d, *J =* 8.2 Hz, 1H), 7.47 (s, 1H), 7.33 (s, 1H), 7.27 - 7.25 (m, 3H), 7.18 - 7.15 (m, 3H), 7.00 - 6.95 (m, 4H), 6.46 (d, *J =* 8.9 Hz, 1H), 5.58 - 5.56 (m, 1H), 5.08 - 5.05 (dd, *J =* 12.8, 5.5 Hz, 1H), 4.28 - 4.23 (m, 1H), 4.14 (d, *J =* 5.8 Hz, 2H), 4.09 (d, *J =* 12.5 Hz, 2H), 3.48 (s, 1H), 3.17 (s, 4H), 3.01 - 2.97 (m, 2H), 2.91 - 2.85 (m, 1H), 2.63 (s, 4H), 2.60 - 2.50 (m, 4H), 2.03 - 2.00 (m, 1H), 1.90 (d, *J =* 10.3 Hz, 4H), 1.76 (d, *J =* 10.5 Hz, 2H), 1.51 - 1.46 (m, 2H), 1.33 - 1.27 (m, 2H), 1.12 - 1.06 (m, 2H). ¹³C NMR (151 MHz, DMSO) δ 172.81, 170.11, 167.61, 166.96, 159.24, 156.80, 154.75, 153.09, 150.14, 141.34, 134.04, 131.49, 128.14, 128.01, 126.69, 126.22, 125.01, 119.12, 117.70, 115.30, 107.82, 93.98, 59.76, 52.90, 48.74, 48.00, 46.61, 43.47, 31.30, 30.98, 30.19, 27.21, 22.19, 14.09. HRMS (ESI) for C₄₈H₅₃N₁₀O₅ [M+H]⁺, calcd: 849.4195, found: 849.4199.

### Example 5: Preparation of compound zlc-5-6

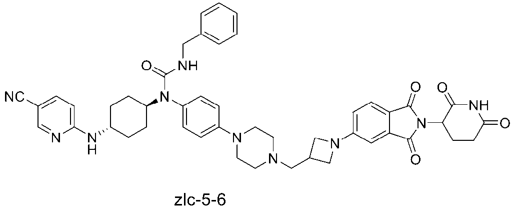

Refer to Example 1 for the synthesis method.

¹H NMR (500 MHz, DMSO-*d*₆) δ 11.07 (s, 1H), 8.29 (d, *J=* 2.2 Hz, 1H), 7.64 (d, *J =* 8.3 Hz, 1H), 7.60 (d, *J =* 10.2 Hz, 1H), 7.47 (d, *J* = 7.0 Hz, 1H), 7.28 - 7.25 (m, 2H), 7.18 - 7.15 (m, 3H), 7.02 - 6.97 (m, 4H), 6.78 (d, *J =* 1.5 Hz, 1H), 6.65 (dd, *J =* 8.4, 1.7 Hz, 1H), 6.47 (d, *J =* 8.8 Hz, 1H), 5.58 (m, 1H), 5.07 - 5.03 (m, 1H), 4.29 - 4.24 (m, 1H), 4.17 - 4.14 (m, 4H), 3.73 - 3.70 (m, 2H), 3.50 (s, 1H), 3.50-3.46 (m, 1H),3.19 (s, 4H), 3.05 (s, 1H), 2.90 - 2.84 (m, 1H), 2.66 (d, *J* = 5.4 Hz, 2H), 2.60 - 2.54 (m, 6H), 2.02 - 2.00 (m, 1H), 1.91 (d, *J* = 9.7 Hz, 2H), 1.77 (d, *J =* 10.2 Hz, 2H), 1.33 - 1.26 (m, 2H), 1.13 - 1.06 (m, 2H). ¹³C NMR (151 MHz, DMSO) δ 172.80, 170.11, 167.49, 167.18, 159.24, 156.80, 155.19, 153.10, 150.12, 141.34, 133.82, 131.50, 128.22, 128.02, 126.69, 126.23, 124.80, 119.12, 116.68, 115.42, 114.06, 104.35, 93.98, 61.76, 55.75, 52.85, 48.70, 47.63, 43.46, 31.30, 30.98, 30.20, 27.00, 22.21. HRMS (ESI) for C₄₇H₃₁N₁₀O₅ [M+H]⁺, calcd: 835.4038, found: 835.4043.

### Example 6: Preparation of compound zlc-5-11

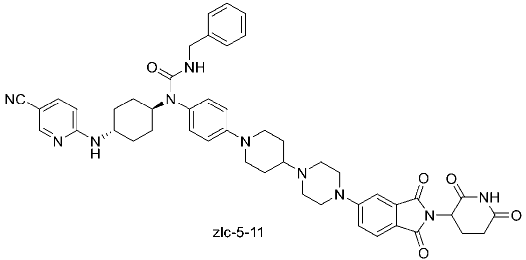

Refer to Example 1 for the synthesis method.

¹H NMR (500 MHz, DMSO-*d*₆) δ 11.09 (s, 1H), 8.31 - 8.28 (m, 1H), 7.68 (d, *J =* 8.7 Hz, 1H), 7.60 (d, *J =* 8.8 Hz, 1H), 7.49 (s, 1H), 7.34 (s, 1H), 7.28 - 7.25 (m, 3H), 7.18 - 7.15 (m, 3H), 6.99 (m, 4H), 6.46 (d, *J =* 8.8 Hz, 1H), 5.59 - 5.57 (m, 1H), 5.07 (dd, *J =* 12.8, 5.4 Hz, 1H), 4.28 - 4.14 (m, 1H), 4.14 (d, *J =* 5.6 Hz, 2H), 3.79 (d, *J* = 9.4 Hz, 2H), 3.44 (m, 4H), 2.92 - 2.85 (m, 1H), 2.75 - 2.70 (m, 2H), 2.65 (m, 4H), 2.60 - 2.56 (m, 2H), 2.41 - 2.36 (m, 1H), 2.03 - 2.00 (m, 1H), 1.91 (s, 4H), 1.76 (d, *J =* 11.0 Hz, 2H), 1.53 - 1.51 (m, 2H), 1.33 - 1.25 (m, 2H), 1.13 - 1.06 (m, 2H). ¹³C NMR (126 MHz, DMSO) δ 172.87, 170.14, 167.60, 167.02, 159.26, 156.85, 153.14, 150.15, 141.36, 133.87, 131.53, 128.06, 127.91, 126.71, 126.27, 124.94, 119.17, 115.82, 94.00, 59.81, 52.91, 48.79, 48.42, 47.47, 43.49, 31.34, 31.01, 30.24, 22.21, 14.13. HRMS (ESI) for C₄₈H₅₃N₁₀O₅ [M+H]⁺, calcd: 849.4195, found: 849.4192.

### Example 7: Preparation of compound zlc-5-15

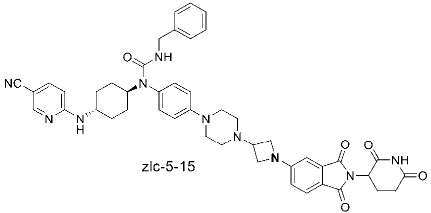

Refer to Example 1 for the synthesis method.

¹H NMR (500 MHz, DMSO-*d*₆) δ 11.09 (s, 1H), 8.29 (d, *J =* 2.3 Hz, 1H), 7.66 (d, *J =* 8.2 Hz, 1H), 7.60 (d, *J =* 8.9 Hz, 1H), 7.49 (s, 1H), 7.28 - 7.25 (m, 2H), 7.18 - 7.15 (m, 3H), 7.03 - 6.98 (m, 4H), 6.82 (s, 1H), 6.67 (d, *J =* 8.4 Hz, 1H), 6.46 (d, *J* = 8.9 Hz, 1H), 5.62 - 5.60 (m, 1H), 5.08 - 5.04 (m, 1H), 4.28 - 4.23 (m, 1H), 4.14 (d, *J =* 5.2 Hz, 4H), 3.92 (s, 2H), 3.39 (s, 1H), 3.23 (s, 4H), 2.91 - 2.84 (m, 1H), 2.64 - 2.51 (m, 6H), 2.02 - 2.01 (m, 1H), 1.90 (d, *J* = 11.6 Hz, 2H), 1.77 - 1.75 (m, 2H), 1.35 - 1.22 (m, 3H), 1.17 - 1.05 (m, 2H). ¹³C NMR (126 MHz, DMSO) δ 172.87, 170.17, 167.51, 167.20, 159.26, 156.83, 154.94, 153.14, 150.06, 141.37, 133.86, 131.57, 128.05, 126.72, 126.27, 124.91, 119.17, 116.92, 115.45, 114.27, 104.56, 94.00, 55.06, 54.19, 52.91, 49.16, 48.74, 47.40, 43.49, 31.33, 31.01, 30.23, 22.23, 14.13. HRMS (ESI) for C₄₆H₄₉N₁₀O₅ [M+H]⁺, calcd: 821.3882, found: 821.3884.

### Example 8: Preparation of compound zlc-5-20

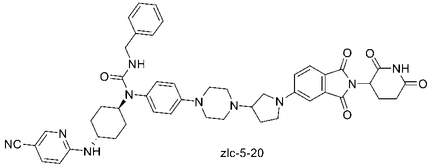

Refer to Example 1 for the synthesis method.

¹H NMR (500 MHz, DMSO-*d*₆) δ 11.08 (s, 1H), 8.29 (d, *J =* 1.8 Hz, 1H), 7.65 (d, *J =* 8.4 Hz, 1H), 7.60 (d, *J =* 8.6 Hz, 1H), 7.48 (d, *J =* 5.9 Hz, 1H), 7.28 - 7.25 (m, 2H), 7.19 - 7.15 (m, 3H), 7.01 - 6.98 (m, 5H), 6.86 (d, *J =* 8.6 Hz, 1H), 6.47 (d, *J =* 8.9 Hz, 1H), 5.59 (t, *J =* 5.7 Hz, 1H), 5.06 (dd, *J* = 12.8, 5.3 Hz, 1H), 4.29 - 4.23 (m, 1H), 4.15 (d, *J =* 5.7 Hz, 2H), 3.75 - 3.72 (m, 1H), 3.61 - 3.58 (m, 1H), 3.50 - 3.38 (m, 2H), 3.29 - 3.22 (m, 5H), 3.05 - 2.98 (m, 1H), 2.91 - 2.85 (m, 1H), 2.69 - 2.61 (m, 4H), 2.61 - 2.52 (m, 2H), 2.31 - 2.27 (m, 1H), 2.02 - 2.00 (m, 1H), 1.91 - 1.89 (m, 3H), 1.77 (d, *J =* 10.5 Hz, 2H), 1.33 - 1.27 (m, 2H), 1.13 - 1.06 (m, 2H). ¹³C NMR (126 MHz, DMSO) δ 172.89, 170.22, 167.75, 167.29, 159.27, 156.85, 153.15, 151.81, 150.13, 141.38, 134.04, 131.56, 128.29, 126.72, 126.27, 124.96, 119.18, 115.80, 115.48, 115.25, 105.63, 94.01, 63.55, 52.92, 52.03, 51.35, 48.71, 47.64, 47.05, 43.50, 31.34, 31.03, 30.24, 28.89, 22.29. HRMS (ESI) for C₄₇H₃₁N₁₀O₅ [M+H]⁺, calcd: 835.4038, found: 835.4036.

### Example 9: Preparation of compound zlc-5-31

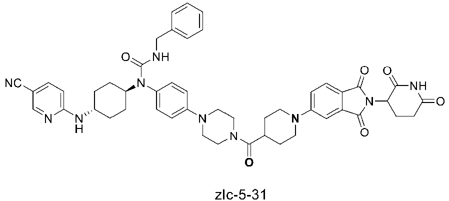

Refer to Example 1 for the synthesis method.

¹H NMR (500 MHz, DMSO-*d*₆) δ 11.10 (s, 1H), 8.30 (d, *J =* 1.8 Hz, 1H), 7.67 (d, *J =* 8.5 Hz, 1H), 7.60 (d, *J =* 8.5 Hz, 1H), 7.54 - 7.46 (m, 1H), 7.34 (s, 1H), 7.28 - 7.24 (m, 3H), 7.19 - 7.15 (m, 3H), 7.05 - 7.00 (m, 4H), 6.47 (d, *J* = 8.9 Hz, 1H), 5.60 - 5.59 (m, 1H), 5.07 (dd, *J =* 12.8, 5.3 Hz, 1H), 4.29 - 4.24 (m, 1H), 4.15 (d, *J =* 5.5 Hz, 2H), 4.08 (d, *J =* 12.6 Hz, 2H), 3.71 (s, 2H), 3.61 (s, 2H), 3.49 (s, 1H), 3.23 (s, 2H), 3.17 (s, 2H), 3.11 - 3.02 (m, 3H), 2.92 - 2.85 (m, 1H), 2.60 - 2.54 (m, 2H), 2.03 - 2.00 (m, 1H), 1.90 (d, *J =* 10.5 Hz, 2H), 1.78 - 1.73 (m, 4H), 1.67 - 1.60 (m, 2H), 1.33 - 1.27 (m, 2H), 1.13 - 1.06 (m, 2H). ¹³C NMR (151 MHz, DMSO) δ 172.81, 172.34, 170.10, 167.60, 166.96, 159.24, 156.77, 154.82, 153.09, 149.92, 141.30, 134.05, 131.60, 128.70, 128.02, 126.70, 126.24, 125.03, 119.11, 117.64, 115.88, 107.85, 93.99, 52.92, 48.73, 48.41, 47.84, 46.72, 44.62, 43.48, 41.07, 36.80, 31.30, 30.98, 30.20, 27.42, 22.19, 14.09. HRMS (ESI) for C₄₉H₅₃N₁₀O₆ [M+H]⁺, calcd: 877.4144, found: 877.4139.

### Example 10: Preparation of compound zlc-5-103

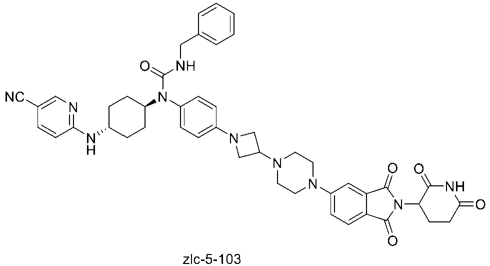

Refer to Example 1 for the synthesis method.

¹H NMR (600 MHz, DMSO-*d*₆) δ 11.08 (s, 1H), 8.30 - 8.28 (m, 1H), 7.68 (d, *J =* 8.5 Hz, 1H), 7.60 (d, *J =* 8.6 Hz, 1H), 7.47 (s, 1H), 7.36 (s, 1H), 7.28 - 7.25 (m, 3H), 7.18 - 7.15 (m, 3H), 6.97 (d, *J =* 8.6 Hz, 2H), 6.49 (d, *J =* 8.7 Hz, 2H), 6.46 (d, *J =* 9.0 Hz, 1H), 5.51 (t, *J =* 5.7 Hz, 1H), 5.07 (dd, *J =* 12.8, 5.4 Hz, 1H), 4.28 - 4.24 (m, 1H), 4.15 (d, *J =* 5.9 Hz, 2H), 3.98 (t, *J =* 7.1 Hz, 2H), 3.70 - 3.68 (m, 2H), 3.50 - 3.42 (m, 5H), 3.38 - 3.37 (m, 2H), 2.91 - 2.85 (m, 1H), 2.60 - 2.52 (m, 3H), 2.02 - 2.00 (m, 1H), 1.90 (d, *J =* 10.2 Hz, 2H), 1.76 (d, *J =* 10.6 Hz, 2H), 1.33 - 1.23 (m, 4H), 1.12 - 1.06 (m, 2H). ¹³C NMR (151 MHz, DMSO) δ 173.28, 170.55, 168.03, 167.45, 159.72, 157.38, 155.70, 153.58, 151.37, 141.79, 134.31, 131.92, 128.50, 127.19, 127.15, 126.71, 125.37, 119.59, 118.93, 118.37, 112.19, 108.49, 94.46, 56.10, 54.60, 53.37, 49.25, 49.10, 47.17, 43.94, 31.78, 31.45, 30.67, 22.65. HRMS (ESI) for C₄₆H₄₉N₁₀O₅ [M+H]⁺, calcd: 821.3882, found: 821.3884.

### Example 11: Preparation of compound zle-5-104

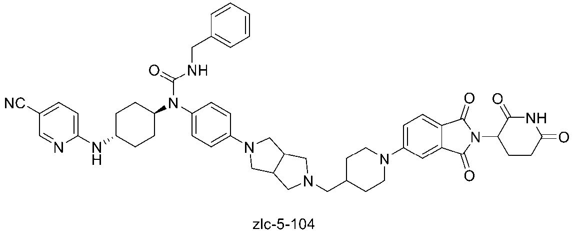

Refer to Example 1 for the synthesis method.

¹H NMR (600 MHz, DMSO-*d*₆) δ 11.07 (s, 1H), 8.29 (d, *J =* 2.3 Hz, 1H), 7.63 (d, *J =* 8.5 Hz, 1H), 7.60 (d, *J =* 8.3 Hz, 1H), 7.48 (s, 1H), 7.28 - 7.25 (m, 3H), 7.21 - 7.15 (m, 4H), 6.97 (d, *J =* 8.5 Hz, 2H), 6.67 (d, *J =* 8.4 Hz, 2H), 6.47 (d, *J = 8.9* Hz, 1H), 5.53 (s, 1H), 5.05 (dd, *J =* 12.8, 5.5 Hz, 1H), 4.29 - 4.25 (m, 1H), 4.15 (d, *J =* 5.8 Hz, 2H), 4.02 (d, *J =* 12.3 Hz, 2H), 3.41 (m, 3H), 3.10 (m, 2H), 2.96 - 2.84 (m, 5H), 2.59 - 2.53 (m, 4H), 2.43 (s, 1H), 2.28 - 2.23 (m, 2H), 2.01 - 1.97 (m, 2H), 1.91 - 1.90 (m, 2H), 1.79 - 1.75 (m, 5H), 1.31 - 1.27 (m, 2H), 1.24 - 1.23 (m, 2H), 1.13 - 1.09 (m, 2H). HRMS (ESI) for C₅₁H₃₇N₁₀O₅ [M+H]⁺, calcd: 889.4508, found: 889.4515.

### Example 12: Preparation of compound zlc-5-105

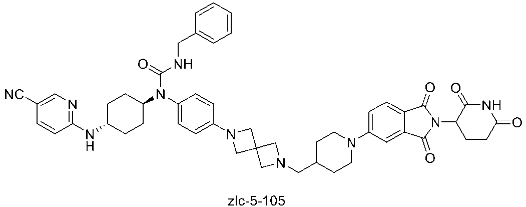

Refer to Example 1 for the synthesis method.

¹H NMR (600 MHz, DMSO-*d*₆) δ 11.08 (s, 1H), 8.29 (d, *J=* 2.3 Hz, 1H), 7.64 (d, *J =* 8.6 Hz, 1H), 7.60 (d, *J =* 8.0 Hz, 1H), 7.48 (s, 1H), 7.29 - 7.25 (m, 3H), 7.21 (dd, *J=* 8.7, 2.1 Hz, 1H), 7.18 - 7.14 (m, 3H), 6.95 (d, *J =* 8.6 Hz, 2H), 6.47 - 6.44 (m, 3H), 5.47 (t, *J* = 5.5 Hz, 1H), 5.06 (dd, *J =* 12.8, 5.5 Hz, 1H), 4.27 - 4.23 (m, 1H), 4.14 (d, *J* = 5.9 Hz, 2H), 4.01 (d, *J =* 12.9 Hz, 2H), 3.90 (s, 4H), 3.30 (s, 4H),2.95 - 2.91 (m, 2H), 2.91 - 2.85 (m, 1H), 2.60 - 2.52 (m, 2H), 2.27 (s, 1H), 2.02 - 2.00 (m, 1H), 1.90 (d, *J =* 12.1 Hz, 2H), 1.75 (d, *J =* 11.8 Hz, 4H), 1.30 - 1.24 (m, 4H), 1.17 - 1.11 (m, 2H), 1.08 - 1.06 (m, 2H).¹³C NMR (151 MHz, DMSO) δ 173.29, 170.59, 168.11, 167.43, 159.71, 157.35, 155.40, 153.57, 151.12, 141.74, 134.52, 131.89, 128.51, 125.49, 119.59, 118.02, 117.81, 112.31, 108.16, 94.46, 70.25, 64.88, 62.14, 53.35, 49.20, 47.71, 43.94, 34.71, 31.77, 31.45, 30.65, 29.90, 22.66. HRMS (ESI) for C₅₀H₅₅N₁₀O₅ [M+H]⁺, calcd: 875.4351, found: 875.4354.

### Example 13: Preparation of compound zlc-5-106

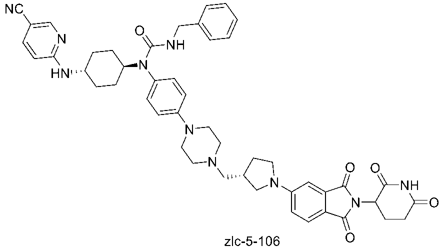

Refer to Example 1 for the synthesis method.

¹H NMR (600 MHz, DMSO-*d*₆) δ 11.07 (s, 1H), 8.29 (d, *J =* 2.2 Hz, 1H), 7.64 (d, *J =* 8.4 Hz, 1H), 7.60 (d, *J =* 8.7 Hz, 1H), 7.48 (s, 1H), 7.28 - 7.25 (m, 2H), 7.18 - 7.15 (m, 3H), 7.02 - 6.97 (m, 4H), 6.90 (d, *J=* 1.8 Hz, 1H), 6.81 (dd, *J =* 8.6, 2.1 Hz, 1H), 6.47 (d, *J* = 8.9 Hz, 1H), 5.57 (t, *J =* 5.6 Hz, 1H), 5.05 (dd, *J =* 12.7, 5.5 Hz, 1H), 4.28 - 4.23 (m, 1H), 4.15 (d, *J =* 5.9 Hz, 2H), 3.59 - 3.57 (m, 1H), 3.52 - 3.49 (m, 2H), 3.43 - 3.37 (m, 2H), 3.21 (s, 4H), 3.17 - 3.14 (m, 1H), 2.91 - 2.85 (m, 1H), 2.68 - 2.63 (m, 1H), 2.59 - 2.56 (m, 5H), 2.41 (d, *J =* 6.9 Hz, 2H), 2.18 - 2.14 (m, 1H), 2.02 - 1.99 (m, 1H), 1.90 (d, *J =* 10.4 Hz, 2H), 1.81 - 1.76 (m, 3H), 1.31 - 1.27 (m, 2H), 1.13 - 1.07 (m, 2H). ¹³C NMR (151 MHz, DMSO) δ 173.29, 170.63, 168.20, 167.71, 159.72, 157.29, 153.57, 152.35, 150.61, 141.80, 134.49, 131.98, 128.50, 127.16, 126.71, 125.44, 119.59, 115.96, 115.84, 115.73, 105.93, 94.46, 61.37, 53.56, 53.39, 52.68, 49.15, 48.14, 47.69, 43.94, 35.99, 31.78, 31.47, 30.67, 29.74, 22.73. HRMS (ESI) for C₄₈H₅₃N₁₀O₅ [M+H]⁺, calcd: 849.4195, found: 849.4200.

### Example 14: Preparation of compound zlc-6-1

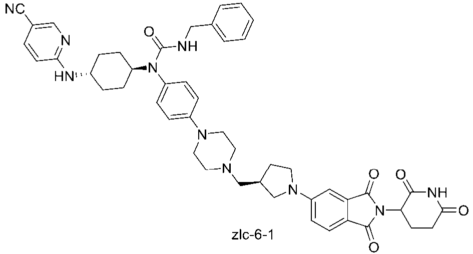

Refer to Example 1 for the synthesis method.

¹H NMR (600 MHz, DMSO-*d*₆) δ 11.06 (s, 1H), 8.29 (d, *J =* 2.2 Hz, 1H), 7.64 (d, *J =* 8.4 Hz, 1H), 7.60 (d, *J =* 8.5 Hz, 1H), 7.48 (s, 1H), 7.28 - 7.25 (m, 2H), 7.18 - 7.15 (m, 3H), 7.02 - 6.97 (m, 4H), 6.91 (d, *J =* 1.7 Hz, 1H), 6.82 (dd, *J =* 8.6, 2.0 Hz, 1H), 6.47 (d, *J =* 8.9 Hz, 1H), 5.58 - 5.56 (m, 1H), 5.05 (dd, *J =* 12.7, 5.5 Hz, 1H), 4.28 - 4.24 (m, 1H), 4.15 (d, *J=* 5.8 Hz, 2H), 3.60 - 3.57 (m, 1H), 3.53 - 3.49 (m, 2H), 3.43 - 3.39 (m, 2H), 3.21 (s, 4H), 3.17 - 3.15 (m, 1H), 2.91 - 2.85 (m, 1H), 2.67 - 2.64 (m, 1H), 2.59 - 2.52 (m, 5H), 2.42 (d, *J =* 7.0 Hz, 2H), 2.18 - 2.15 (m, 1H), 2.02 - 1.96 (m, 1H), 1.90 (d, *J =* 10.3 Hz, 2H), 1.81 - 1.76 (m, 3H), 1.31 - 1.29 (m, 2H), 1.13 - 1.07 (m, 2H). ¹³C NMR (151 MHz, DMSO) δ 173.29, 170.63, 168.20, 167.71, 159.72, 157.29, 153.58, 152.35, 150.61, 141.80, 134.49, 131.98, 128.50, 127.16, 126.71, 125.45, 119.59, 115.96, 115.84, 115.73, 105.93, 94.46, 61.37, 53.57, 53.39, 52.68, 49.15, 48.14, 47.69, 43.94, 35.99, 31.78, 31.46, 30.67, 29.74, 22.56. HRMS (ESI) for C₄₈H₅₃N₁₀O₅ [M+H]⁺, calcd: 849.4195, found: 849. 4191.

### Example 15: Preparation of compound zlc-6-35

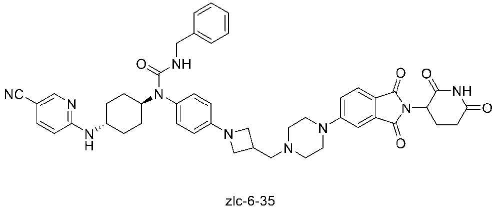

Refer to Example 1 for the synthesis method.

¹H NMR (600 MHz, DMSO-*d*₆) δ 11.08 (s, 1H), 8.29 (d, *J =* 2.2 Hz, 1H), 7.68 (d, *J =* 8.5 Hz, 1H), 7.60 (d, *J =* 8.9 Hz, 1H), 7.47 (s, 1H), 7.36 - 7.34 (m, 1H), 7.28 - 7.25 (m, 3H), 7.18 - 7.14 (m, 3H), 6.95 (d, *J =* 8.5 Hz, 2H), 6.47 - 6.45 (m, 3H), 5.48 - 5.47 (m, 1H), 5.07 (dd, *J =* 12.8, 5.4 Hz, 1H), 4.27 - 4.23 (m, 1H), 4.14 (d, *J =* 5.9 Hz, 2H), 3.97 (m, 2H), 3.54 - 3.50 (m, 2H), 3.45 (s, 4H), 3.00 - 2.95 (m, 1H), 2.64 (d, *J =* 7.3 Hz, 2H), 2.61 - 2.57 (m, 2H), 2.54 - 2.52 (m, 5H), 2.03 - 1.99 (m, 1H), 1.90 (d, *J =* 10.4 Hz, 2H), 1.75 (d, *J =* 11.6 Hz, 2H), 1.31 - 1.27 (m, 2H), 1.11 - 1.05 (m, 2H). ¹³C NMR (151 MHz, DMSO) δ 172.82, 170.09, 167.57, 166.99, 159.25, 156.92, 155.24, 153.11, 150.90, 141.29, 133.87, 131.44, 128.05, 126.70, 126.49, 124.91, 119.13, 118.35, 117.85, 111.48, 107.93, 94.00, 56.11, 52.87, 52.34, 48.78, 46.88, 43.48, 31.30, 30.98, 30.20, 29.03, 27.22. HRMS (ESI) for C₄₇H₅₁N₁₀O₃ [M+H]⁺, calcd: 835.4038, found: 835.4045.

### Example 16: Preparation of compound zlc-6-37

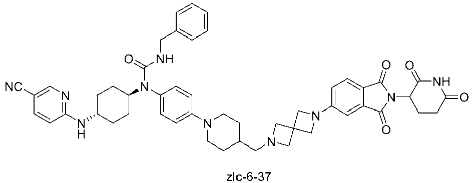

Refer to Example 1 for the synthesis method.

¹H NMR (600 MHz, DMSO-*d*₆) δ 11.07 (s, 1H), 8.29 (d, *J =* 2.2 Hz, 1H), 7.64 (d, *J =* 8.3 Hz, 1H), 7.60 (d, *J =* 7.8 Hz, 1H), 7.47 (s, 1H), 7.28 - 7.25 (m, 2H), 7.18 - 7.14 (m, 3H), 6.99 - 6.94 (m, 4H), 6.79 (s, 1H), 6.65 - 6.63 (dd, *J = 8*.4*,* 1.9 Hz, 1H), 6.47 - 6.46 (m, 1H), 5.54 (t, *J =* 5.7 Hz, 1H), 5.05 (dd, *J =* 12.8, 5.4 Hz, 1H), 4.28 - 4.22 (m, 1H), 4.14 (d, *J =* 5.9 Hz, 2H), 4.10 (s, 4H), 3.71 (d, *J =* 12.1 Hz, 2H), 3.36 (m, 2H), 2.90 - 2.84 (m, 1H), 2.67 - 2.63 (m, 2H), 2.59 - 2.56 (m, 1H), 2.56 - 2.52 (m, 2H), 2.48 - 2.46 (m, 2H), 2.02 - 1.98 (m, 1H), 1.90 (d, *J =* 10.3 Hz, 2H), 1.76 (d, *J =* 11.9 Hz, 4H), 1.45 - 1.38 (m, 1H), 1.33 - 1.26 (m, 2H), 1.24 - 1.17 (m, 4H), 1.13 - 1.06 (m, 2H). ¹³C NMR (151 MHz, DMSO) δ 173.28, 170.57, 167.96, 167.64, 159.72, 157.30, 155.34, 153.57, 151.04, 141.79, 134.26, 131.92, 128.50, 128.23, 127.15, 126.71, 125.28, 119.59, 117.46, 116.27, 114.92, 105.17, 94.46, 64.77, 61.69, 53.38, 49.19, 48.61, 43.94, 34.69, 31.77, 31.45, 30.67, 22.67. HRMS (ESI) for C₅₀H₅₅N₁₀O₅ [M+H]⁺, calcd: 875.4351, found: 875.4349.

### Example 17: Preparation of compound zlc-6-38

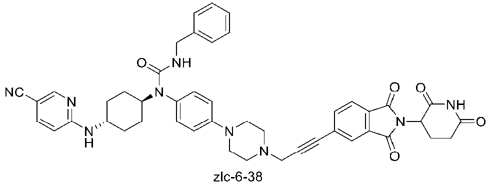

Refer to Example 1 for the synthesis method.

¹H NMR (600 MHz, DMSO-*d*₆) δ 11.14 (s, 1H), 8.29 (d, *J =* 2.2 Hz, 1H), 7.92 (d, *J =* 2.9 Hz, 3H), 7.60 (d, *J =* 8.9 Hz, 1H), 7.47 (s, 1H), 7.28 - 7.25 (m, 2H), 7.18 - 7.15 (m, 3H), 7.02 - 6.99 (s, 4H), 6.46 (d, *J=* 8.9 Hz, 1H), 5.57 (*t, J =* 5.8 Hz, 1H), 5.16 (dd, *J* = 12.9, 5.4 Hz, 1H), 4.28 - 4.23 (m, 1H), 4.14 (d, *J =* 6.0 Hz, 2H), 3.68 (s, 2H), 3.39 - 3.36 (m, 1H), 3.27 3.22 (m, 4H), 2.92 -2.86 (m, 1H), 2.72 -2.69(m, 4H), 2.62 - 2.59 (m, 1H), 2.56 -2.53 (m, 1H), 2.08 -2.04 (m, 1H), 1.93 - 1.87 (m, 2H), 1.76 (d, *J =* 11.4 Hz, 2H), 1.32 - 1.26 (m, 2H), 1.12 -1.06 (m, 2H). ¹³C NMR (151 MHz, DMSO) δ 172.75, 169.76, 166.50, 166.42, 156.80, 153.11, 150.14, 141.33, 137.58, 131.83, 131.52, 130.28, 128.77, 128.32, 128.03, 126.70, 126.24, 125.74, 123.83, 119.13, 115.55, 93.99, 90.49, 83.72, 52.91, 51.46, 49.12, 47.62, 46.75, 43.47, 31.31, 30.92, 30.20, 21.93. HRMS (ESI) for C₄₆H₄₆N₉O₅ [M+H]⁺, calcd: 804.3616, found: 804.3623.

### Example 18: Preparation of compound zlc-6-42

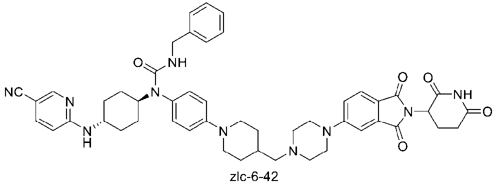

Refer to Example 1 for the synthesis method.

¹H NMR (600 MHz, DMSO-*d*₆) δ 11.08 (s, 1H), 8.29 (d, *J =* 2.4, 1H), 7.68 (d, *J =* 8.5 Hz, 1H), 7.60 (d, *J* = 9.0 Hz, 1H), 7.47 (s, 1H), 7.34 (d, *J =* 2.2 Hz, 1H), 7.28 - 7.25 (m, 3H), 7.18 - 7.15 (m, 3H), 6.99 - 6.96 (m, 4H), 6.47 (d, *J =* 8.9 Hz, 1H), 5.55 (t*, J* = 6.0 Hz, 1H), 5.07 (dd, *J =* 12.8, 5.4 Hz, 1H), 4.28 - 4.23 (m, 1H), 4.15 (d, *J* = 6.1 Hz, 2H), 3.74 (d, *J =* 11.8 Hz, 2H), 3.44 (s, 4H), 3.42 - 3.36 (m, 4H), 2.90 - 2.85 (m, 1H), 2.72 - 2.68 (m, 2H), 2.61 - 2.54 (m, 2H), 2.23 (d, *J* = 7.2 Hz, 2H), 2.03 - 1.99 (m, 1H), 1.90 (d, *J=* 11.6 Hz, 2H), 1.84 (d, *J=* 12.1 Hz, 2H), 1.77 - 1.72 (m, 3H), 1.31 - 1.28 (m, 2H), 1.25 - 1.20 (m, 4H), 1.13 - 1.07 (m, 2H). ¹³C NMR (151 MHz, DMSO) δ 172.83, 170.10, 167.58, 167.00, 159.26, 156.85, 155.27, 153.11, 150.62, 141.33, 133.87, 131.46, 128.04, 127.74, 126.70, 126.25, 124.92, 119.14, 118.32, 117.78, 115.80, 107.89, 94.00, 63.74, 52.91, 52.76, 48.78, 48.12, 46.96, 43.48, 32.44, 31.32, 30.99, 30.32, 30.21, 22.19. HRMS (ESI) for C₄₉H₅₅N₁₀O₃ [M+H]⁺, calcd: 863.4351, found: 863.4357.

### Example 19: Preparation of compound zlc-6-101

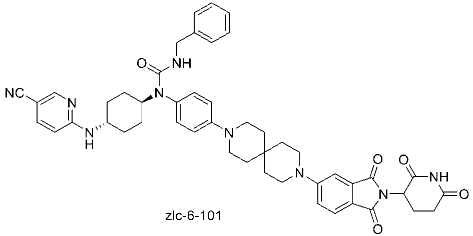

Refer to Example 1 for the synthesis method.

¹H NMR (600 MHz, DMSO-*d*₆) δ 11.07 (s, 1H), 8.29 (d, *J =* 2.1 Hz, 1H), 7.66 (d, *J =* 8.5 Hz, 1H), 7.60 (d, *J =* 8.7 Hz, 1H), 7.48 (s, 1H), 7.32 (s, 1H), 7.28 - 7.23 (m, 3H), 7.18 - 7.15 (m, 3H), 6.99 (s, 4H), 6.50 - 6.43 (m, 1H), 5.54 - 5.52 (m, 1H), 5.06 (dd, *J =* 12.8, 5.5 Hz, 1H), 4.28 - 4.24 (m, 1H), 4.15 (d, *J =* 5.8 Hz, 2H), 3.51 (s, 6H), 3.22 (s, 4H), 2.91 - 2.85 (m, 1H), 2.60 - 2.57 (m, 2H), 2.03 - 1.99 (m, 1H), 1.91 (d, *J =* 10.6 Hz, 2H), 1.77 (d, *J =* 10.4 Hz, 2H), 1.63 -1.59 (m, 8H), 1.33 - 1.28 (m, 2H), 1.14 - 1.07 (m, 2H). ¹³C NMR (151 MHz, DMSO) δ 172.83, 170.14, 167.67, 167.00, 159.25, 156.84, 154.97, 153.11, 141.31, 134.04, 131.46, 128.05, 127.66, 126.70, 126.26, 125.00, 119.13, 117.35, 115.47, 107.50, 94.00, 69.79, 52.90, 48.74, 43.48, 42.89, 34.82, 34.12, 31.31, 30.99, 30.21, 29.14, 22.21. HRMS (ESI) for C₄₈H₅₂N₉O₅ [M+H]⁺, calcd: 834.4086, found: 834.4073.

### Example 20: Preparation of compound zlc-6-102

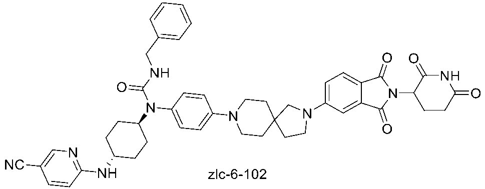

Refer to Example 1 for the synthesis method.

¹H NMR (600 MHz, DMSO-*d*₆) δ 11.06 (s, 1H), 8.30 (d, *J =* 2.2 Hz, 1H), 7.65 (d, *J =* 8.4 Hz, 1H), 7.60 (d, *J =* 8.2 Hz, 1H), 7.48 - 7.45 (m, 1H), 7.28 - 7.26 (m, 2H), 7.19 - 7.15 (m, 3H), 7.02 - 6.99 (m, 4H), 6.96 (s, 1H), 6.83 (dd, *J =* 8.6*,* 1.6 Hz, 1H), 6.47 (d, *J =* 8.9 Hz, 1H), 5.52 (t, *J =* 5.6 Hz, 1H), 5.07 - 5.04 (m, 1H), 4.29 - 4.24 (m, 1H), 4.15 (d, *J =* 5.9 Hz, 2H), 3.53 - 3.50 (m, 2H), 3.37 (m, 4H), 3.24 - 3.21 (m, 1H), 2.91 - 2.85 (m, 1H), 2.61 - 2.52 (m, 4H), 2.02 - 1.98 (m, 1H), 1.97 - 1.95 (m, 2H), 1.91 (d, *J =* 10.7 Hz, 2H), 1.77 (d, *J =* 10.3 Hz, 2H), 1.73 - 1.66 (m, 4H), 1.33 - 1.27 (m, 2H), 1.13 - 1.07 (m, 2H). ¹³C NMR (151 MHz, DMSO) δ 172.83, 170.16, 167.74, 167.26, 159.25, 156.82, 153.12, 152.06, 150.42, 141.28, 134.02, 131.48, 128.05, 127.83, 126.70, 126.27, 124.94, 119.13, 115.80, 115.54, 115.22, 105.58, 94.00, 57.28, 52.90, 48.69, 46.32, 45.44, 43.49, 35.02, 34.06, 31.31, 31.00, 30.22, 22.26. HRMS (ESI) for C₄₇H₅₀N₉O₅ [M+H]⁺, calcd: 820.3929, found: 820.3933.

### Example 21: Preparation of compound zlc-6-103

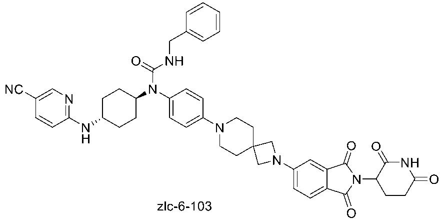

Refer to Example 1 for the synthesis method.

¹H NMR (600 MHz, DMSO-*d*₆) δ 11.07 (s, 1H), 8.30 (d, *J=* 2.2 Hz, 1H), 7.65 (d, *J =* 8.3 Hz, 1H), 7.60 (d, *J =* 8.1 Hz, 1H), 7.48 (s, 1H), 7.28 - 7.26 (m, 2H), 7.19 - 7.15 (m, 3H), 7.06 - 7.03 - 6.99 (m, 4H), 6.80 (d, *J =* 1.8 Hz, 1H), 6.67 (dd, *J =* 8.4, 2.0 Hz, 1H), 6.47 (d, *J =* 8.8 Hz, 1H), 5.56 (t*, J* = 5.6 Hz, 1H), 5.05 (dd, *J =* 12.8, 5.5 Hz, 1H), 4.29 - 4.23 (m, 1H), 4.15 (d, *J =* 5.8 Hz, 1H), 3.83 (m, 4H), 3.43 - 3.41 (m, 2H), 3.22 (m, 3H), 2.91 - 2.85 (m, 1H), 2.59 - 2.53 (m, 2H), 2.02 - 1.89 (m, 1H), 1.91 - 1.89 (m, 6H), 1.77 (d, *J =* 10.1 Hz, 2H), 1.33 - 1.27 (m, 2H), 1.13 - 1.07 (m, 2H). ¹³C NMR (151 MHz, DMSO) δ 172.83, 170.12, 167.53, 167.21, 159.26, 156.83, 155.20, 153.12, 150.21, 141.33, 133.86, 131.53, 128.04, 126.71, 126.26, 124.87, 122.51, 119.14, 116.69, 115.95, 114.18, 104.42, 94.00, 60.78, 52.92, 48.73, 45.32, 43.49, 34.65, 34.21, 31.32, 30.99, 30.21, 22.23, 20.45. HRMS (ESI) for C₄₆H₄₈N₉O₅ [M+H]⁺, calcd: 806.3773, found: 806.3767.

### Example 22: Preparation of compound zlc-7-36

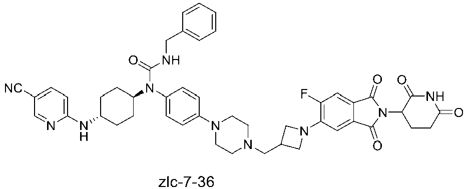

Refer to Example 1 for the synthesis method.

¹H NMR (600 MHz, DMSO-*d*₆) δ 11.08 (s, 1H), 8.29 (d, *J =* 2.2 Hz, 1H), 7.60 - 7.58 (m, 2H), 7.51 - 7.44 (m, 1H), 7.29 - 7.25 (m, 2H), 7.18 - 7.15 (m, 3H), 7.01 - 6.97 (m, 4H), 6.91 (d, *J =* 7.6 Hz, 1H), 6.46 (d, *J=* 8.9 Hz, 1H), 5.57 (t*, J* = 5.6 Hz, 1H), 5.06 (dd, *J* = 12.9, 5.4 Hz, 1H), 4.30 - 4.24 (m, 3H), 4.15 (d, *J =* 5.8 Hz, 2H), 3.87 - 3.85 (m, 2H), 3.53 - 3.44 (m, 1H), 3.19 (s, 4H), 3.04 - 3000 (m, 1H), 2.90 - 2.84 (m, 1H), 2.67 (d, *J =* 6.8 Hz, 2H), 2.61 - 2.51 (m, 6H), 2.03 - 1.99 (m, 1H), 1.90 (d, *J =* 10.5 Hz, 2H), 1.76 (d, *J =* 10.6 Hz, 2H), 1.31 - 1.27 (m, 2H), 1.12 - 1.07 (m, 2H). ¹³C NMR (151 MHz, DMSO) δ 172.80, 170.02, 166.84, 166.43, 159.26, 156.83, 154.43, 153.11, 152.79, 144.21, 144.13, 141.34, 131.52, 129.38, 128.23, 128.04, 126.70, 119.14, 118.30, 115.43, 111.33, 111.19, 107.95, 94.00, 61.57, 57.75, 52.93, 52.83, 48.94, 47.64, 43.48, 31.32, 30.97, 30.21, 27.85, 22.17, 22.08, 13.99. HRMS (ESI) for C₄₇H₅₀FN₁₀O₅ [M+H]⁺, calcd: 853.3944, found: 853.3956.

### Example 23: 3-benzyl-1-(4-(2-((2-(2,6-dioxopiperidin-3-yl)-1,3-dioxoisoindol-4-yl)oxy)acetyl)piperazin-1-yl)phenyl)-1-((1r,4r)-4-(quinazolin-2-ylamino)cyclohexyl)urea (YJZ9069)

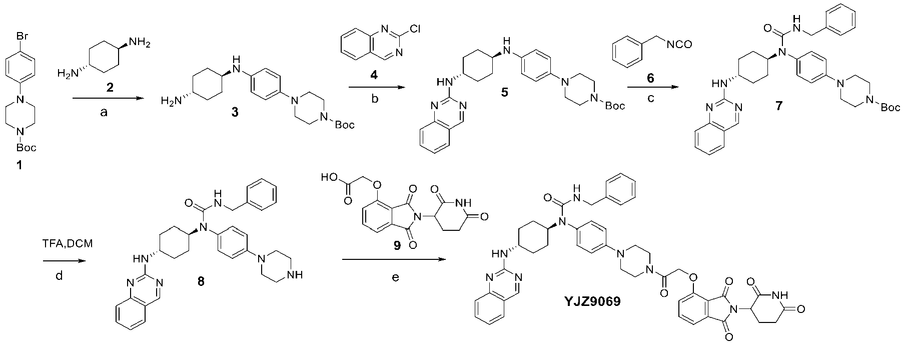

### Step 1: Preparation of tert-butyl 4-(4-(((1r,4r)-4-aminocyclohexyl)amino)phenyl)piperazin-1- carboxylate (compound 3)

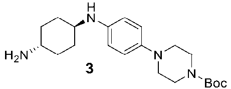

Potassium phosphate (31 g, 146 mmol), trans-cyclohexan-1,4-diamine 2 (29.3 g, 256.4 mmol), tert-butyl 4-(4-bromophenyl)piperazin-1-carboxylate 1 (25 g, 73.26 mmol), CuI (1.39 g 7.3 mmol) and D-proline (843 mg, 7.3 mmol) were dissolved in anhydrous DMSO (500 mL). The resultant suspension was protected with argon gas and argon replacement was performed for three times. The reaction mixture was then heated and stirred at 100°C for 10 h, and filtered through celite, and the residue was washed for 2-3 times with ethyl acetate. The filtrate was evaporated under reduced pressure and purified by silica gel column to obtain 12 g of the target compound as an off-white solid (yield 44%). ¹H NMR (400 MHz, DMSO-*d*₆) δ 6.74 (d, *J =* 8.8 Hz, 2H), 6.49 (d, *J =* 8.9 Hz, 2H), 4.88 (d, *J=* 8.2 Hz, 1H), 3.42 (t*, J* = 5.1 Hz, 4H), 3.03 (s, 1H), 2.83 (t*, J* = 5.1 Hz, 4H), 2.76 (s, 1H), 1.95 (d, *J =* 12.8 Hz, 2H), 1.85 (d, *J* = 12.4 Hz, 2H), 1.41 (s, 9H), 1.26 (q, *J =* 10.9 Hz, 2H), 1.11 (q, *J =* 11.6 Hz, 2H). HRMS (ESI) for C₂₁H₃₄N₄O₂ [M+H]⁺, calcd: 375.2755, found: 375.2739.

### Step 2: Preparation of tert-butyl 4-(4-(((1r,4r)-4-(quinazolin-2-ylamino)cyclohexyl)amino)phenyl)piperazin-1-carboxylate (Compound 5)

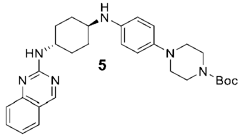

To a solution of tert-butyl 4-(4-(((1r,4r)-4-aminocyclohexyl)amino)phenyl)piperazin-1-carboxylate **3** (9 g, 24 mmol) in DMF (40 mL) were added 2-chloroquinazoline **4** (4 g, 24 mol) and Cs₂CO₃ (9.4 g, 28.9 mol). The mixture was stirred at room temperature for 15 min and then heated to 60°C for 40 min. The reaction was then filtered, and the filtrate was concentrated by rotary evaporation under reduced pressure. The residue was purified by column chromatography to obtain 11.2 g of an off-white solid (yield 93%). ¹H NMR (400 MHz, DMSO-*d*₆) δ 9.09 (s, 1H), 7.77 (d, *J =* 8.0 Hz, 1H), 7.67 (t, *J =* 7.7 Hz, 1H), 7.45 (d, *J =* 8.5 Hz, 1H), 7.29 (d, *J =* 8.0 Hz, 1H), 7.20 (t, *J =* 7.4 Hz, 1H), 6.76 (d, *J =* 8.2 Hz, 2H), 6.52 (d, *J =* 8.3 Hz, 2H), 4.95 (s, 1H), 3.86 (d, *J =* 9.8 Hz, 1H), 3.43 (t, *J =* 5.0 Hz, 4H), 3.18 - 3.01 (m, 1H), 2.84 (s, 4H), 2.01 (d, *J =* 11.6 Hz, 4H), 1.50 - 1.32 (m, 11H), 1.30 - 1.15 (m, 2H).

### Step 3: Preparation of tert-butyl 4-(4-(3-benzyl-1-((1r,4r)-4-(quinazolin-2-ylamino) cyclohexyl)ureido)phenyl)piperazin-1-carboxylate (Compound 7)

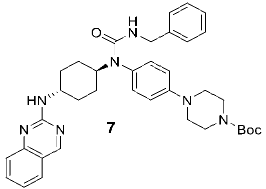

Tert-butyl 4-(4-(((1r,4r)-4-(quinazolin-2-ylamino)cyclohexyl)amino)phenyl)piperazin-1-carboxylate **5** (11.2 g, 22.25 mmol), DIPEA (8.6 g, 66.76 mmol) and benzyl isocyanate **6** (8.9 g, 66.76 mmol) were dissolved in 15 mL DMF. The mixture was stirred at 95°C for 4 h. The solvent was removed under reduced pressure and the residue was purified by column chromatography to obtain 10.2 g of a white solid (yield 72%). ¹H NMR (400 MHz, DMSO-*d*₆) δ 9.05 (s, 1H), 7.74 (d, *J =* 8.0 Hz, 1H), 7.63 (t, *J=* 7.8 Hz, 1H), 7.40 (d, *J =* 8.8 Hz, 1H), 7.31 - 7.22 (m, 3H), 7.21 - 7.13 (m, 4H), 7.08 - 6.98 (m, 4H), 5.58 (t, *J =* 6.1 Hz, 1H), 4.28 (t, *J =* 12.3 Hz, 1H), 4.16 (d, *J =* 5.8 Hz, 2H), 3.58 (s, 1H), 3.47 (t, *J* = 5.1 Hz, 4H), 3.18 (t, *J =* 5.2 Hz, 4H), 1.96 *(d, J=* 12.0 Hz, 2H), 1.79 (d, *J =* 12.3 Hz, 2H), 1.50 - 1.33 (m, 12H), 1.13 (q, *J =* 13.5, 12.5 Hz, 2H).

### Step 4: Preparation of 3-benzyl-1-(4-(piperazin-1-yl)phenyl)-1-(1r,4r)-4-(quinazolin-2-ylamino)cyclohexyl)urea (Compound 8)

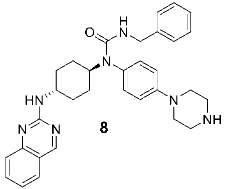

Tert-butyl 4-(4-(3-benzyl-1-((1r,4r)-4-(quinazolin-2-ylamino)cyclohexyl)ureido)phenyl) piperazin-1-carboxylate 7 (7.3 g, 11.5 mmol) was dissolved in DCM (20 mL), and TFA (10 mL) was added. The mixture was stirred and refluxed at 50°C overnight. The reaction mixture was then concentrated under reduced pressure and purified by column chromatography to obtain 4.3 g of a transparent oil (yield 70%). ¹H NMR (400 MHz, DMSO-*d*₆) δ 9.05 (s, 1H), 7.74 (dd, *J =* 8.0, 1.5 Hz, 1H), 7.63 (t, *J =* 8.5 Hz, 1H), 7.41 (d, *J =* 8.5 Hz, 1H), 7.31 - 7.21 (m, 3H), 7.21 - 7.13 (m, 4H), 7.00 (q, *J* = 9.1 Hz, 4H), 5.56 (t, *J =* 6.1 Hz, 1H), 4.28 (tt, *J* = 12.1, 3.7 Hz, 1H), 4.16 (d, J= 6.0 Hz, 2H), 3.65 - 3.53 (m, 1H), 3.11 (dd, *J* = 6.3, 3.7 Hz, 4H), 2.84 (t, *J* = 5.0 Hz, 4H), 1.96 (d, *J =* 10.9 Hz, 2H), 1.79 (d, *J =* 10.8 Hz, 2H), 1.41 (*q, J =* 13.1 Hz, 2H), 1.13 (q, *J* = 13.0 Hz, 2H).

### Step 5: Preparation of 3-benzyl-1-(4-(2-((2-(2,6-dioxopiperidin-3-yl)-1,3-dioxoisoindol-4-yl)oxy)acetyl)piperazin-1-yl)phenyl)-1-((1r,4r)-4-(quinazolin-2-ylamino)cyclohexyl) (Compound YJZ9069)

2-(2,6-dioxopiperidin-3-yl)-1,3-dioxoisoindolin-5-carboxylic acid **9** (39.9 mg, 0.12), HATU (45.1 mg, 0.12 mmol), DIPEA (21.3 mg, 0.16 mmol) and compound **8** (59 mg, 0.11mmol) were dissolved in DMF (6 mL). The mixture was stirred at room temperature for 15 minutes, and then the reaction was subjected to rotary evaporation under reduced pressure and purified by silica gel column chromatography to obtain 70 mg of the title compound as a white solid (yield 75%).

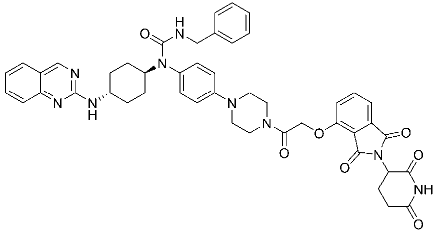

¹H NMR (400 MHz, DMSO-*d*₆) δ 11.12 (s, 1H), 9.05 (s, 1H), 7.79 (t, *J=* 7.9 Hz, 1H), 7.74 (d, *J =* 8.0 Hz, 1H), 7.63 (t, *J* = 7.8 Hz, 1H), 7.46 (d, *J* = 7.2 Hz, 1H), 7.39 (t, *J* = 8.1 Hz, 2H), 7.27 (q, *J =* 9.9, 8.7 Hz, 3H), 7.22 - 7.13 (m, 4H), 7.11 - 7.02 (m, 4H), 5.60 (t*, J* = 6.0 Hz, 1H), 5.26 (s, 2H), 5.12 (dd, *J* = 12.8, 5.4 Hz, 1H), 4.27 (d, *J* = 12.4 Hz, 1H), 4.17 (d, *J =* 6.0 Hz, 2H), 3.62 (s, 4H), 3.31 (s, 1H), 3.24 (s, 2H), 2.96 - 2.82 (m, 1H), 2.65 - 2.53 (m, 2H), 2.10 - 2.00 (m, 1H), 1.97 (d, *J* = 10.8 Hz, 2H), 1.80 (d, *J* = 11.8 Hz, 2H), 1.41 (q, *J =* 12.5 Hz, 2H), 1.13 (q, *J* = 12.3 Hz, 2H). ¹³C NMR (151 MHz, DMSO-*d*₆) δ 173.31, 170.43, 167.29, 165.76, 165.61, 162.50, 157.33, 156.06, 150.34, 141.73, 137.09, 134.48, 133.55, 132.13 (2C), 129.17, 128.52 (3C), 128.32, 127.17 (4C), 126.75, 122.21, 120.67, 119.95, 116.60, 116.40 (2C), 116.00, 66.57, 53.54, 49.23, 49.13, 48.29, 48.16, 44.33, 43.94, 41.69, 40.043, 31.75, 31.40, 30.87 (2C), 22.46. HRMS (ESI) for C₄₇H₄₇N₉O₇[M+H]⁺, calcd: 850.36712, found: 850.3646. HPLC analysis: MeOH-H₂O (80:20), 12.08 min, 97.4% purity.

### Example 24: 3-benzyl-1-(4-(2-((2-(2,6-dioxopiperidin-3-yl)-1,3-dioxoisoindol-5-yl)oxy) acetyl)piperazin-1-yl)phenyl)-1-((1r,4r)-4-(quinazolin-2-ylamino)cyclohexyl)urea (Compound YJZ1090)

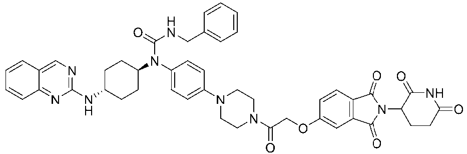

The synthesis method is similar to the method shown in Example 23.

¹H NMR (400 MHz, DMSO-*d*₆) δ 11.11 (s, 1H), 9.05 (s, 1H), 7.85 (d, *J =* 8.3 Hz, 1H), 7.74 (d, *J =* 8.3 Hz, 1H), 7.63 (t, *J =* 7.9 Hz, 1H), 7.48 (d, *J =* 2.3 Hz, 1H), 7.43 - 7.35 (m, 2H), 7.32 - 7.24 (m, 3H), 7.23 (d, *J =* 8.2 Hz, 1H), 7.21 - 7.13 (m, 4H), 7.11 - 7.00 (m, 4H), 5.60 (t, *J =* 5.8 Hz, 1H), 5.21 (s, 2H), 5.12 (dd, *J =* 12.9, 5.4 Hz, 1H), 4.27 (t, *J =* 12.3 Hz, 1H), 4.17 (d, *J =* 6.0 Hz, 2H), 3.70 - 3.52 (m, 5H), 3.31 (s, 2H), 3.24 (s, 2H), 2.95 - 2.83 (m, 1H), 2.65 - 2.54 (m, 2H), 2.10 - 2.01 (m, 1H), 1.97 (d, *J =* 11.4 Hz, 2H), 1.80 (d, *J =* 11.9 Hz, 2H), 1.41 (q, *J =* 12.4 Hz, 2H), 1.14 (q, *J =* 12.0 Hz, 2H). ¹³C NMR (151 MHz, DMSO-*d*₆) δ 173.30, 170.42, 167.42, 167.27, 165.69, 164.15, 162.50, 157.32, 150.32, 141.74, 134.47, 134.17, 132.13 (2C), 129.19, 128.52 (3C), 128.32, 127.17 (4C), 126.74, 125.65, 123.70, 122.21, 121.69, 119.95, 116.39 (2C), 109.58, 66.60, 53.54, 49.45, 49.13, 48.34, 48.16, 44.28, 43.94, 41.64, 40.44, 31.75, 31.41, 30.87 (2C), 22.52. HRMS (ESI) for C₄₇H₄₇N₉O₇[M+H]⁺, calcd: 850.36712, found: 850.3633. HPLC analysis: MeOH-H₂O (75:25), 6.96 min, 95.0% purity.

### Example 25: 3-benzyl-1-(4-(2-((2-(2,6-dioxopiperidin-3-yl)-3-oxoisoindol-5-yl)oxy)acetyl) piperazin-1-yl)phenyl)-1-((1r,4r)-4-(quinazolin-2-ylamino)cyclohexyl)urea (Compound YJZ1094)

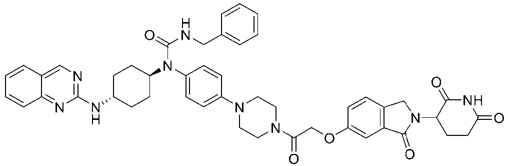

The synthesis method is similar to the method shown in Example 23.

¹H NMR (400 MHz, DMSO-*d*₆) δ 10.98 (s, 1H), 9.05 (s, 1H), 7.74 (d, *J =* 7.9 Hz, 1H), 7.63 (t, *J =* 7.7 Hz, 1H), 7.51 (d, *J =* 8.2 Hz, 1H), 7.40 (d, *J =* 8.5 Hz, 1H), 7.31 - 7.21 (m, 5H), 7.21 - 7.13 (m, 4H), 7.08 - 7.00 (m, 4H), 5.60 (t, *J=* 5.9 Hz, 1H), 5.11 (dd, *J =* 13.2, 5.1 Hz, 1H), 5.03 (s, 2H), 4.43 - 4.21 (m, 3H), 4.16 (d, *J =* 6.0 Hz, 2H), 3.71 - 3.53 (m, 5H), 3.30 (s, 2H), 3.23 (s, 2H), 2.96 - 2.84 (m, 1H), 2.68 - 2.53 (m, 2H), 2.04 - 1.90 (s, 3H), 1.80 (d, *J =* 12.1 Hz, 2H), 1.41 (q, *J =* 12.3 Hz, 2H), 1.20 - 1.06 (m, 2H). ¹³C NMR (151 MHz, DMSO-*d*₆) δ 173.41, 171.47, 168.57, 166.31, 162.50, 158.74, 157.33, 150.34, 141.75, 134.97, 134.48, 133.27, 132.11 (2C), 129.16, 128.52 (3C), 128.32, 127.16 (4C), 126.74, 124.92, 122.21, 120.65, 119.95, 116.38 (2C), 107.67, 66.52, 53.54, 52.21 (2C), 48.45, 48.18, 47.28, 44.44, 43.93, 41.63, 40.43, 31.74, 31.66, 30.86 (2C), 22.92. HRMS (ESI) for C₄₇H₄₉N₉O₆[M+H]⁺, calcd: 836.38786, found: 836.3843. HPLC analysis: MeOH-H₂O (75:25), 6.33 min, 95.6% purity.

### Example 26: 3-benzyl-1-(4-(4-((2-(2,6-dioxopiperidin-3-yl)-1,3-dioxoisoindol-5-yl)glycine) piperazin-1-yl)phenyl)-1-((1r,4r)-4-(quinazolin-2-ylamino)cyclohexyl)urea (Compound YJZ1091)

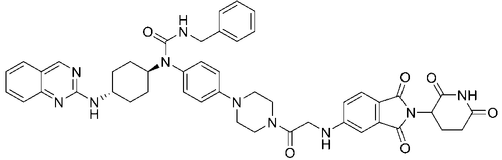

The synthesis method is similar to the method shown in Example 23.

¹H NMR (400 MHz, DMSO-*d*₆) δ 11.06 (s, 1H), 9.05 (s, 1H), 7.74 (d, *J =* 8.0 Hz, 1H), 7.63 (t, *J =* 7.5 Hz, 1H), 7.59 (d, *J =* 8.3 Hz, 1H), 7.40 (d, *J =* 8.5 Hz, 1H), 7.31 - 7.25 (dd, *J =* 8.5, 6.5 Hz, 2H), 7.23 (d, *J =* 8.2 Hz, 1H), 7.21 - 7.11 (m, 6H), 7.09 - 9.00 (m, 5H), 5.60 (t, *J =* 5.6 Hz, 1H), 5.05 (dd, *J =* 12.9, 5.5 Hz, 1H), 4.27 (t, *J =* 12.3 Hz, 1H), 4.22 (d, *J =* 5.1 Hz, 2H), 4.16 (d, *J =* 6.2 Hz, 2H), 3.68 (s, 4H), 3.58 (s, 1H), 3.30 (s, 2H), 3.24 (s, 2H), 2.93 - 2.81 (m, 1H), 2.62 - 2.53 (m, 2H), 2.45 - 1.89 (m, 3H), 1.80 (d, *J =* 12.1 Hz, 2H), 1.41 (q, *J =* 12.2 Hz, 2H), 1.14 (d, *J* = 13.3 Hz, 2H). ¹³C NMR (151 MHz, DMSO-*d*₆) δ 173.34, 170.66, 168.23, 167.70, 167.35, 162.50, 159.29, 157.33, 154.52, 152.04, 150.36, 141.74, 134.47, 132.12 (2C), 129.17, 128.52 (4C), 128.32, 127.17 (4C), 126.74, 125.28, 122.21, 119.95, 117.14, 116.39 (2C), 53.54, 49.11 (2C), 48.40, 48.18, 44.73, 44.30, 43.94, 41.83, 40.43, 31.75, 31.44, 30.87 (2C), 22.69. HRMS (ESI) for C₄₇H₄₈N₁₀O₆[M+H]⁺, calcd: 849.38311, found: 849.3803. HPLC analysis: MeOH-H₂O (75:25), 7.47 min, 96.8% purity.

### Example 27: 3-benzyl-1-(4-(4-((2-(2,6-dioxopiperidin-3-yl)-3-oxoisoindol-5-yl)glyceryl) piperazin-1-yl)phenyl)-1-((1r,4r)-4-(quinazolin-2-ylamino)cyclohexyl)urea (Compound YJZ1095)

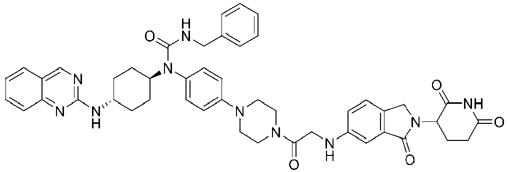

The synthesis method is similar to the method shown in Example 23.

¹H NMR (400 MHz, DMSO-*d*₆) δ 10.95 (s, 1H), 9.05 (s, 1H), 7.74 (d, *J =* 7.3 Hz, 1H), 7.63 (t, *J =* 7.8 Hz, 1H), 7.40 (d, *J =* 8.5 Hz, 1H), 7.28 (t, *J =* 7.6 Hz, 3H), 7.23 (d, *J =* 8.0 Hz, 1H), 7.18 (t, *J = 7.1* Hz, 4H), 7.10 - 6.99 (m, 5H), 6.94 (d, *J = 2.2* Hz, 1H), 5.97 (t, *J =* 5.1 Hz, 1H), 5.60 (t, *J =* 6.0 Hz, 1H), 5.08 (dd, *J =* 13.3, 5.1 Hz, 1H), 4.35 - 4.23 (d, *J =* 16.5 Hz, 2H), 4.19 - 4.12 (m, 3H), 4.07 (d, *J* = 5.1 Hz, 2H), 3.69 (d, *J =* 17.3 Hz, 4H), 3.59 (s, 1H), 3.31 (s, 2H), 3.23 (s, 2H), 2.96 - 2.84 (m, 1H), 2.69 - 2.55 (m, 2H), 2.43 - 2.30 (m, 1H), 1.97 (d, *J=* 11.7 Hz, 2H), 1.80 (d, *J =* 11.7 Hz, 2H), 1.41 (q, *J =* 12.6, 12.2 Hz, 2H), 1.14 (q, *J =* 13.5, 11.1 Hz, 2H). ¹³C NMR (151 MHz, DMSO-*d*₆) δ 173.41, 171.47, 168.57, 166.31, 162.50, 158.74, 157.33, 150.34, 141.75, 134.97, 134.48, 133.27, 132.11 (2C), 129.16, 128.52 (3C), 128.32, 127.16 (4C), 126.74, 124.92, 122.21, 120.65, 119.95, 116.38 (2C), 107.67, 66.52, 53.54, 52.21, 49.11, 48.45, 48.18, 47.28, 44.44, 43.93, 41.63, 40.43, 31.75, 31.66, 30.86 (2C), 22.92. HRMS (ESI) for C₄₇H₅₀N₁₀O₃[M+H]⁺, calcd: 835.40384, found: 835.4012. HPLC analysis: MeOH-H₂O (75:25), 6.70 min, 95.8% purity.

### Example 28: 3-benzyl-1-(4-(4-(1-(2-(2,6-dioxopiperidin-3-yl)-1,3-dioxoisoindole-5-yl) azetidin-3-carbonyl)piperazin-1-yl)phenyl)-1-((1r,4r)-4-(quinazolin-2-ylamino)cyclohexyl) urea (Compound YJZ1093)

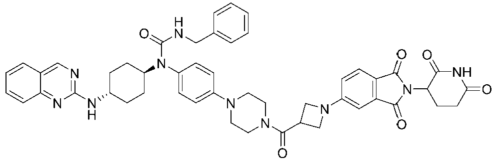

The synthesis method is similar to the method shown in Example 23.

¹H NMR (400 MHz, DMSO-*d*₆) δ 11.07 (s, 1H), 9.05 (s, 1H), 7.74 (d, *J =* 7.9 Hz, 1H), 7.66 (d, *J =* 8.3 Hz, 1H), 7.62 (*t, J =* 8.0 Hz, 1H), 7.40 (d, *J =* 8.5 Hz, 1H), 7.31 - 7.25 (m, 2H), 7.24 (d, *J =* 8.2 Hz, 1H), 7.21 - 7.13 (m, 4H), 7.10 - 7.00 (m, 4H), 6.85 (d, *J =* 2.1 Hz, 1H), 6.71 (dd, *J =* 8.4, 2.2 Hz, 1H), 5.58 (t, *J=* 6.2 Hz, 1H), 5.06 (dd, *J =* 12.9, 5.5 Hz, 1H), 4.34 - 4.22 (m, 3H), 4.22 - 4.08 (m, 4H), 4.05 - 3.94 (m, 1H), 3.66 (s, 2H), 3.58 (s, 1H), 3.51 (s, 2H), 3.29 - 3.16 (m, 4H), 2.94 - 2.82 (m, 1H), 2.70 - 2.52 (m, 2H), 2.06 - 1.90 (m, 3H), 1.80 (d, *J =* 11.9 Hz, 2H), 1.41 (q, *J =* 12.3 Hz, 2H), 1.19 - 1.06 (m, 2H). ¹³C NMR (151 MHz, DMSO-*d*₆) δ 173.34, 170.59, 169.88, 167.92, 167.65, 162.50, 157.33, 155.46, 150.39, 141.70, 134.49, 134.25, 132.11 (2C), 129.20, 128.53 (3C), 128.32, 127.17 (4C), 126.76, 125.33, 122.22, 119.95, 117.67, 116.46 (2C), 114.80, 105.02, 54.09 (2C), 53.53, 49.18 (2C), 48.53, 48.20, 44.89, 43.93, 41.79, 40.40, 31.74, 31.68, 31.42, 30.87 (2C), 22.65. HRMS (ESI) for C₄₉H₅₀N₁₀O₆[M+H]⁺, calcd: 875.39876, found: 875.3958. HPLC analysis: MeOH-H₂O (75:25), 8.69 min, 97.7% purity.

### Example 29: 3-benzyl-1-(4-(4-((E)-3-(2-(2,6-dioxopiperidin-3-yl)-1,3-dioxoisoindol-5-yl) acryloyl)piperazin-1-yl)phenyl)-1-((1r,4r)-4-(quinazolin-2-ylamino)cyclohexyl)urea (Compound YJZ1114)

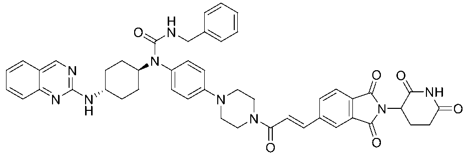

The synthesis method is similar to the method shown in Example 23.

¹H NMR (400 MHz, DMSO-*d*₆) δ 11.15 (s, 1H), 9.05 (s, 1H), 8.48 (s, 1H), 8.19 (d, *J =* 7.8 Hz, 1H), 7.96 (d, *J =* 7.7 Hz, 1H), 7.74 (d, *J* = 7.9 Hz, 1H), 7.71 (s, 2H), 7.63 (t, *J =* 7.8 Hz, 1H), 7.39 (d, *J =* 8.6 Hz, 1H), 7.31 - 7.21 (m, 3H), 7.21 - 7.13 (m, 4H), 7.07 (s, 4H), 5.59 (t, *J =* 6.4 Hz, 1H), 5.19 (dd, *J =* 13.0, 5.4 Hz, 1H), 4.27 (*t, J =* 11.9 Hz, 1H), 4.17 (d, *J =* 5.8 Hz, 2H), 3.95 (s, 2H), 3.76 (s, 2H), 3.58 (s, 1H), 2.97 - 2.83 (m, 1H), 2.69 - 2.56 (m, 2H), 2.13 - 2.04 (m, 2H), 1.96 (d, *J =* 11.9 Hz, 2H), 1.80 (d, *J =* 11.9 Hz, 2H), 1.41 (q, *J =* 12.1 Hz, 2H), 1.16 (q, *J =* 12.1 Hz, 2H). ¹³C NMR (151 MHz, DMSO-*d*₆) δ 173.26, 170.31, 167.43, 167.23, 164.45, 162.49, 157.29, 150.37, 142.28, 141.79, 140.14, 135.37, 134.45, 132.59, 132.13 (2C), 131.66, 129.21, 128.51(3C), 128.31, 127.18(4C), 126.72, 124.38, 122.87, 122.63, 122.18, 119.95, 116.38 (2C), 53.54, 49.57 (2C), 48.90, 48.27, 45.38, 43.95, 42.19, 40.50, 31.76, 31.41, 30.88 (2C), 22.45. HRMS (ESI) for C₄₈H₄₇N₉O₆[M+H]⁺, calcd: 846.37221, found: 846.3687. HPLC analysis: MeOH-H₂O (75:25), 14.00 min, 99.3% purity.

### Example 30: 3-benzyl-1-(4-(4-(3-(2-(2,6-dioxopiperidin-3-yl)-1,3-dioxoisoindole-5-yl) benzoyl)piperazin-1-yl)phenyl)-1-((1r,4r)-4-(quinazolin-2-ylamino)cyclohexyl)urea (Compound YJZ1130)

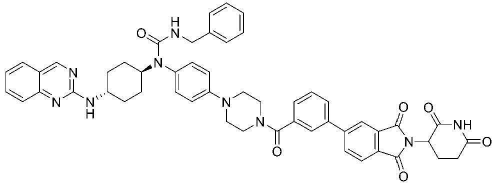

The synthesis method is similar to the method shown in Example 23.

¹H NMR (400 MHz, DMSO-*d*₆) δ 11.15 (s, 1H), 9.05 (s, 1H), 8.30 - 8.21 (m, 2H), 8.03 (d, *J =* 7.7 Hz, 1H), 7.97 (d, *J =* 7.9 Hz, 1H), 7.92 (s, 1H), 7.75 (d, *J =* 8.1 Hz, 1H), 7.68 - 7.60 (m, 2H), 7.55 (*d, J =* 7.6 Hz, 1H), 7.40 (d, *J =* 8.5 Hz, 1H), 7.31 - 7.23 (m, 3H), 7.21 - 7.13 (m, 4H), 7.11 - 6.98 (m, 4H), 5.57 (t, *J =* 6.8 Hz, lH), 5.19 (dd, *J =* 12.6, 5.3 Hz, 1H), 4.28 (t, *J =* 12.1 Hz, 1H), 4.16 (d, *J =* 5.7 Hz, 2H), 3.82 (s, 1H), 3.57 (s, 4H), 3.25 (s, 4H), 2.97 - 2.84 (m, 1H), 2.69 - 2.58 (m, 2H), 2.14 - 2.04 (m, 1H), 1.96 (d, *J =* 11.4 Hz, 2H), 1.80 (d, *J =* 12.0 Hz, 2H), 1.41 (q, *J* = 12.1 Hz, 2H), 1.14 *(q, J=* 12.1 Hz, 2H). ¹³C NMR (151 MHz, DMSO-*d*₆) δ 173.26, 170.33, 169.02, 167.42, 167.39, 162.48, 157.27, 150.37, 146.31, 141.76, 138.78, 137.40, 134.44, 133.72, 132.81, 132.13 (2C), 130.67, 129.99, 129.25, 128.96, 128.51 (3C), 128.31, 127.93, 127.17 (4C), 126.72, 126.26, 124.58, 122.21, 122.18, 119.96, 116.46 (2C), 53.53, 49.57 (2C), 49.12, 48.28, 47.52, 43.95, 40.51, 31.75, 31.42 (2C), 30.88 (2C), 22.47. HRMS (ESI) for C₅₂H₄₉N₉O₆[M+H]⁺, calcd: 896.38786, found: 896.3845. HPLC analysis: MeOH-H₂O (75:25), 19.82 min, 98.9% purity.

### Example 31: 3-benzyl-1-(4-(4-(1-(2-(2,6-dioxopiperidin-3-yl)-1,3-dioxoisoindole-5-yl)-1-pyrazol-3-carbonyl)piperazin-1-yl)phenyl)-1-((1r,4r)-4-(quinazolin-2-ylamino)cyclohexyl) urea (Compound YJZ1131)

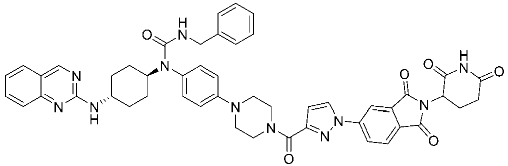

The synthesis method is similar to the method shown in Example 23.

¹H NMR (400 MHz, DMSO-*d*₆) δ 11.16 (s, 1H), 9.19 (s, 1H), 9.05 (s, 1H), 8.49 (s, 1H), 8.44 (d, *J =* 8.3 Hz, 1H), 8.16 (s, 1H), 8.09 (d, *J =* 8*.*2 Hz, 1H), 7.74 (d, *J=* 8.0 Hz, 1H), 7.63 *(t, J =* 7.8 Hz, 1H), 7.39 (d, *J =* 8.6 Hz, 1H), 7.31 - 7.21 (m, 3H), 7.21 - 7.13 (m, 4H), 7.11 - 6.97 (m, 4H), 5.60 (t, *J =* 6.2 Hz, 1H), 5.21 (dd, *J =* 13.0, 5.4 Hz, 1H), 4.29 (t, *J* = 12.2 Hz, 1H), 4.17 (d, *J =* 6.0 Hz, 2H), 3.83 (s, 4H), 3.58 (s, 1H), 3.31 (s, 4H), 2.98 - 2.85 (m, 1H), 2.69 - 2.57 (m, 2H), 2.15 - 2.04 (m, 1H), 1.97 (d, J= 12.6 Hz, 2H), 1.80 (d, *J =* 11.9 Hz, 2H), 1.41 (q, *J =* 12.9, 12.4 Hz, 2H), 1.14 (q, *J =* 12.9, 12.1 Hz, 2H). ¹³C NMR (151 MHz, DMSO-*d*₆) δ 173.24, 170.29, 166.93, 166.88, 162.48, 162.33, 157.29, 150.33, 144.34, 143.30, 141.78, 134.44, 133.77, 132.17 (2C), 130.30, 129.21, 129.05, 128.51(3C), 128.31, 127.18 (4C), 126.73, 125.67, 125.25, 124.64, 122.17, 119.98, 119.96, 116.32 (2C), 113.73, 53.55, 49.66 (2C), 49.13, 48.46, 43.96 (2C), 40.51, 31.76, 31.41(2C), 30.89 (2C), 22.45. HRMS (ESI) for C₄₉H₄₇N₁₁O₆[M+H]⁺, calcd: 886.37835, found: 886.3749. HPLC analysis: MeOH-H₂O (75:25), 14.54 min, 96.4% purity.

### Example 32: 3-benzyl-1-(4-(2-((2-(1-methyl-2,6-dioxopiperidin-3-yl)-1,3-dioxoisoindol-4-yl)oxy)acetyl)piperazin-1-yl)phenyl)-1-((1r,4r)-4-(quinazolin-2-ylamino)cyclohexyl)urea (Compound YJZ1078)

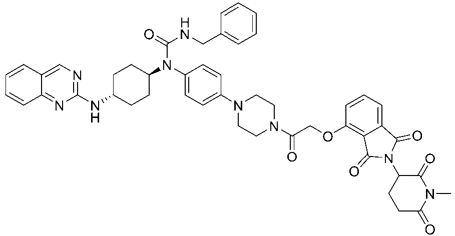

The synthesis method is similar to the method shown in Example 23.

¹H NMR (400 MHz, DMSO-*d*₆) δ 9.05 (s, 1H), 7.79 (t, *J =* 7.8 Hz, 1H), 7.74 (d, *J =* 8.0 Hz, 1H), 7.63 (t, *J =* 7.8 Hz, 1H), 7.47 (d, *J =* 7.3 Hz, 1H), 7.39 (d, *J =* 8.4 Hz, 2H), 7.31 - 7.21 (m, 3H), 7.21 - 7.13 (m, 4H), 7.10 - 6.99 (s, 4H), 5.59 (t, *J =* 6.0 Hz, 1H), 5.25 (s, 2H), 5.18 (dd, *J =* 12.7, 5.0 Hz, 1H), 4.28 (t, *J =* 10.4 Hz, 1H), 4.17 (d, *J =* 6.0 Hz, 2H), 3.67 - 3.56 (m, 5H), 3.32 (s, 2H), 3.24 (s, 2H), 3.02 (s, 3H), 3.00 - 2.88 (m, 1H), 2.81 - 2.70 (m, 1H), 2.60 - 2.54 (m, 1H), 2.06 (d, *J =* 9.7 Hz, 1H), 1.97 (d, *J =* 11.6 Hz, 2H), 1.80 (d, *J =* 11.9 Hz, 2H), 1.42 (q, *J =* 12.8, 12.4 Hz, 2H), 1.14 (q, *J =* 12.3, 11.6 Hz, 2H). ¹³C NMR (151 MHz, DMSO-*d*₆) δ 172.28, 170.18, 167.28, 165.76, 165.60, 162.50, 157.33, 156.07, 150.34, 141.72, 137.14, 134.48, 133.54, 132.12 (2C), 129.17, 128.52 (3C), 128.32, 127.17 (4C), 126.75, 122.21, 120.71, 119.95, 116.58, 116.39 (2C), 116.03, 66.61, 53.54, 49.81, 49.13, 48.31, 48.15, 44.34, 43.94, 41.70, 40.43, 31.75, 31.55, 30.87 (2C), 27.08, 21.67. HRMS (ESI) for C₄₈H₄₉N₉O₇[M+H]⁺, calcd: 864.38277, found: 864.3804. HPLC analysis: MeOH-H₂O (75:25), 9.27 min, 97.5% purity.

### Example 33: 3-benzyl-1-(4-(4-(2-((2-(2,6-dioxopiperidin-3-yl)-1,3-dioxoisoindol-4-yl)oxy) acetyl)piperazin-1-yl)phenyl)-1-((1r,4r)-4-((1-propyl-1H-pyrazolo[3,4-d]pyrimidin-6-yl) amino)cyclohexyl)urea (Compound YJZ9108)

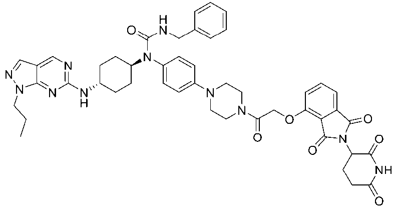

The synthesis method is similar to the method shown in Example 23.

¹H NMR (400 MHz, DMSO-*d*₆) δ 11.12 (s, 1H), 8.73 (s, 1H), 7.89 (s, 1H), 7.79 (dd, *J =* 8.5, 7.3 Hz, 1H), 7.46 (d, *J =* 7.2 Hz, 1H), 7.38 (d, *J =* 8.6 Hz, 1H), 7.28 (dd, *J =* 8.4, 6.4 Hz, 3H), 7.21 - 7.13 (m, 3H), 7.09 - 7.00 (m, 4H), 5.57 (s, 1H), 5.25 (s, 2H), 5.11 (dd, *J =* 12.9, 5.4 Hz, 1H), 4.26 (s, 1H), 4.16 (d, *J =* 6.0 Hz, 2H), 4.09 (t, *J =* 6.8 Hz, 2H), 3.62 (s, 4H), 3.51 (s, 1H), 3.30 (s, 2H), 3.24 (s, 2H), 2.96 - 2.82 (m, 1H), 2.64 - 2.53 (m, 2H), 2.09 - 1.92 (m, 3H), 1.84 - 1.70 (m, 4H), 1.40 (q, *J =* 12.7 Hz, 2H), 1.16 - 1.03 (m, 2H), 0.76(s, 3H).

### Example 34: N-(4-(4-(3-(3-benzyl-1-((1r,4r)-4-(quinazolin-2-ylamino)cyclohexyl)ureido) phenyl)piperazin-1-yl)butyl)-2-((2-(2,6-dioxopiperidin-3-yl)-1,3-dioxoisoindol-4-yl)oxy) acetamide (Compound YJZ9049)

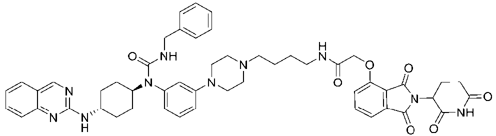

The synthesis method is similar to the method shown in Example 23.

¹H NMR (400 MHz, DMSO-*d*₆) δ 11.12 (s, 1H), 9.05 (s, 1H), 7.97 (t, *J =* 4.9 Hz 1H), 7.82 (t, *J =* 7.9 Hz, 1H), 7.74 (d, *J =* 8.1 Hz, 1H), 7.63 (t, *J =* 7.8 Hz, 1H), 7.50 (d, *J =* 7.3 Hz, 1H), 7.40 (d, *J =* 8.5 Hz, 2H), 7.33 - 7.21 m, 4H), 7.21 - 7.13 (m, 4H), 6.97 (d, *J =* 8.5 Hz, 1H), 6.69 (s, 1H), 6.61 (d, *J =* 7.6 Hz, 1H), 5.60 (t, *J =* 6.2 Hz, 1H), 5.12 (dd, *J =* 13.0, 5.4 Hz, 1H), 4.78 (s, 2H), 4.25 (t, *J =* 12.4 Hz, 1H), 4.18 (d, *J =* 5.9 Hz, 2H), 3.61 (d, *J =* 9.3 Hz, 1H), 3.25 - 3.08 (m, 6H), 2.96 - 2.83 (m, 1H), 2.63 - 2.53 (m, 2H), 2.48 - 2.44 (m, 2H), 2.32 (d, *J =* 6.6 Hz, 2H), 2.03 (d, *J =* 12.5 Hz, 1H), 1.97 (*d, J=* 12.0 Hz, 2H), 1.83 (d, *J =* 11.8 Hz, 2H), 1.48 (s, 4H), 1.40 (t, *J=* 12.4 Hz, 2H), 1.27 - 1.13 (m, 4H).

### Example 35: N-(5-(4-(3-(3-benzyl-1-((1r,4r)-4-(quinazolin-2-ylamino)cyclohexyl)ureido)phenyl)piperazin-1-yl)pentyl)-2-((2-(2,6-dioxopiperidin-3-yl)-1,3-dioxoisoindol-4-yl)oxy)acetamide (Compound YJZ9048)

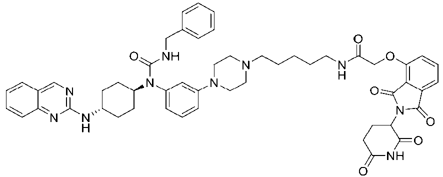

The synthesis method is similar to the method shown in Example 23.

¹H NMR (400 MHz, DMSO-*d*₆) δ 11.15 (s, 1H), 9.05 (s, 1H), 7.94 (t, *J=* 5.8 Hz, 1H), 7.81 (t, *J =* 8.0 Hz, 1H), 7.74 (d, *J =* 8.0 Hz, 1H), 7.63 (t, *J= 7.7* Hz, 1H), 7.50 (d, *J =* 7.2 Hz, 1H), 7.40 (d, *J =* 8.5 Hz, 2H), 7.33 - 7.24 (m, 3H), 7.22 (d, *J =* 8.0 Hz, 1H), 7.20 - 7.14 (m, 4H), 6.97 (d, *J =* 8.5 Hz, 1H), 6.69 (s, 1H), 6.61 (d, *J =* 7.7 Hz, 1H), 5.60 (t, *J =* 6.1 Hz, 1H), 5.12 (dd, *J =* 12.9, 5.3 Hz, 1H), 4.77 (s, 2H), 4.28 (t, *J =* 12.3 Hz, 1H), 4.17 (d, *J =* 5.9 Hz, 2H), 3.61 (s, 1H), 3.20 - 3.11 (m, 6H), 2.96 - 2.82 (m, 1H), 2.65 - 2.53 (m, 2H), 2.47 (s, 2H), 2.29 (t, *J =* 7.4 Hz, 2H), 2.03 (dd, *J =* 11.8, 6.3 Hz, 1H), 1.97 (d, *J =* 11.6 Hz, 2H), 1.83 (*d, J =* 11.9 Hz, 2H), 1.44 (dd, *J =* 22.9, 10.2 Hz, 6H), 1.30 (d, *J =* 7.3 Hz, 2H), 1.26 - 1.14 (m, 2H).

### Example 36: N-(6-(4-(3-(3-benzyl-1-((1r,4r)-4-(quinazolin-2-ylamino)cyclohexyl)ureido)phenyl)piperazin-1-yl)hexyl)-2-((2-(2,6-dioxopiperidin-3-yl)-1,3-dioxoisoindol-4-yl)oxy)acetamide (Compound YJZ9043)

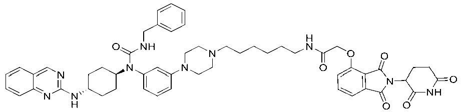

The synthesis method is similar to the method shown in Example 23.

¹H NMR (400 MHz, DMSO-*d*₆) δ 11.16 (s, 1H), 9.05 (s, 1H), 7.93 (t, *J =* 5.7 Hz, 1H), 7.81 (t, *J =* 7.9 Hz, 1H), 7.74 (d, *J =* 8.0 Hz, 1H), 7.63 (t, *J =* 7*.*7 Hz, 1H), 7.50 (d, *J =* 7.3 Hz, 1H), 7.40 (d, *J =* 8.5 Hz, 2H), 7.33 - 7.24 (m, 3H), 7.22 (d, *J =* 7.8 Hz, 1H), 7.20 - 7.14 (m, 4H), 6.97 (d, *J =* 8.6 Hz, 1H), 6.69 (s, 1H), 6.61 (d, *J =* 7.6 Hz, 1H), 5.59 (t, *J=* 6.0 Hz, 1H), 5.12 (dd, *J =* 12.9, 5.4 Hz, 1H), 4.77 (s, 2H), 4.27 (t, *J =* 11.8 Hz, 1H), 4.18 (d, *J =* 6.0 Hz, 2H), 3.62 (s, 1H), 3.16 (t, *J=* 6.2 Hz, 8H), 2.95 - 2.84 (m, 1H), 2.64 - 2.54 (m, 2H), 2.49 - 2.45 (m, 2H), 2.30 *(t, J =* 7.5 Hz, 2H), 2.08 - 2.00 (m, 1H), 1.97 (d, *J =* 12.1 Hz, 2H), 1.83 (d, *J =* 11.9 Hz, 2H), 1.50 - 1.35 (m, 6H), 1.33 - 1.26 (m, 4H), 1.23 - 1.14 (m, 2H).

### Example 37: N-(7-(4-(3-(3-benzyl-1-((1r,4r)-4-(quinazolin-2-ylamino)cyclohexyl)ureido)phenyl)piperazin-1-yl)heptyl)-2-(2-(2,6-dioxopiperidin-3-yl)-1,3-dioxoisoindol-4-yl)oxy)acetamide (Compound YJZ9047)

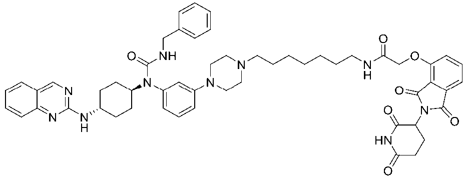

The synthesis method is similar to the method shown in Example 23.

¹H NMR (400 MHz, DMSO-*d*₆) δ 11.15 (s, 1H), 9.05 (s, 1H), 7.92 (t, *J =* 5.6 Hz, 1H), 7.81 (t, *J =* 7.9 Hz, 1H), 7.74 (d, *J =* 8.0 Hz, 1H), 7.63 (t, *J =* 7.7 Hz, 1H), 7.50 (d, *J =* 7.3 Hz, 1H), 7.43 - 7.35 (m, 2H), 7.28 (q, *J =* 8.2, 7.8 Hz, 3H), 7.22 (d, *J =* 7.8 Hz, 1H), 7.21 - 7.15 (m, 4H), 6.97 (d, *J =* 8.5 Hz, 1H), 6.69 (s, 1H), 6.61 (d, *J =* 7.6 Hz, 1H), 5.76 (s, 1H), 5.60 (t, *J =* 6.1 Hz, 1H), 5.12 (dd, *J =* 13.0, 5.4 Hz, 1H), 4.77 (s, 2H), 4.25 (t, *J =* 12.6 Hz, 1H), 4.18 (d, *J= 6.0* Hz, 2H), 3.62 (s, 1H), 3.16 (dd, *J =* 8.9, 5.0 Hz, 7H), 2.95 - 2.84 (m, 1H), 2.65 - 2.54 (m, 2H), 2.48 (s, 2H), 2.30 (t, *J=* 7.5 Hz, 2H), 2.08 - 2.01 (m, 1H), 1.97 (d, *J=* 12.3 Hz, 2H), 1.83 (d, *J =* 11.9 Hz, 2H), 1.51 - 1.34 (m, 6H), 1.33 - 1.13 (m, 10H).

### Example 38: 3-benzyl-1-(3-(4-(4-((2-(2,6-dioxopiperidin-3-yl)-1,3-dioxoisoindol-4-yl) amino)butanoyl)piperazin-1-yl)phenyl)-1-((1r,4r)-4-(quinazolin-2-ylamino)cyclohexyl)urea (Compound YJZ9058)

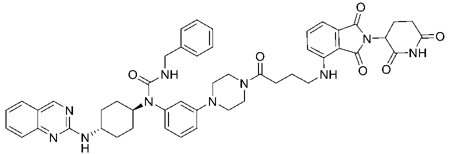

The synthesis method is similar to the method shown in Example 23.

¹H NMR (400 MHz, DMSO-*d*₆) δ 11.08 (s, 1H), 9.05 (s, 1H), 7.74 (d, *J =* 7.9 Hz, 1H), 7.61 (dt, *J =* 15.9, 7.8 Hz, 2H), 7.39 (d, *J =* 8.5 Hz, 1H), 7.35 - 7.24 (m, 3H), 7.22 (d, *J =* 7.9 Hz, 1H), 7.20 - 7.14 (m, 5H), 7.03 -6.97 (m, 2H), 6.72 (s, 1H), 6.70 - 6.61 (m, 2H), 5.61 (t, *J =* 6.1 Hz, 1H), 5.05 (dd, *J =* 12.9, 5.4 Hz, 1H), 4.26 (t, *J =* 12.1 Hz, 1H), 4.18 (d, *J =* 6.0 Hz, 2H), 3.68 - 3.54 (m, 5H), 3.23 - 3.09 (m, 4H), 2.93 - 2.82 (m, 1H), 2.62 - 2.54 (m, 2H), 2.46 (t, *J= 7.0* Hz, 2H), 2.06 - 1.93 (m, 4H), 1.89 - 1.78 (m, 4H), 1.42 *(q, J=* 12.4 Hz, 2H), 1.18 (q, *J =* 12.0 Hz, 2H).

### Example 39: 3-benzyl-1-(3-(4-(6-((2-(2,6-dioxopiperidin-3-yl)-1,3-dioxoisoindol-4-yl) amino)hexyl)piperazin-1-yl)phenyl)-1-((1r,4r)-4-(quinazolin-2-ylamino)cyclohexyl)urea (Compound YJZ9052)

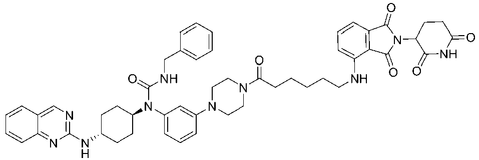

The synthesis method is similar to the method shown in Example 23.

¹H NMR (400 MHz, DMSO-*d*₆) δ 11.09 (s, 1H), 9.05 (s, 1H), 7.74 (d, *J =* 7.7 Hz, 1H), 7.63 (t, *J =* 7.7 Hz, 1H), 7.57 (dd, *J =* 8.6, 7.1 Hz, 1H), 7.39 (d, *J =* 8.5 Hz, 1H), 7.35 - 7.25 (m, 3H), 7.23 (d, *J =* 7.8 Hz, 1H), 7.21 - 7.13 (m, 4H), 7.09 (*d, J =* 8.6 Hz, 1H), 7.03 - 6.97 (m, 2H), 6.73 (s, 1H), 6.65 (d, *J* = 7.5 Hz, 1H), 6.54 (t, *J =* 5.9 Hz, 1H), 5.62 (t, *J =* 6.1 Hz, 1H), 5.04 (dd, *J =* 12.9, 5.3 Hz, 1H), 4.29 (t, *J =* 12.0 Hz, 1H), 4.18 (d, *J =* 6.0 Hz, 2H), 3.60 (s, 5H), 3.32 - 3.26 (m, 1H), 3.16 (d, *J =* 19.2 Hz, 4H), 2.93 - 2.81 (m, 1H), 2.62 - 2.53 (m, 2H), 2.37 (t, *J=* 7.3 Hz, 2H), 2.06 - 1.91 (m, 3H), 1.83 (d, *J =* 11.8 Hz, 2H), 1.59 (dp, *J =* 14.9, 7.2 Hz, 4H), 1.48 - 1.34 (m, 4H), 1.18 (q, *J =* 12.6 Hz, 2H).

### Example 40: 3-benzyl-1-(3-(4-(8-(2-(2,6-dioxopiperidin-3-yl)-1,3-dioxoisoindol-4-yl) amino)octyl)piperazin-1-yl)phenyl)-1-((1r,4r)-4-(quinazolin-2-ylamino)cyclohexyl)urea (Compound YJZ9053)

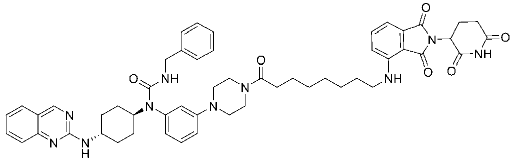

The synthesis method is similar to the method shown in Example 23.

¹H NMR (400 MHz, DMSO-*d*₆) δ 11.09 (s, 1H), 9.04 (s, 1H), 7.74 (d, *J =* 8.0 Hz, 1H), 7.62 (t, *J =* 7.7 Hz, 1H), 7.59 - 7.54 (m, 1H), 7.39 (d, *J =* 8.5 Hz, 1H), 7.35 - 7.25 (m, 3H), 7.22 (d, *J=* 8.0 Hz, 1H), 7.20 - 7.14 (m, 4H), 7.08 (d, *J =* 8.6 Hz, 1H), 7.04 - 6.98 (m, 2H), 6.73 (s, 1H), 6.65 (d, *J =* 7.6 Hz, 1H), 6.52 (t, *J =* 6.0 Hz, 1H), 5.62 (t, *J =* 6.0 Hz, 1H), 5.04 (dd, *J =* 12.9, 5.4 Hz, 1H), 4.26 (t, *J =* 12.1 Hz, 1H), 4.17 (d, *J =* 6.0 Hz, 2H), 3.59 (s, 5H), 3.31 - 3.24 (m, 1H), 3.16 *(d, J =* 20.5 Hz, 4H), 2.93 - 2.81 (m, 1H), 2.62 - 2.53 (m, 2H), 2.35 *(t, J =* 7.4 Hz, 2H), 2.06 - 1.91 (m, 3H), 1.83 (d, *J =* 12.0 Hz, 2H), 1.61 - 1.48 (m, 4H), 1.41 (q, *J =* 12.5 Hz, 2H), 1.36 - 1.27 (m, 6H), 1.18 (q, *J =* 12.5 Hz, 2H).

### Example 41: 3-benzyl-1-(3-(4-(10-(2-(2,6-dioxopiperidin-3-yl)-1,3-dioxoisoindol-4-yl) amino)decanoyl)piperazin-1-yl)phenyl)-1-((1r,4r)-4-(quinazolin-2-ylamino)cyclohexyl)urea (Compound YJZ9055)

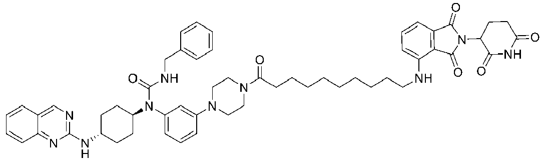

The synthesis method is similar to the method shown in Example 23.

¹H NMR (400 MHz, DMSO-*d*₆) δ 11.09 (s, 1H), 9.04 (s, 1H), 7.74 (d, *J =* 8.0 Hz, 1H), 7.62 (t, *J =* 7.7 Hz, 1H), 7.57 (dd, *J = 8.6,* 7.1 Hz, lH), 7.39 (d, *J =* 8.5 Hz, 1H), 7.35 - 7.24 (m, 3H), 7.23 (d, *J = 7.9* Hz, 1H), 7.21 - 7.13 (m, 4H), 7.07 *(d, J =* 8.6 Hz, 1H), 7.04 - 6.97 (m, 2H), 6.73 (s, 1H), 6.65 (*d, J =* 7.6 Hz, 1H), 6.51 (*t, J =* 5.9 Hz, 1H), 5.62 (t, *J =* 5.9 Hz, 1H), 5.05 (dd, *J =* 12.9, 5.4 Hz, 1H), 4.27 (t, *J =* 12.1 Hz, 1H), 4.17 (d, *J =* 6.0 Hz, 2H), 3.59 (s, 5H), 3.27 (p, *J =* 6.6 Hz, 2H) 3.16 (d, *J =* 20.9 Hz, 4H), 2.94 - 2.82 (m, 1H), 2.63 - 2.55 (m, 2H), 2.34 *(t, J =* 7.4 Hz, 2H), 2.06 - 1.90 (m, 3H), 1.83 (d, *J =* 11.9 Hz, 2H), 1.61 - 1.48 (m, 4H), 1.41 (q, *J =* 12.3 Hz, 2H), 1.36 - 1.12 (m, 12H).

### Example 42: N-(3-(3-benzyl-1-((1r,4r)-4-(quinazolin-2-ylamino)cyclohexyl)ureido) phenyl)-4-((2-(2,6-dioxopiperidin-3-yl)-1,3-dioxoisoindol-4-yl)amino)butanamide (Compound YJZ9062)

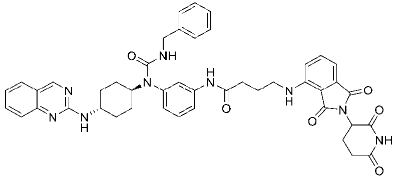

The synthesis method is similar to the method shown in Example 23.

¹H NMR (400 MHz, DMSO-*d*₆) δ 11.10 (s, 1H), 10.13 (s, 1H), 9.04 (s, 1H), 7.72 (dd, *J =* 11.5, 7.9 Hz, 2H), 7.66 - 7.55 (m, 2H), 7.48 (s, 1H), 7.43 - 7.35 (m, 2H), 7.30 - 7.21 (m, 3H), 7.21 - 7.12 (m, 5H), 7.03 (d, *J =* 7.0 Hz, 1H), 6.87 (*d, J =* 8.1 Hz, 1H), 6.69 (*t, J =* 6.2 Hz, 1H), 5.79 (*t, J =* 6.1 Hz, 1H), 5.05 (dd, *J* = 12.8, 5.4 Hz, 1H), 4.27 (*t, J =* 11.8 Hz, 1H), 4.16 (d, *J =* 6.0 Hz, 2H), 3.58 (s, 1H), 2.94 - 2.81 (m, 1H), 2.64 - 2.53 (m, 2H), 2.45 (t, *J = 7.2* Hz, 2H), 2.07 - 1.86 (m, 5H), 1.80 (d, *J =* 12.1 Hz, 2H), 1.42 (q, *J =* 12.2 Hz, 2H), 1.28 - 1.11 (m, 4H).

### Example 43: N-(3-(3-benzyl-1-((1r,4r)-4-(quinazolin-2-ylamino)cyclohexyl)ureido) phenyl)-8-((2-(2,6-dioxopiperidin-3-yl)-1,3-dioxoisoindole-4-yl)amino)octanamide (Compound YJZ9059)

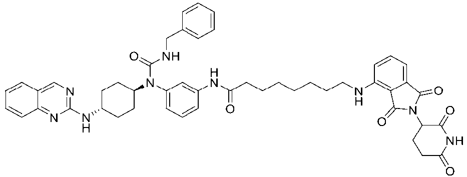

The synthesis method is similar to the method shown in Example 23.

¹H NMR (400 MHz, DMSO-*d*₆) δ 11.10 (s, 1H), 10.03 (s, 1H), 9.04 (s, 1H), 7.74 (d, *J =* 8.0 Hz, 1H), 7.69 (d, *J* = 8.4 Hz, 1H), 7.65 - 7.52 (m, 2H), 7.49 (s, 1H), 7.43 - 7.34 (m, 2H), 7.30 - 7.20 (m, 4H), 7.20 - 7.14 (m, 4H), 7.07 (d, *J =* 8.6 Hz, 1H), 7.01 (d, *J =* 7.1 Hz, 1H), 6.86 (d, *J =* 7.5 Hz, 1H), 6.52 (t, *J =* 5.9 Hz, 1H), 5.79 (t, *J =* 5.9 Hz, 1H), 5.04 (dd, *J =* 13.0, 5.4 Hz, 1H), 4.28 *(t, J =* 12.1 Hz, 1H), 4.16 (d, *J =* 5.9 Hz, 2H), 3.58 (s, 1H), 3.29 (p, *J =* 6.7 Hz, 2H)2.94 - 2.80 (m, 1H), 2.63 - 2.54 (m, 2H), 2.32 (t, *J =* 7.3 Hz, 2H), 2.06 - 1.91 (m, 3H), 1.79 (d, *J =* 12.0 Hz, 2H), 1.66 - 1.51 (s, 4H), 1.43 (q, *J=* 12.3 Hz, 2H), 1.37 - 1.31 (m, 4H), 1.28 - 1.22 (m, 2H), 1.21 - 1.10 (m, 2H).

### Example 44: 3-benzyl-1-(4-((1-(2-(2,6-dioxopiperidin-3-yl)-1,3-dioxoisoindol-5-yl) azetidin-3-ylmethyl)piperazin-1-yl)phenyl)-1-((1r,4r)-4-(quinazolin-2-ylamino)cyclohexyl) urea (Compound YJZ1102)

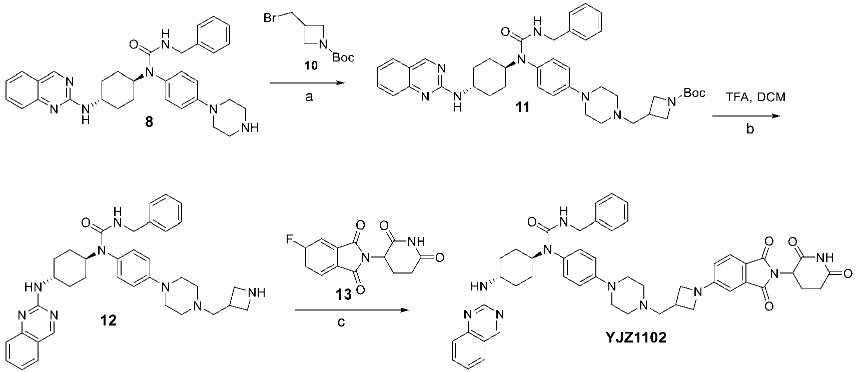

### Step 1: Preparation of tert-butyl 3-(4-(4-(3-benzyl-1-((1r,4r)-4-(quinazolin-2-ylamino)cyclohexyl)ureido)phenyl)piperazin-1-yl)methyl)azetidin-1-carboxylate (Compound 11)

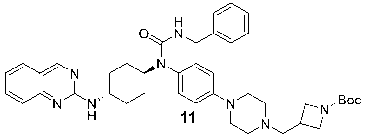

KHCO₃ (345 mg, 2.5 mmol) was added to DMF (40 mL) mixed with compound **8** (620 mg, 1.2 mmol) and tert-butyl 3-(bromomethyl)azetidin-1-carboxylate **10** (630 mg, 2.5 mmol). After stirring at 80°C for 5 h, the reaction was filtered. The filtrate was concentrated by rotary evaporation under reduced pressure and purified by column chromatography to obtain 560 mg of a white solid (yield 65%). ¹H NMR (400 MHz, DMSO-*d*₆) δ 9.05 (s, 1H), 7.74 (d, *J =* 8.0 Hz, 1H), 7.63 (t, *J=* 7.8 Hz, 1H), 7.40 (d, *J =* 8.7 Hz, lH), 7.27 (t, *J =* 7.5 Hz, 2H), 7.23 (d, *J =* 8.0 Hz, 1H), 7.21 - 7.12 (m, 4H), 7.01 *(q, J=* 9.2 Hz, 4H), 5.57 *(t, J=* 6.1 Hz, 1H), 4.26 (t, *J =* 12.1 Hz, 1H), 4.16 (d, *J* = 6.0 Hz, 2H), 3.92 (s, 2H), 3.67 - 3.44 (m, 4H), 3.31 (s, 2H), 3.17 (d, *J =* 6.2 Hz, 4H), 2.89 (s, 1H), 2.73 (s, 1H), 2.57 (d, *J =* 7.2 Hz, 2H), 1.96 (d, *J=* 11.3 Hz, 2H), 1.79 (d, *J =* 11.9 Hz, 2H), 1.47 - 1.31 (m, 11H), 1.13 *(q, J =* 12.6 Hz, 2H).

### Step 2: Preparation of 1-(4-(4-(azetidin-3-ylmethyl)piperazin-1-yl)phenyl)-3-benzyl-1-((1r,4r)-4-(quinazolin-2-ylamino)cyclohexyl)urea (Compound 12)

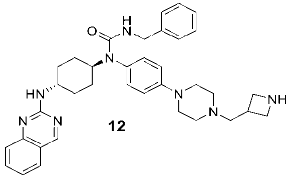

Tert-butyl 3-(4-(4-(3-benzyl-1-((1r,4r)-4-(quinazolin-2-ylamino)cyclohexyl)ureido)phenyl)piperazin-1-yl)methyl)azetidin-1-carboxylate 11 (100 mg, 0.14 mmol) was dissolved in DCM (4 mL), and TFA (2 mL) was added. The mixture was stirred and refluxed at 50°C overnight. The reaction mixture was then concentrated under reduced pressure and purified by column chromatography to obtain 60 mg of a white object (yield 70%).

### Step 3: 3-benzyl-1-(4-((1-(2-(2,6-dioxopiperidin-3-yl)-1,3-dioxoisoindol-5-yl)azetidin-3-ylmethyl)piperazin-1-yl)phenyl)-1-((1r,4r)-4-(quinazolin-2-ylamino)cyclohexyl)urea (Compound YJZ1102)

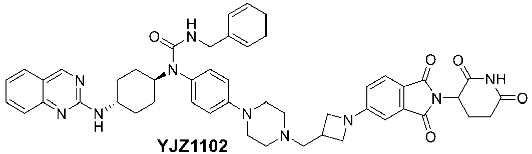

2-(2,6-dioxopiperidin-3-yl)-5-fluoroisoindolin-1,3-dione 13 (42.9 mg, 0.16 mmol) was dissolved in 6 mL DMSO, and DIPEA (25.1 mg, 0.19 mmol) and compound 12 (84.5 mg, 0.13 mmol) were added at room temperature. The resulting mixture was stirred at 120°C for 8 h. After the reaction, the solvent was removed in vacuum, and the residue was purified by column chromatography to obtain 71 mg of the compound as a yellow solid (yield 63%). ¹H NMR (400 MHz, DMSO-*d*₆) δ 11.07 (s, 1H), 9.05 (s, 1H), 7.74 (d, *J =* 7.9 Hz, 1H), 7.69 - 7.59 (m, 2H), 7.40 (d, *J =* 8.5 Hz, 1H), 7.32 - 7.25 (m, 2H), 7.23 (d, *J =* 8.0 Hz, 1H), 7.18 (t, *J =* 7.5 Hz, 4H), 7.09 - 6.95 (m, 4H), 6.79 (d, *J =* 2.1 Hz, 1H), 6.66 (dd, *J =* 8.4, 2.1 Hz, lH), 5.58 *(t, J=* 6.1 Hz, 1H), 5.06 (dd, *J =* 12.9, 5.4 Hz, 1H), 4.30 *(t, J=* 12.4 Hz, 2H), 4.22 - 4.09 (m, 4H), 3.72 (dd, *J =* 8.4, 5.4 Hz, 2H), 3.59 (s, 1H), 3.21 (t, *J =* 4.8 Hz, 4H), 3.06 (p, *J =* 6.9 Hz, 1H), 2.95 - 2.81 (m, 1H), 2.67 (d, *J =* 7.2 Hz, 2H), 2.63 - 2.53 (m, 6H), 2.08 - 1.89 (m, 3H), 1.79 (d, *J =* 11.7 Hz, 2H), 1.41 (q, *J =* 12.3, 11.6 Hz, 2H), 1.13 (q, *J =* 12.4 Hz, 2H). ¹³C NMR (151 MHz, DMSO-*d*₆) δ 173.33, 170.61, 167.99, 167.68, 162.49, 157.35, 155.66, 150.60, 141.76, 134.46, 134.28, 132.02 (2C), 128.68, 128.52 (3C), 128.31, 127.16 (4C), 126.74, 125.30, 122.20, 119.95, 117.11, 115.90 (2C), 114.55, 104.80, 62.22, 56.21 (2C), 53.52, 53.30 (2C), 49.17 (2C), 48.06 (2C), 43.93, 40.43, 31.75, 31.44, 30.87 (2C), 27.47, 22.68. HRMS (ESI) for C₄₉H₅₂N₁₀O₅[M+H]⁺, calcd: 861.41949, found: 861.4163. HPLC analysis: MeOH-H₂O (80:20), 11.34 min, 98.3% purity.

### Example 45: Preparation of 3-benzyl-1-(4-(4-(1-(2-(2,6-dioxopiperidin-3-yl)-1,3-dioxoisoindol-5-yl)piperidin-4-yl)-piperazin-1-yl)phenyl)-1-((1r,4r)-4-(quinazolin-2-ylamino)cyclohexyl)urea (Compound YJZ1105)

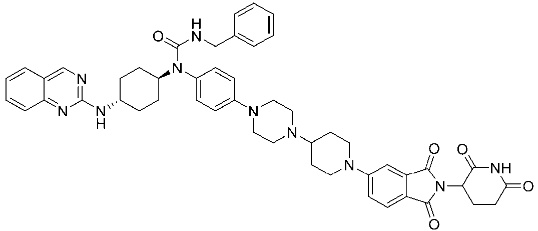

The synthesis method is similar to the method shown in Example 44.

¹H NMR (400 MHz, DMSO-*d*₆) δ 11.08 (s, 1H), 9.05 (s, 1H), 7.74 (d, *J =* 8.0 Hz, 1H), 7.67 (d, *J =* 8.5 Hz, lH), 7.63 (t, *J =* 7.5 Hz, 2H), 7.40 (d, *J =* 8.5 Hz, 1H), 7.34 (d, *J =* 2.2 Hz, 1H), 7.30 - 7.24 (m, 3H), 7.23 (d, *J =* 8.1 Hz, 1H), 7.20 - 7.12 (m, 4H), 7.01 (q, *J =* 8.8 Hz, 4H), 5.57 (t, *J =* 6.0 Hz, 1H), 5.07 (dd, *J =* 12.9, 5.4 Hz, 1H), 4.26 (t, *J* = 12.1 Hz, 1H), 4.16 (d, *J =* 6.0 Hz, 2H), 4.09 (d, *J =* 10.1 Hz, 2H), 3.65 - 3.53 (m, 1H), 3.26 - 3.12 (m, 4H), 3.00 (t, *J= 12.3* Hz, 2H), 2.94 - 2.82 (m, 1H), 2.65 (t, *J =* 4.9 Hz, 4H), 2.62 - 2.59 (m, 1H), 2.59 - 2.53 (m, 2H), 2.07 - 1.86 (m, 5H), 1.78 (d, *J =* 11.6 Hz, 2H), 1.50 (q, *J =* 11.1 Hz, 2H), 1.41 (q, *J =* 12.5 Hz, 4H), 1.13 (q, *J =* 12.5, 11.9 Hz, 2H). ¹³C NMR (151 MHz, DMSO-*d*₆) δ 173.37, 170.61, 168.11, 167.48, 162.50, 157.39, 155.22, 150.62, 141.70, 134.48, 131.99 (2C), 128.56, 128.52 (3C), 128.32, 127.14 (4C), 126.75, 125.53, 125.24, 122.23, 119.94, 118.17, 118.01, 115.79 (2C), 108.24, 60.99, 53.51, 49.20 (3C), 48.38 (2C), 47.06 (2C), 43.91, 40.73, 40.36, 31.75, 31.42, 30.85 (2C), 27.64 (2C), 22.65. HRMS (ESI) for C₅₀H₃₄N₁₀O₅[M+H]⁺, calcd: 875.43514, found: 875.4322. HPLC analysis: MeOH-H₂O (80:20), 11.36 min, 99.2% purity.

### Example 46: 3-benzyl-1-(4-(4-(2-(2,6-dioxopiperidin-3-yl)-1,3-dioxoisoindol-5-yl) azetidin-3-yl(piperazin-1-yl)phenyl)-1-((1r,4r)-4-(quinazolin-2-ylamino)cyclohexyl)urea (Compound YJZ1103)

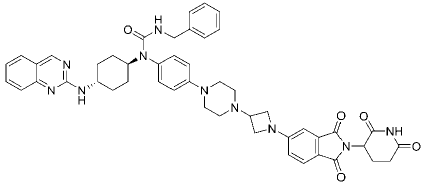

The synthesis method is similar to the method shown in Example 44.

¹H NMR (400 MHz, DMSO-*d*₆) δ 11.08 (s, 1H), 9.05 (s, 1H), 7.74 (d, *J =* 8.5 Hz, 1H), 7.67 (d, *J =* 8.3 Hz, 1H), 7.63 (t, *J=* 7.7 Hz, 1H), 7.40 (d, *J =* 8.5 Hz, 1H), 7.31 - 7.25 (m, 2H), 7.23 (d, *J =* 7.9 Hz, 1H), 7.21 - 7.14 (m, 4H), 7.08 - 6.98 (m, 4H), 6.82 (d, *J =* 2.1 Hz, 1H), 6.68 (dd, *J =* 8.4, 2.1 Hz, 1H), 5.58 (t, *J =* 6.3 Hz, 1H), 5.06 (dd, *J =* 12.9, 5.4 Hz, 1H), 4.34 - 4.23 (m, 1H), 4.15 (t, *J =* 7.6 Hz, 4H), 3.94 (dd, *J =* 8.8, 4.9 Hz, 2H), 3.66 - 3.51 (m, 1H), 3.45 - 3.37 (m, 1H), 3.26 (t, *J =* 5.7 Hz, 4H), 2.94 - 2.80 (m, 1H), 2.64 - 2.53 (m, 6H), 2.06 - 1.99 (m, 1H), 1.96 (d, *J* = 14.5 Hz, 2H), 1.79 (d, *J =* 11.7 Hz, 2H), 1.41 (q, *J =* 12.4 Hz, 2H), 1.13 (q, *J =* 12.8 Hz, 2H). ¹³C NMR (151 MHz, DMSO-*d*₆) δ 173.33, 170.61, 167.97, 167.67, 162.49, 157.35, 155.42, 150.54, 141.76, 134.47, 134.29, 132.04 (2C), 128.76, 128.52 (3C), 128.31, 127.16 (4C), 126.73, 125.35, 122.20, 119.95, 117.33, 115.94 (2C), 114.72, 104.97, 55.50 (2C), 54.62, 53.51, 49.59 (2C), 49.18 (2C), 47.83 (2C), 43.92, 40.43, 31.74, 31.43, 30.86 (2C), 22.67. HRMS (ESI) for C₄₈H₅₀N₁₀O₅[M+H]⁺, calcd: 847.40384, found: 847.4000. HPLC analysis: MeOH-H₂O (75:25), 13.02 min, 97.9% purity.

### Example 47: 3-benzyl-1-(4-(4-(1-(2-(2,6-dioxopiperidin-3-yl)-1,3-dioxoisoindol-5-yl) pyrrolidin-3-yl(piperazin-1-yl)phenyl)-1-((1r,4r)-4-(quinazolin-2-ylamino)cyclohexyl)urea (Compound YJZ1096)

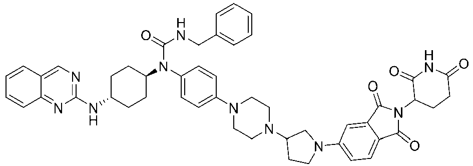

The synthesis method is similar to the method shown in Example 44.

¹H NMR (400 MHz, DMSO-*d*₆) δ 11.07 (s, 1H), 9.05 (s, 1H), 7.74 (d, *J=* 8.0 Hz, 1H), 7.68 - 7.59 (m, 2H), 7.40 (d, *J= 8.4* Hz, 1H), 7.28 (t, *J=* 7.5 Hz, 2H), 7.23 (d, *J=* 8.1 Hz, 1H), 7.18 (t, *J =* 7.3 Hz, 4H), 7.04 (s, 4H), 6.99 (s, 1H), 6.87 (d, *J =* 9.0 Hz, 1H), 5.58 (d, *J =* 7.0 Hz, 1H), 5.06 (dd, *J =* 12.7, 5.4 Hz, 1H), 4.30 *(*t*, J =* 12.4 Hz, 1H), 4.16 (d, *J* = 5.9 Hz, 2H), 3.75 (t, *J =* 8.4 Hz, 1H), 3.66 - 3.53 (m, 2H), 3.42 (q, *J =* 8.8 Hz, 2H), 3.31 - 3.27 (m, 1H), 3.24 (t, *J =* 6.2 Hz, 4H), 3.02 (p, *J =* 7.6 Hz, 1H), 2.95 - 2.82 (m, 1H), 2.67 (s, 4H), 2.63 - 2.53 (m, 2H), 2.34 - 2.26 (m, 1H), 2.05 - 1.88 (m, 4H), 1.79 (d, *J =* 11.7 Hz, 2H), 1.41 (q, *J =* 12.4 Hz, 2H), 1.13 (q, *J =* 12.8 Hz, 2H). ¹³C NMR (151 MHz, DMSO-*d*₆) δ 173.35, 170.65, 168.20, 167.74, 162.49, 157.36, 152.27, 150.57, 141.76, 134.46, 132.03 (2C), 128.74, 128.52 (4C), 128.31, 127.15 (4C), 126.74, 125.41, 122.21, 119.95, 116.22, 115.92 (2C), 115.71, 106.05, 63.97, 53.52, 52.42, 51.76 (2C), 49.15 (2C), 48.05 (2C), 47.49, 43.93, 40.43, 31.76, 31.45, 30.87 (2C), 29.28, 22.71. HRMS (ESI) for C₄₉H₅₂N₁₀O₅[M+H]⁺, calcd: 861.41949, found: 861.4168. HPLC analysis: MeOH-H₂O (75:25), 15.48 min, 98.2% purity.

### Example 48: 3-benzyl-1-(4-(4-((1-(2-(2,6-dioxopiperidin-3-yl)-1,3-dioxoisoindole-5-yl) piperidin-4-yl)methyl)piperazin-1-yl)phenyl)-1-((1r,4r)-4-(quinazolin-2-ylamino) cyclohexyl)urea (YJZ1097)

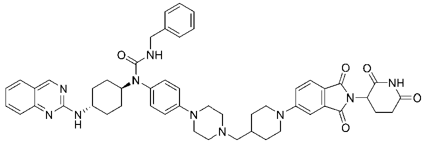

The synthesis method is similar to the method shown in Example 44.

¹H NMR (600 MHz, DMSO-*d*₆) δ 11.09 (s, 1H), 9.05 (s, 1H), 7.75 (d, *J =* 7.9 Hz, 1H), 7.66 (d, *J =* 8.5 Hz, 1H), 7.63 (t, *J =* 7.6 Hz, 1H), 7.41 (d, *J =* 8.6 Hz, 1H), 7.32 (s, 1H), 7.30 - 7.26 (m, 2H), 7.24 (d, *J =* 8.8 Hz, 2H), 7.21 - 7.13 (m, 4H), 7.02 (q, *J =* 8.7 Hz, 4H), 5.58 (t, *J =* 6.1 Hz, 1H), 5.07 (dd, *J =* 12.8, 5.5 Hz, 1H), 4.29 *(t, J=* 11.7 Hz, 1H), 4.16 (d, *J =* 6.0 Hz, 2H), 4.06 *(d, J =* 12.8 Hz, 2H), 3.65 - 3.54 (m, 1H), 3.26 - 3.15 (s, 4H), 2.99 *(t, J=* 12.3 Hz, 2H), 2.93 - 2.83 (m, 1H), 2.64 - 2.54 (m, 2H), 2.52 (m, 4H), 2.21 (d, *J =* 5.2 Hz, 2H), 2.05 - 1.99 (m, 1H), 1.99 - 1.93 (m, 2H), 1.92 - 1.85 (m, 1H), 1.85 - 1.75 (m, 4H), 1.41 (q, *J =* 12.4 Hz, 2H), 1.20 - 1.09 (m, 4H). ¹³C NMR (151 MHz, DMSO-*d*₆) δ 173.30, 170.60, 168.12, 167.45, 162.48, 157.32, 155.48, 150.62, 141.81, 134.52, 134.42, 132.02 (2C), 128.67, 128.51 (3C), 128.31, 127.16 (4C), 126.71, 125.49, 125.28, 122.17, 119.96, 118.06, 117.80, 115.83 (2C), 108.21, 64.18, 53.68 (2C), 53.52, 49.20 (2C), 48.15, 47.74 (2C), 43.94, 40.51, 32.94, 31.76, 31.45, 30.88 (2C), 30.11 (2C), 29.45, 22.67. HRMS (ESI) for C₅₁H₅₆N₁₀O₅[M+H]⁺, calcd: 889.45079, found: 889.4473. HPLC analysis: MeOH-H₂O (80:20), 15.05 min, 96.9% purity.

### Example 49: 3-benzyl-1-(4-(4-(1-(2-(2-(2,6-dioxopiperidin-3-yl)-6-fluoro-1,3-dioxoisoindole-5-yl)piperidin-4-yl)-piperazin-1-yl)phenyl)-1-((1r,4r)-4-(quinazolin-2-ylamino)cyclohexyl)urea (YJZ1201)

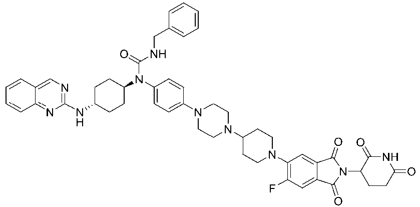

The synthesis method is similar to the method shown in Example 44.

¹H NMR (400 MHz, DMSO-*d*₆) δ 11.11 (s, 1H), 9.05 (s, 1H), 7.77 - 7.68 (m, 2H), 7.63 (t, *J =* 7.9 Hz, 1H), 7.47 (d, *J =* 7.3 Hz, 1H), 7.40 (d, *J* = 8.5 Hz, 1H), 7.31 - 7.24 (m, 2H), 7.23 (d, *J =* 7.0 Hz, 1H), 7.21 - 7.13 (m, 4H), 7.07 - 6.95 (m, 4H), 5.57 (t, *J =* 6.8 Hz, 1H), 5.11 (dd, *J* = 13.1, 5.3 Hz, 1H), 4.28 (t*, J =* 11.4 Hz, 1H), 4.16 (d, *J* = 5.8 Hz, 2H), 3.68 (d, *J =* 11.7 Hz, 2H), 3.58 (s, 1H), 3.21 (s, 4H), 2.99 - 2.82 (m, 3H), 2.68 (s, 4H), 2.64 - 2.54 (m, 3H), 2.08 - 1.89 (m, 5H), 1.78 (d, *J* = 9.6 Hz, 2H), 1.61 (q, *J =* 12.2 Hz, 3H), 1.41 (q, *J* = 14.5, 13.7 Hz, 2H), 1.13 (q, *J* = 13.1 Hz, 2H). ¹³C NMR (151 MHz, DMSO-*d*₆) δ 173.25, 170.40, 167.17, 166.69, 162.47, 158.57 (d, *J* = 253.3 Hz, 1C), 157.32, 150.63, 146.01(d, *J* = 8.8 Hz, 1C), 141.82, 134.43, 132.02 (2C), 129.26 (d, *J =* 2.2 Hz, 1C), 128.65, 128.50 (3C), 128.30, 127.16 (4C), 126.71, 123.51(d, *J* = 9.6 Hz, 1C), 122.17, 119.96, 115.79 (2C), 114.23 (d, *J* = 4.8 Hz, 1C), 112.49 (d, *J* = 25.3 Hz, 1C), 60.86, 53.52, 52.46, 49.84, 49.81,49.51,49.25 (2C), 48.49 (2C), 43.94, 40.51, 31.78, 31.42, 30.87 (2C), 28.35 (2C), 22.55. HRMS (ESI) for C₅₀H₅₃N₁₀O₅F[M+H]⁺, calcd: 893.42572, found: 893.4229. HPLC analysis: MeOH-H₂O (80:20), 11.79 min, 95.8% purity.

### Example 50: 1-(4-(4-(1-(1-(2-(2,6-dioxopiperidin-3-yl)-1,3-dioxoisoindole-5-yl)piperidin-4-yl)-piperazin-1-yl)phenyl)-3-(2-fluorobenzyl)-1-((1r,4r)-4-(quinazolin-2-ylamino) cyclohexyl)urea (YJZ1203)

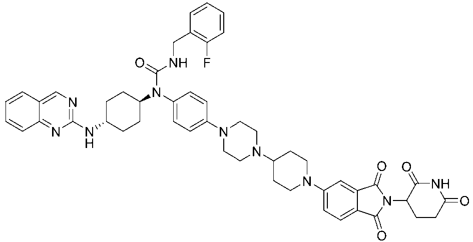

The synthesis method is similar to the method shown in Example 44.

¹H NMR (400 MHz, DMSO-*d*₆) δ 11.08 (s, 1H), 9.05 (s, 1H), 7.74 (d, *J* = 8.0 Hz, 1H), 7.67 (d, *J* = 8.6 Hz, 1H), 7.63 (t, *J =* 7.7 Hz, 1H), 7.40 (d, *J* = 8.6 Hz, 1H), 7.34 (s, 1H), 7.29 - 7.14 (m, 6H), 7.14 - 7.08 (m, 1H), 7.03 (q, *J* = 8.6 Hz, 4H), 5.59 (s, 1H), 5.07 (dd, *J* = 13.4, 5.5 Hz, 1H), 4.31 - 4.17 (m, 3H), 4.10 (d, *J* = 12.7 Hz, 2H), 3.58 (s, 1H), 3.20 (s, 4H), 3.00 (t, *J* = 12.3 Hz, 2H), 2.95 - 2.82 (m, 1H), 2.66 (s, 4H), 2.63 - 2.53 (m, 3H), 2.07 - 1.86 (m, 5H), 1.79 (d, *J =* 11.9 Hz, 2H), 1.50 (q, *J* = 11.5 Hz, 2H), 1.44 - 1.33 (m, 2H), 1.20 - 1.09 (m, 2H). ¹³C NMR (151 MHz, DMSO-*d*₆) δ 173.30, 170.59, 168.09, 167.44, 162.47, 161.01 (d, *J* = 243.4 Hz, 1C), 157.23, 155.23, 150.66, 134.51, 134.43, 131.98 (2C), 129.03 (d, *J* = 4.7 Hz, 2C), 128.58, 128.55, 128.53, 128.44 (d, *J* = 14.2 Hz, 1C), 128.30, 125.49, 125.29, 124.61(d, *J* = 3.3 Hz, 1C), 122.17, 119.96, 118.18, 118.10, 115.81 (2C), 115.12 (d, *J* = 21.1 Hz, 1C), 108.29, 61.02, 53.57, 49.25 (2C), 49.21, 49.10, 48.46 (2C), 47.09 (2C), 40.51, 37.73, 31.76, 31.45, 30.84 (2C), 27.67 (2C), 22.66. HRMS (ESI) for C₅₀H₅₃N₁₀O₅F[M+H]⁺, calcd: 893.42572, found: 893.4224. HPLC analysis: MeOH-H₂O (80:20), 9.19 min, 96.2% purity.

### Example 51: 3-benzyl-1-(6-(4-(1-(1-(2-(2,6-dioxopiperidin-3-yl)-1,3-dioxoisoindole-5-yl) piperidin-4-yl)pyrazin-1-yl)pyridin-3-yl]-1-((1r,4r)-4-(quinazolin-2-ylamino)cyclohexyl) urea (YJZ1205)

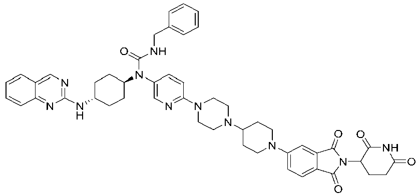

The synthesis method is similar to the method shown in Example 44.

¹H NMR (400 MHz, DMSO-*d*₆) δ 11.08 (s, 1H), 9.05 (s, 1H), 7.91 (s, 1H), 7.74 (d, *J* = 8.0 Hz, 1H), 7.70 - 7.58 (m, 2H), 7.40 (d, *J =* 8.4 Hz, 1H), 7.34 (s, 2H), 7.31 - 7.21 (m, 4H), 7.21 - 7.13 (m, 4H), 6.88 (d, *J =* 9.0 Hz, 1H), 6.05 (t, *J* = 6.6 Hz, 1H), 5.07 (dd, *J* = 12.8, 5.3 Hz, 1H), 4.26 (t, *J=* 12.8 Hz, 1H), 4.12 (dd, *J* = 18.7, 9.2 Hz, 4H), 3.60 (s, 1H), 3.52 (s, 4H), 3.00 (t, *J =* 12.4 Hz, 2H), 2.94 - 2.81 (m, 1H), 2.70 - 2.53 (m, 7H), 2.07 - 1.93 (m, 3H), 1.90 (d, *J =* 12.2 Hz, 2H), 1.78 (d, *J =* 11.7 Hz, 2H), 1.57 - 1.33 (m, 4H), 1.09 (q, *J =* 12.9 Hz, 2H). ¹³C NMR (151 MHz, DMSO-*d*₆) δ 173.30, 170.59, 168.09, 167.44, 162.47, 158.32, 157.45, 155.22, 149.85, 141.82, 140.60, 134.51, 134.43, 128.48 (3C), 128.31, 127.18 (4C), 126.68, 125.49, 124.43, 122.17, 119.97, 118.17, 118.09, 108.28, 107.40, 61.12, 55.39, 53.49, 49.21(2C), 49.10 (2C), 47.10 (2C), 45.29, 43.96, 31.70, 31.45, 30.85 (2C), 27.66 (2C), 22.66. HRMS (ESI) for C₄₉H₅₃N₁₁O₅[M+H]⁺, calcd: 876.43039, found: 876.4276. HPLC analysis: MeOH-H₂O (80:20), 6.94 min, 97.8% purity.

### Example 52: 3-benzyl-1-(6-(4-(1-(1-(2-(2-(2,6-dioxopiperidin-3-yl)-6-fluoro-1,3-dioxoisoindole-5-yl)piperidin-4-yl)pyrrolizin-1-yl)pyridin-3-yl]-1-((1r,4r)-4-(quinazolin-2-ylamino)cyclohexyl)urea (YJZ1206)

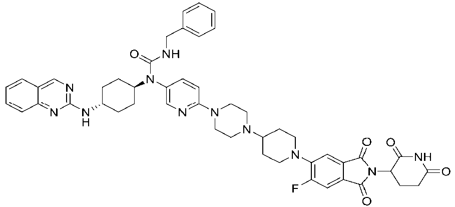

The synthesis method is similar to the method shown in Example 44.

¹H NMR (400 MHz, DMSO-*d*₆) δ 11.11 (s, 1H), 9.05 (s, 1H), 7.92 (s, 1H), 7.73 (t, *J* = 9.7 Hz, 2H), 7.63 (t, *J* = 8.0 Hz, 1H), 7.46 (d, *J =* 7.4 Hz, 1H), 7.41 (d, *J* = 8.5 Hz, 1H), 7.35 (d, *J* = 9.1 Hz, 1H), 7.31 - 7.25 (m, 2H), 7.21 (d, *J =* 7.5 Hz, 1H), 7.21 - 7.13 (m, 3H), 6.90 (d, *J* = 9.0 Hz, 1H), 6.05 (t, *J* = 5.4 Hz, 1H), 5.11 (dd, *J* = 13.0, 5.3 Hz, 1H), 4.28 (t, *J* = 12.3 Hz, 1H), 4.15 (d, *J* = 6.0 Hz, 2H), 3.68 (d, *J* = 11.9 Hz, 2H), 3.63 - 3.48 (m, 5H), 2.99 - 2.82 (m, 3H), 2.64 (s, 4H), 2.59 - 2.52 (m, 3H), 2.08 - 1.87 (m, 5H), 1.79 (d, *J =* 11.7 Hz, 2H), 1.61 (d, *J =* 12.0 Hz, 2H), 1.41 (q*, J =* 13.6, 13.0 Hz, 2H), 1.10 (d, *J* = 12.8 Hz, 3H). ¹³C NMR (151 MHz, DMSO-*d*₆) δ 173.25, 170.40, 167.17, 166.69, 162.47, 158.33, 157.45, 156.89 (d, *J* = 253.4 Hz, 1C), 149.86, 146.00 (d, *J* = 8.49 Hz, 1C), 141.83, 140.60, 134.42, 129.26 (d, *J* = 2.2 Hz, 1C), 128.48 (3C), 128.30, 127.19 (4C), 126.69, 125.28, 124.42, 123.52 (d, *J* = 9.9 Hz, 1C), 122.17, 119.97, 114.24 (d, *J* = 4.5 Hz, 1C), 112.48 (d*, J =* 25.5 Hz, 1C), 107.40, 60.96, 55.39, 53.50, 49.84 (2C), 49.52 (2C), 49.11 (2C), 45.30, 43.97, 40.51, 31.72, 31.42, 30.85 (2C), 28.32 (2C), 22.55. HRMS (ESI) for C₄₉H₅₂N₁₁O₃F[M+H]⁺, calcd: 894.42097, found: 894.4176. HPLC analysis: MeOH-H₂O (80:20), 8.79 min, 98.9% purity.

### Example 53: 3-benzyl-1-(4-(4-(2-(2,6-dioxopiperidin-3-yl)-1,3-dioxoisoindole-5-yl) piperazin-1-yl)piperidin-1-ylphenyl)-1-((1r,4r)-4-(quinazolin-2-ylamino)cyclohexyl)urea (YJZ1202)

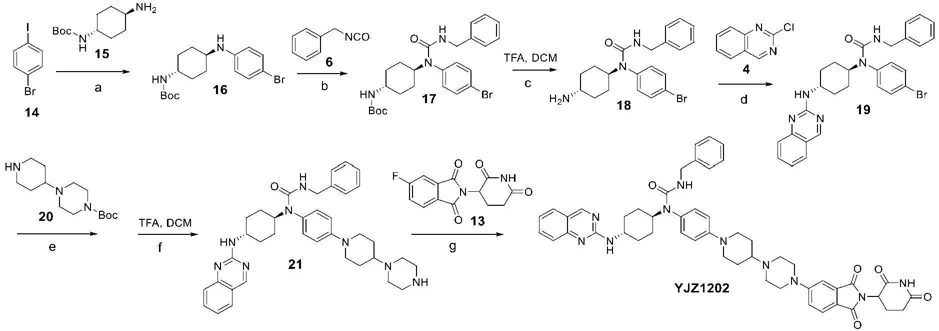

### Step 1: Preparation of tert-butyl ((1r,4r)-4-((4-bromophenyl)amino)cyclohexyl) carbamate (Compound 16)

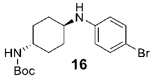

Compound **14** (5.0 g, 17.7 mmol), compound **15** (3.2 g, 14.7 mmol), Pd₂(dba)₃ (1.37 mg, 1.5 mmol), Xantphos (1.7 g, 2.94 mmol), and *tert*-ButONa (2.8 g, 29.4 mmol) were mixed and dissolved in 150 mL of toluene. After three times of argon replacement, the reaction was heated to 100°C and reacted overnight. After the reaction was completed, the reaction was filtered through celite and concentrated by rotary evaporation under reduced pressure. The residue was purified by column chromatography to obtain 3.9 g of a yellow solid (yield 72%). ¹H NMR (400 MHz, DMSO-*d*₆) δ 7.15 (d, *J =* 6.8 Hz, 2H), 6.76 (d, *J =* 7.9 Hz, 1H), 6.50 (d, *J =* 8.9 Hz, 2H), 5.60 (d, *J =* 8.0 Hz, 1H), 3.27 - 3.13 (m, 1H), 3.12 - 2.99 (m, 1H), 1.94 (d, *J =* 12.7 Hz, 2H), 1.79 (d, *J =* 12.4 Hz, 2H), 1.38 (s, 9H), 1.26 (q, *J =* 12.2, 11.3 Hz, 2H), 1.14 (q, *J* = 12.2 Hz, 2H).

### Step 2: Preparation of tert-butyl ((1r,4r)-4-(3-benzyl-1-(4-bromophenyl)ureido) cyclohexyl)carbamate (Compound 17)

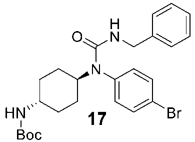

The compound **16** (3.9 g, 10.6 mmol) were dissolved in 4 mL DMF, and benzyl isocyanate **6** (4.2 g, 31.7 mmol) and DIPEA (1.59 g, 12.3 mmol) were added. The mixture was reacted at 95°C for 5 h. The solvent was removed by rotary evaporation, and the residue was purified by column chromatography to obtain 3.7 g of a yellow solid (69% yield). ¹H NMR (400 MHz, DMSO-*d*₆) δ 7.63 (d, *J* = 8.4 Hz, 2H), 7.30 - 7.22 (m, 2H), 7.20 - 7.14 (m, 3H), 7.11 (d, *J =* 7.5 Hz, 2H), 6.66 (d, *J* = 8.0 Hz, 1H), 5.98 (t*, J =* 6.2 Hz, 1H), 4.22 - 4.07 (m, 3H), 3.02 - 2.87 (m, 1H), 1.73 (d, *J =* 10.8 Hz, 4H), 1.35 (s, 9H), 1.24 (q, *J =* 12.5 Hz, 2H), 1.01 (q, *J* = 12.5, 12.0 Hz, 2H).

### Step 3: Preparation of 1-((1r,4r)-4-aminocyclohexyl)-3-benzyl-1-(4-bromophenyl)urea (Compound 18)

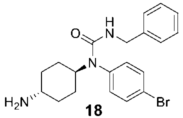

Compound **17** (3.7 g, 7.3 mmol) was dissolved in 5 mL DCM and 2.5 mL of trifluoroacetic acid (TFA) was added. After the mixture was heated to 55°C and refluxed for 6 h, the solvent was removed by rotary evaporation under reduced pressure and the residue was purified by column chromatography to obtain 2.5 g of the target compound as a yellow solid (yield 70%). ¹H NMR (400 MHz, DMSO-*d*₆) δ 7.62 (d, *J =* 8.0 Hz, 2H), 7.30 - 7.22 (m, 2H), 7.21 - 7.14 (m, 3H), 7.11 (d, *J=* 8.0 Hz, 2H), 5.95 (t, *J* = 6.1 Hz, 1H), 4.20 (t, *J=* 11.9 Hz, 1H), 4.14 (d, *J=* 6.1 Hz, 2H), 2.26 (t, *J* = 7.8 Hz, 1H), 1.80 - 1.60 (m, 4H), 1.10 (q, *J=* 11.9, 11.2 Hz, 2H), 0.98 (q, *J=* 12.5, 12.1 Hz, 2H).

### Step 4: Preparation of 3-benzyl-1-(4-bromophenyl)-1-((1r,4r)-4-(quinazolin-2-ylamino) cyclohexyl)urea (Compound 19)

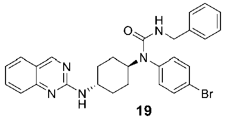

Compound **18** (2.5 g, 5.1 mmol) was dissolved in 15 mL DMF. 2-chloroquinazoline **4** (1.0 g, 6.1 mol) and Cs₂CO₃ (2.0 g, 6.1 mol) were added, and the mixture was stirred for 15 min at room temperature and then heated to 60°C for 40 min. After the reaction was completed, the solvent was removed by rotary evaporation under reduced pressure and the residue was purified by column chromatography to obtain 1.9 g (yield 70%). ¹H NMR (400 MHz, DMSO-*d*₆) δ 9.05 (s, 1H), 7.74 (d, *J* = 8.0 Hz, 1H), 7.69 - 7.59 (m, 3H), 7.40 (d, *J* = 8.5 Hz, 1H), 7.31 - 7.25 (m, 2H), 7.23 (d, *J* = 7.8 Hz, 1H), 7.21 - 7.12 (m, 6H), 6.02 (t, *J* = 6.2 Hz, 1H), 4.29 (t, *J=* 12.5 Hz, 1H), 4.16 (d, *J* = 5.9 Hz, 2H), 3.68 - 3.53 m, 1H), 1.98 (d, *J =* 13.6 Hz, 2H), 1.81 (d, *J* = 12.1 Hz, 2H), 1.42 (q, *J =* 12.4 Hz, 2H), 1.13 (q, *J =* 12.5 Hz, 2H).

### Step 5: Preparation of tert-butyl 4-(1-(4-(3-benzyl-1-((1r,4r)-4-(quinazolin-2-ylamino) cyclohexyl)ureido)phenyl)piperidin-4-yl)piperazin-1-carboxylate (Compound 21)

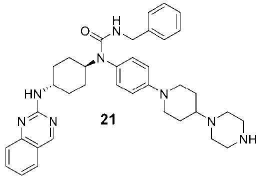

Compound **19** (1.9 g, 3.6 mmol), compound **20** (1.25 g, 4.6 mmol), Pd₂(dba)₃ (327 mg, 0.36 mmol), Xantphos (414 mg, 0.72 mmol), and *tert*-ButONa (688 mg, 7.2 mmol) were mixed and dissolved in 80 mL of toluene. After three times of argon replacement, the reaction was heated to 100°C overnight. After the reaction was completed, the reaction was filtered through celite and concentrated by rotary evaporation under reduced pressure. The residue was purified by column chromatography to obtain 1785 mg of a light yellow solid (yield 69%). ¹H NMR (400 MHz, DMSO-*d*₆) δ 9.05 (s, 1H), 7.74 (d, *J =* 8.2 Hz, 1H), 7.66 - 7.60 (m, 1H), 7.40 (d, *J* = 9.1 Hz, 1H), 7.30 - 7.24 (m, 2H), 7.24 - 7.13 (m, 5H), 7.04 - 6.95 (m, 4H), 5.53 (t, *J =* 7.4 Hz, 1H), 4.29 (t, *J* = 12.6 Hz, 1H), 4.16 (d, *J =* 6.0 Hz, 2H), 3.79 (d, *J =* 12.1 Hz, 2H), 3.65 - 3.51 (m, 1H), 3.30 (s, 4H), 2.71 (t, *J* = 12.1 Hz, 2H), 2.46 (s, 4H), 2.35 (t, *J =* 11.5 Hz, 1H), 2.01 - 1.91 (m, 2H), (d, *J =* 15.7 Hz, 2H), 1.87 (d, *J =* 11.8 Hz, 2H), 1.78 (d, *J =* 12.0 Hz, 2H), 1.48 (q, *J =* 12.0 Hz, 2H), 1.44 - 1.32 (m, 11H), 1.13 (q, *J* = 12.5 Hz, 2H). The above reaction product was dissolved in 5 mL of dichloromethane, and 2.5 mL of trifluoroacetic acid was added. The mixture was refluxed at 55°C for 4 h. After removing the Boc protecting group, 1.1 g was obtained and used in the next step.

### Step 6: Preparation of 3-benzyl-1-(4-(4-(2-(2,6-dioxopiperidin-3-yl)-1,3-dioxoisoindole-5-yl)piperazin-1-yl)piperidin-1-ylphenyl)-1-((1,4r)-4-(quinazolin-2-ylamino)cyclohexyl)urea (YJZ1202)

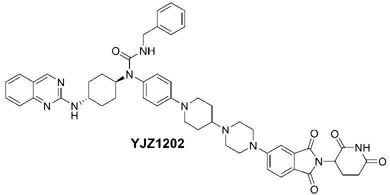

YJZ1102 was obtained by a similar method to that of Step 3 of example 44 using compound 21. ¹H NMR (400 MHz, DMSO-*d*₆) δ 11.08 (s, 1H), 9.05 (s, 1H), 7.74 (d, *J =* 8.0 Hz, 1H), 7.68 (d, *J* = 8.4 Hz, 1H), 7.63 (t, *J* = 7.9 Hz, 1H), 7.40 (d, *J* = 8.5 Hz, 1H), 7.34 (s, 1H), 7.31 - 7.21 (m, 4H), 7.21 - 7.13 (m, 4H), 7.01 (s, 4H), 5.55 (t, *J* = 6.5 Hz, 1H), 5.08 (dd, *J* = 13.0, 5.4 Hz, 1H), 4.28 (t, *J* = 12.3 Hz, 1H), 4.16 (d, *J* = 6.0 Hz, 2H), 3.81 (d, *J* = 11.7 Hz, 2H), 3.59 (s, 1H), 3.44 (s, 4H), 2.95 - 2.83 (m, 1H), 2.74 (t, *J =* 11.8 Hz, 2H), 2.66 (s, 4H), 2.63 - 2.53 (m, 2H), 2.40 (t, *J* = 11.0 Hz, 1H), 2.06 - 1.87 (m, 5H), 1.79 (d, *J =* 11.9 Hz, 2H), 1.53 (q, *J =* 11.9 Hz, 2H), 1.41 (q, *J* = 12.5 Hz, 2H), 1.13 (q, *J* = 12.6 Hz, 2H). ¹³C NMR (151 MHz, DMSO-*d*₆) δ 173.29, 170.56, 168.04, 167.47, 162.47, 157.33, 155.73, 150.64, 141.80, 134.42, 134.32, 131.99 (2C), 128.51 (3C), 128.38, 128.30, 127.16 (4C), 126.71, 125.36, 122.17, 119.96, 118.76, 118.21, 116.24 (2C), 108.28, 61.03, 53.53, 49.24, 49.13, 48.91(2C), 47.94 (2C), 47.71 (2C), 43.94, 40.51, 31.77, 31.45, 30.88 (2C), 28.38 (2C), 22.65. HRMS (ESI) for C₅₀H₅₄N₁₀O₅[M+H]⁺, calcd: 875.43514, found: 875.4315. HPLC analysis: MeOH-H₂O (80:20), 9.43 min, 95.2% purity.

### Example 54: 3-benzyl-1-(4-(4-(1-(2-(2,6-dioxopiperidin-3-yl)-1,3-dioxoisoindole-5-yl) piperidin-4-yl)-piperazin-1-yl)-3-fluorophenyl)-1-((1r,4r)-4-(quinazolin-2-ylamino) cyclohexyl)urea (YJZ1204)

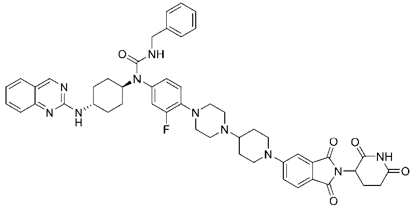

The synthesis method is similar to the method shown in Example 53.

¹H NMR (400 MHz, DMSO-*d*₆) δ 11.08 (s, 1H), 9.05 (s, 1H), 7.74 (d, *J=* 8.0 Hz, 1H), 7.69 - 7.59 (m, 2H), 7.40 (d, *J* = 8.5 Hz, 1H), 7.34 (s, 1H), 7.31 - 7.21 (m, 4H), 7.21 - 7.14 (m, 4H), 7.06 (t, *J* = 9.2 Hz, 1H), 7.02 - 6.91 (m, 2H), 5.92 (t, *J* = 6.4 Hz, 1H), 5.07 (dd, *J* = 13.2, 5.4 Hz, 1H), 4.24 (t, *J* = 13.3 Hz, 1H), 4.10 (d, *J* = 12.7 Hz, 2H), 3.67 - 3.54 (m, 1H), 3.08 (s, 4H), 3.00 (t, *J* = 12.3 Hz, 2H), 2.94 - 2.82 (m, 1H), 2.67 (s, 4H), 2.63 - 2.53 (m, 3H), 2.07 - 1.93 (m, 3H), 1.90 (d, *J =* 12.4 Hz, 2H), 1.81 (d, *J* = 11.8 Hz, 2H), 1.50 (q, *J* = 11.8 Hz, 2H), 1.40 (q, *J* = 12.2 Hz, 2H), 1.14 (q, *J* = 11.9 Hz, 2H). ¹³C NMR (151 MHz, DMSO-*d*₆) δ 173.30, 170.59, 168.09, 167.44, 162.48, 157.02, 155.23, 153.97 (d, *J =* 246.4 Hz, 1C), 141.77, 139.82 (d, *J =* 8.1 Hz, 1C), 134.51, 134.44, 132.01 (d, *J* = 9.2 Hz, 1C), 128.49 (3C), 128.32, 128.04, 127.17 (4C), 126.70, 125.49, 122.18, 119.97, 119.43, 119.30 (d, *J =* 14.5 Hz, 1C), 118.09 (d, *J* = 14.1 Hz, 1C), 108.29, 61.06, 53.80, 50.74, 49.27 (2C), 49.21 (2C), 49.06, 47.10 (2C), 43.94, 40.51, 31.76 , 31.45, 30.77 (2C), 27.64 (2C), 22.66. HRMS (ESI) for C₅₀H₅₃N₁₀O₅F[M+H]⁺, calcd: 893.42572, found: 893.4225. HPLC analysis: MeOH-H₂O (80:20), 10.01 min, 95.8% purity.

### Example 55: Tests for Degradation Activity of Representative Compounds on CDK12/13 in MDA-MB-231 Cells

Experimental methods: The cell line MDA-MB-231 was purchased from the American Type Culture Collection (ATCC). Conventional Western Blot was used for detection, as follows: MDA-MB-231 cells were seeded at a density of 2 x 10⁵/mL in a 12-well plate, with 1 mL per well, and cultured overnight in an incubator in an adherent manner. Then, a certain concentration of compounds was added to act for 15 h. The cells were lysed with a lysis buffer, and protein samples were collected. An appropriate amount of the sample was taken for SDS-PAGE electrophoresis. After electrophoresis, the protein was transferred to a polyvinylidene fluoride (PVDF) membrane using a wet electro-transfer system. The PVDF membrane after electro-transfer was cut into several required strips, which were then placed in a blocking solution (5% skim milk powder diluted in TBS containing 0.1% Tween 20) and blocked at room temperature for 1.5 h. A primary antibody solution was prepared by diluting a primary antibody against the corresponding target protein in TBS containing 0.1% Tween 20 according to the dilution requirements of different antibodies, such as 1:1000 (CST), 1:3000 (Proteintech, Bethyl), and 1:200 (Santacruz). The blocked membrane was placed in the corresponding primary antibody solution and incubated at 4°C for 12-14 h. Then, the cells were washed three times with TBS containing 0.1% Tween 20, each time for 5 min. After washing, the membrane was placed in a secondary antibody solution (horseradish peroxidase-labeled goat anti-rabbit IgG, diluted at 1:2000 in TBS containing 0.1% Tween 20) and reacted at room temperature for 2 h. After washing the membrane three times as above, the membrane was developed with StarSignal plus reagent (GenStar^{®}) and Omni-ECL reagent (Yazyme^{®}), and photographed with Amersham Imager 800 system.

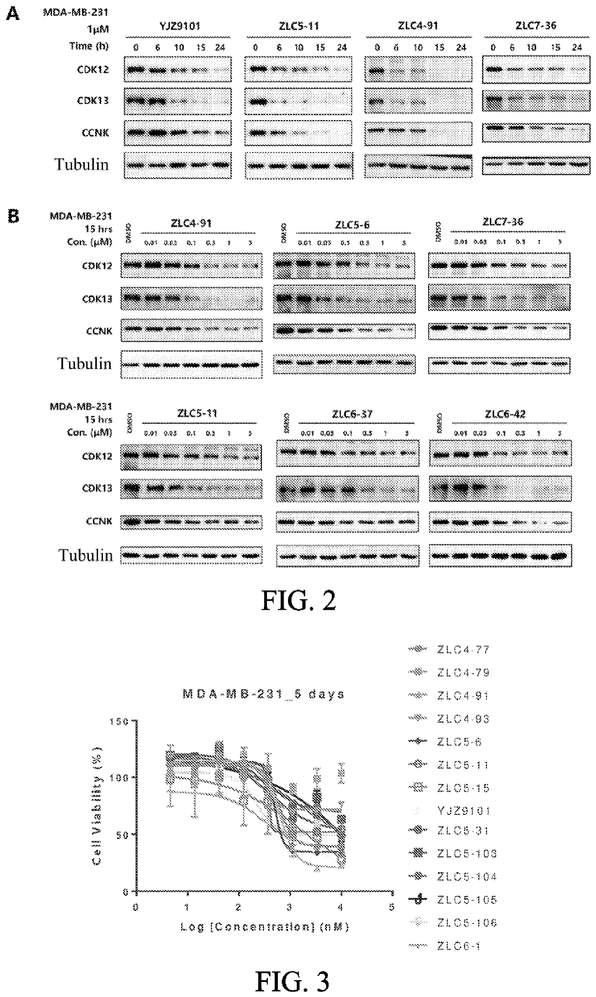

The results in FIG. 1 shows that some of the compounds can effectively degrade CDK12 and CDK13 protein levels in cells, such as compounds **ZLC4-77, ZLC4-91, ZLC5-6, ZLC5-11, ZLC7-36, ZLC5-106, ZLC6-1, ZLC6-37, ZLC6-42 and ZLC6-103,** etc. The results in FIG. 2 shows that the compounds **ZLC4-91** and **ZLC5-11** can degrade CDK12 and CDK13 proteins in MDA-MB-231 cells in a time-dependent manner, and the proteins have been almost completely degraded after 15 hours of treatment at a concentration of 1 µM. In addition, the compounds have a time-dependent degradation effect on the partner protein Cyclin K of CDK12 and CDK13. After 15 hours, the Cyclin K protein in the cells has been almost completely degraded.

We further determined the degradation of CDK12 and CDK13 proteins in MDA-MB-231 cells by representative compounds at different concentrations. The experimental results show that these representative compounds can degrade CDK12 and CDK13 proteins in MDA-MB-231 cells in a dose-dependent manner, and the target proteins can be effectively degraded at a concentration of 1 µM (FIG. 2).

FIG. 1 is (A) WB results of degradation of CDK12 and CDK13 proteins after treatment of MDA-MB-231 cells by some compounds at 0.1µM for 15 h; (B) WB results of degradation of CDK12 and CDK13 proteins after treatment of MDA-MB-231 cells by some compounds at 0.3µM for 15 hours; (C) the CDK12/13 levels obtained by quantifying the gray value of the bands in the WB result image (FIG. A), with the control group DMSO as the reference standard; and (D) the CDK12/13 levels obtained by quantifying the gray value of the bands in the WB result image (FIG. B), with the control group DMSO as the reference standard.

FIG. 2 is (A) WB measurement results of time-dependent degradation of CDK12 and CDK13 proteins in MDA-MB-231 cells by representative compounds; and (B) WB measurement results of the intracellular CDK12/13 protein levels after treatment of MDA-MB-231 cells by representative compounds at different concentrations for 15 h.

### Example 56: Study on the Proliferation-Inhibiting Activity of Compounds on Triple Negative Breast Cancer Cells MDA-MB-231

The cell proliferation-inhibiting activity of the compounds was determined using Cell Counting Kit-8 Cell Viability Assay (Selleck.cn). The cells were seeded into a 384-well plate (CORNING^{®} PS 3701) containing the corresponding culture medium and incubated in an incubator at 37°C with 5% CO₂. After overnight incubation, a high-concentration stock solution of each compound to be tested was prepared; the compound was added to the 384-well plate according to a concentration gradient using an Echo^{®} 650 Liquid Handler (LABCYTE^{®}). After administration, the cell plate was placed in an incubator at 37°C with 5% CO₂ and incubated for 5 days. Then, 5 µL of cck-8 reagent was added to each well of the 384-well plate using MULTIDROP^{®} (Thermo Scientific^{™}), and the plate was returned to the incubator for incubation for 1.5-2 h. After incubation, the plate was removed and centrifuged to remove bubbles. The absorbance of each well at 450 nm and 650 nm was obtained using EnVision^{®} (PerkinElmer^{®}). GraphPad Prism software (GraphPad Software Inc.) was used to process and analyze the data.

The results in FIG. 3 show that the compounds that can effectively degrade CDK12 and CDK13 protein levels, such as **ZLC4-77, ZLC4-91, ZLC5-6, ZLC5-31** and **ZLC6-1,** can effectively inhibit the growth of triple negative breast cancer cells MDA-MB-231, with IC₅₀ of 610.4 nM, 622.5 nM, 497.9 nM, 389.0 nM and 288.6 nM for the MDA-MB-231 cell line, respectively.

### Example 57: In Vivo Pharmacokinetic Experiments of Representative Compounds (zlc-4-91 and zlc-4-93) as Degradation Agent for Cyclin-dependent Kinase 12/13 (CDK12/13)

Pharmacokinetics and bioavailability tests in rats. SD rats were given a single oral (10 mg/kg), intravenous (2.5 mg/kg) and intraperitoneal (10 mg/kg) dose, followed by animal blood sample collection at appropriate time points. Heparin was added for anticoagulation, and the samples were centrifuged at 8000 rpm for 6 min. The supernatant was collected and stored at -20°C for HPLC-MS analysis. The blood samples underwent protein precipitation with acetonitrile at 12 000 rpm * 10 min, and the supernatant was used for HPCL-MS analysis. The data underwent parameter fitting with DAS2.0, to obtain compartmental model and non-compartmental model parameters, respectively. The oral bioavailability of the compounds was calculated based on the area under the plasma concentration-time curve (AUC) data. The results are shown in Table 1. The experimental results (Table 1) show that the representative compounds **zlc-4-91** and **zlc-4-93** have excellent oral pharmacokinetic properties.

**Table 1. Pharmacokinetic experimental results of compound zlc-4-93**

| Parameter | **zlc-4-91 (mg/kg)** | | **zlc-4-93 (mg/kg)** | |
|---|---|---|---|---|
| | i.v. (2.5) | Oral (10) | i.v. (2.5) | Oral (10) |
| T_{1/2}(h) | 2.46 | 2.92 | 2.38 | 3.04 |
| Tₘₐₓ(h) | 0.08 | 4.67 | 0.08 | 4.00 |
| Cₘₐₓ(ng/mL) | 4586.27 | 1309.81 | 2593.29 | 308.30 |
| AUC(0-t)(h*ng/mL) | 6815.36 | 12759.01 | 4722.41 | 3108.90 |
| AUC(0-∞)(h*ng/mL) | 6875.03 | 12833.57 | 4741.52 | 3128.96 |
| CL(mL/h/kg) | 383.07 | / | 591.09 | / |
| MRT(0-t) | 2.20 | 6.09 | 2.37 | 6.06 |
| MRT(0-∞) | 2.32 | 6.21 | 2.44 | 6.19 |
| F(%) | / | 46.80 | / | 16.46 |

| | | | | |
|---|---|---|---|---|
| *C*ₘₐₓ refers to maximum blood concentration, *T*_{1/2} refers to half life, CL refers to clearance rate, and F refers to bioavailability. | | | | |

### Example 58: Tests for Degradation Activity of Representative Compounds on CDK12/13 in 22RV1 Cells

Experimental methods: The cell line, 22RV1 cell line was purchased from the American Type Culture Collection (ATCC). Conventional Western Blot was used for detection as follows: 22RV1 cells were seeded in a certain number in a 12-well plate, and cultured overnight in an incubator in an adherent manner. Then, a certain concentration of compounds was added to act for 6 hours. The cells were lysed with a lysis buffer, and samples were collected. Then, an appropriate amount of the samples was taken for SDS-PAGE electrophoresis. After electrophoresis, the proteins were transferred to nitrocellulose membranes using a semi-dry electro-transfer system. The nitrocellulose membrane was placed in a blocking solution (5% skim milk powder diluted in TBS containing 0.1% Tween 20) and blocked at room temperature for 2 h. The membranes were then placed in a primary antibody solution (1: 1000 diluted in TBS containing 0.1% Tween 20), respectively, and incubated at 4°C overnight. The cells were washed three times with TBS containing 0.1% Tween 20, each time for 15 min. The membrane was placed in a secondary antibody solution (horseradish peroxidase-labeled goat anti-rabbit IgG, diluted at 1:1000 in TBS containing 0.1% Tween 20) and reacted at room temperature for 1 h. After washing the membrane three times as above, the membrane was developed with ECL plus reagent and photographed using Amersham Imager 600 system.

The results in FIG. 4 shows that some of the compounds can effectively degrade CDK12 and CDK13 protein levels in cells, such as compounds **YJZ9069, YJZ1102, YJZ1105, YJZ1201, YJZ1202, YJZ1203, YJZ1204, YJZ1097, YJZ1205** and **YJZ1206,** etc. Among them, compound **YJZ9069** can degrade CDK12 and CDK13 proteins in 22RV1 cells in a dose-dependent manner. After treatment of 22RV1 cells by compound **YJZ9069** at a concentration of 0.5 µM for 15 hours, CDK12 and CDK13 proteins in cells are almost completely degraded, and phosphorylation of serine at position 2 (phoSer2) of the C-terminal domain of RNA polymeraseII (RNA Pol II) are inhibited in a dose-dependent manner.

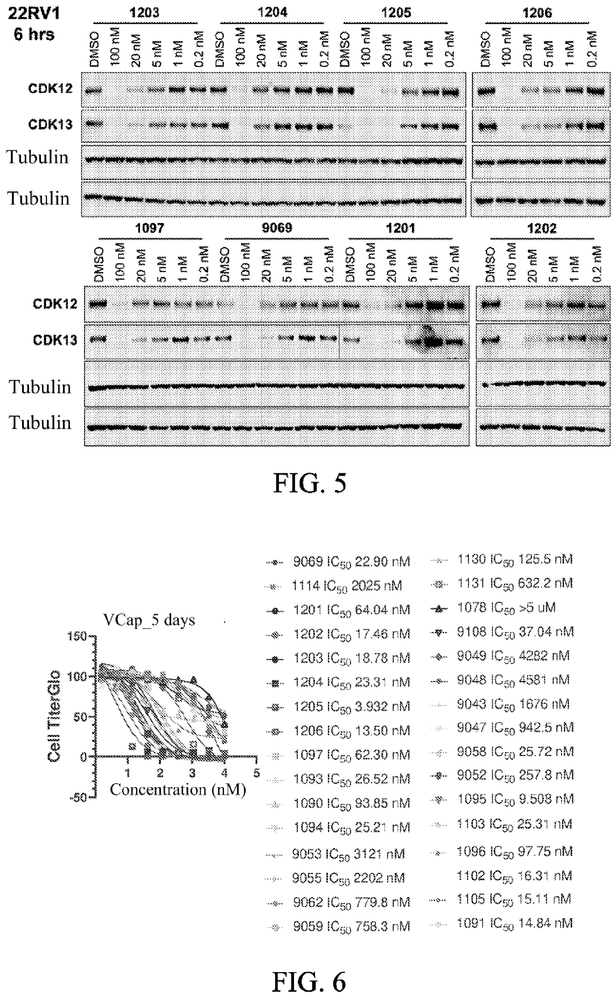

We further determined the degradation of CDK12 and CDK13 proteins in 22RV1 cells by representative compounds **YJZ9069, YJZ1201, YJZ1202, YJZ1203, YJZ1204, YJZ1097, YJZ1205,** and **YJZ1206** at different concentrations (FIG. 2). WB experiment results show that the representative compounds **YJZ9069, YJZ1201, YJZ1202, YJZ1203, YJZ1204, YJZ1097, YJZ1205,** and **YJZ1206** can degrade CDK12 and CDK13 protein levels in 22RV1 cells in a dose-dependent manner, and almost completely degrade CDK12 and CDK13 proteins in cells at a concentration of 100 nM (FIG. 5).

FIG. 4 is(A) WB results of degradation of CDK12 and CDK13 proteins after treatment of 22RV1 cells by some compounds at a concentration of 500 nM for 6 hours. (B) compound **YJZ9069** degrades CDK12 and CDK13 proteins in 22RV1 cells in a dose-dependent manner and inhibits phosphorylation of serine at position 2 (phoSer2) in the C-terminal domain of RNA polymerase II (RNA Pol II) in a dose-dependent manner; (C) WB results of degradation of CDK12 and CDK13 proteins after treatment of LnCap cells by some compounds at a concentration of 500 nM for 6 hours; (D) the protein levels by quantifying the gray value of the bands in the WB result images (FIGs. A and C) using the control group DMSO as the reference standard.

FIG. 5 is WB measurement results of the intracellular CDK12 and CDK13 protein levels after treatment of 22RV1 cells by the representative compounds **YJZ9069, YJZ1201, YJZ1202, YJZ1203, YJZ1204, YJZ1097, YJZ1205** and **YJZ1206** at different concentrations for 6 h.

### Example 59: Study on the proliferation-inhibiting activity of compounds on prostate cancer VCap cells

The cell proliferation-inhibiting activity of the compounds was determined using the CellTiter-Glo Luminescent Cell Viability Assay (Promega, Madison, WI). The cells were seeded in a 96-well plate in their respective culture media and incubated at 37°C in a 5% CO₂ incubator. After overnight incubation, serial dilutions of the compounds were prepared and added to the 96-well plate. A volume of CellTiter-Glo reagent equal to the volume of the cell culture medium present in each well was added (e.g., 100 microliters of the reagent was added to 100 microliters of the culture medium for the cells in the 96-well plate). The contents were mixed on an orbital shaker for 2 min to induce cell lysis. The 96-well plate was incubated at room temperature for 10 minutes to stabilize a luminescent signal. Luminescence was recorded: the luminescence signal of each well was acquired using an Infinite M1000 Pro plate reader (Tecan, Zürich, Switzerland), and the data were analyzed using GraphPad Prism software (GraphPad Software Inc, La Jolla, CA).

The results in FIG. 6 show that the compounds that can effectively degrade CDK12 and CDK13 protein levels, such as **YJZ9069, YJZ1102, YJZ1105, YJZ1201, YJZ1202, YJZ1203, YJZ1204, YJZ1097, YJZ1205** and **YJZ1206,** can potently inhibit the growth of prostate cancer Vcap cells, with IC₅₀ of 22.90 nM, 16.31 nM, 15.11 nM, 64.4 nM, 17.46 nM, 18.78 nM, 23.31 nM, 3.93 nM, and 13.50 nM for the VCap cell line, respectively. The compounds that cannot effectively induce degradation of CDK12 and CDK13 proteins, such as **YJZ9055** and **YJZ1078,** have IC₅₀ of 2202 nM and >5µM, respectively, for the VCap cell line, indicating that the effective inhibition of the growth of Vcap cells by this series of compounds is due to the degradation of CDK12 and CDK13 proteins.

### Example 60: In Vivo Pharmacokinetic Experiments of Representative Compounds YJZ9069, YJZ1102, YJZ1105, YJZ1201, YJZ1202, YJZ1203, YJZ1204, YJZ1097, YJZ1205 and YJZ1206, as Degradation Agent for Cyclin-dependent Kinase 12/13 (CDK12/13)

Pharmacokinetics and bioavailability tests in rats. SD rats were given a single oral (10 mg/kg), intravenous (2.5 mg/kg) and intraperitoneal (10 mg/kg) dose, followed by animal blood sample collection at appropriate time points. Heparin was added for anticoagulation, and the samples were centrifuged at 8000 rpm for 6 min. The supernatant was collected and stored at -20°C for HPLC-MS analysis. The blood samples underwent protein precipitation with acetonitrile at 12 000 rpm * 10 min, and the supernatant was used for HPCL-MS analysis. The data underwent parameter fitting with DAS2.0, to obtain compartmental model and non-compartmental model parameters, respectively. The oral bioavailability of the compounds was calculated based on the area under the plasma concentration-time curve (AUC) data. The results are shown in Tables 2-6. The experimental results (Tables 2-6) show that the representative compounds **YJZ1102, YJZ1105, YJZ1201, YJZ1202, YJZ1203, YJZ1204, YJZ1097, YJZ1205** and **YJZ1206** have excellent oral pharmacokinetic properties.

**Table 2. Pharmacokinetic experimental results of compounds YJZ9069 and YJZ1102**

| Parameter | **YJZ9069** (Compound of Example 23) | | **YJZ1102** (Compound of Example 44) | | |
|---|---|---|---|---|---|
| | PO (10 mg/kg) | IV (2.5 mg/kg) | IV (2.5 mg/kg) | PO (10 mg/kg) | IP (10 mg/kg) |
| *T*_{1/2}(h) | NA | 4.39 | 10.67 | 10.38 | 26.4 |
| *T*ₘₐₓ | 4.0 | 0.08 | 0.08 | 6.0 | 6.67 |
| *C*ₘₐₓ (ng/mL) | 120.96 | 3507.58 | 2203.73 | 147.55 | 661.64 |
| CL (mL/h/kg) | NA | 848.1 | 199.42 | NA | NA |
| *F (%)* | 5.04% | | | 17.28% | 94.15% |

| | | | | | |
|---|---|---|---|---|---|
| *C*ₘₐₓ refers to maximum blood concentration, *T*_{1/2} refers to half life, CL refers to clearance rate, and *F* refers to bioavailability. | | | | | |

**Table 3. Pharmacokinetic experimental results of compounds YJZ1105 and YJZ1202**

| Parameter | **YJZ1105** (Compound | of Example 45) | **YJZ1202** (Compound of Example 53) | |
|---|---|---|---|---|
| | IV (2.5 mg/kg) | PO (10 mg/kg) | IV (2.5 mg/kg) | PO (10 mg/kg) |
| *T*_{1/2}(h) | 7.05 | 7.2 | 4.13 | 3.76 |
| *T*ₘₐₓ | 0.08 | 6 | 0.08 | 5.33 |
| *C*ₘₐₓ (ng/mL) | 1385.47 | 625.4 | 2309.49 | 290.81 |
| CL (mL/h/kg) | 288.16 | NA | 401.73 | NA |
| *F (%)* | 26.64% | | 13.51% | |

| | | | | |
|---|---|---|---|---|
| *C*ₘₐₓ refers to maximum blood concentration, *T*_{1/2} refers to half life, CL refers to clearance rate, and *F* refers to bioavailability. | | | | |

**Table 4. Pharmacokinetic experimental results of compounds YJZ1203 and YJZ1204**

| Parameter | **YJZ1203** (Compound of Example 50) | | **YJZ1204** (Compound of Example 54) | |
|---|---|---|---|---|
| | IV (2.5 mg/kg) | PO (10 mg/kg) | IV (2.5 mg/kg) | PO (10 mg/kg) |
| *T*_{1/2}(h) | 6.19 | 6.4 | 8.54 | 10.85 |
| *T*ₘₐₓ | 0.08 | 6.7 | 0.08 | 7.33 |
| *C*ₘₐₓ (ng/mL) | 3063.71 | 382.2 | 2293.02 | 456.88 |
| CL (mL/h/kg) | 313.45 | NA | 360.47 | NA |
| *F* (%) | 16.95% | | 30.13% | |

| | | | | |
|---|---|---|---|---|
| *C*ₘₐₓ refers to maximum blood concentration, *T*_{1/2} refers to half life, CL refers to clearance rate, and *F* refers to bioavailability. | | | | |

**Table 5. Pharmacokinetic experimental results of compounds YJZ1205 and YJZ1201**

| Parameter | **YJZ1205** (Compound of Example 51) | | **YJZ1201** (Compound of Example 49) | |
|---|---|---|---|---|
| | IV (2.5 mg/kg) | PO (10 mg/kg) | IV (2.5 mg/kg) | PO (10 mg/kg) |
| *T*_{1/2}(h) | 3.81 | 4.2 | 10.78 | 12.36 |
| *T*ₘₐₓ | 0.08 | 4.7 | 0.08 | 6.67 |
| *C*ₘₐₓ (ng/mL) | 3623.58 | 580.8 | 2510.82 | 1037.26 |
| CL (mL/h/kg) | 235.84 | NA | 195.99 | NA |
| *F (%)* | 15.87% | | 39.60% | |

| | | | | |
|---|---|---|---|---|
| *C*ₘₐₓ refers to maximum blood concentration, *T*_{1/2} refers to half life, CL refers to clearance rate, and *F* refers to bioavailability. | | | | |

**Table 6. Pharmacokinetic experimental results of compounds YJZ1206 and YJZ1097**

| Parameter | **YJZ1206** (Compound of Example 52) | | **YJZ1097** (Compound of Example 48) | |
|---|---|---|---|---|
| | IV (2.5 mg/kg) | PO (10 mg/kg) | IV (2.5 mg/kg) | PO (10 mg/kg) |
| *T*_{1/2}(h) | 5.15 | 6.24 | 13.82 | 15.2 |
| *T*ₘₐₓ | 0.08 | 6.0 | 0.08 | 6.7 |
| *C*ₘₐₓ (ng/mL) | 3117.16 | 1129.63 | 2491.60 | 592.6 |
| CL (mL/h/kg) | 257.41 | NA | 187.29 | NA |
| *F* (%) | 39.16% | | 23.89% | |

| | | | | |
|---|---|---|---|---|
| *C*ₘₐₓ refers to maximum blood concentration, *T*_{1/2} refers to half life, CL refers to clearance rate, and *F* refers to bioavailability. | | | | |

All documents mentioned herein are incorporated by reference into this application to the same extent as if each individual document were incorporated by reference individually. The above-mentioned examples only express several embodiments of the present invention, and the description thereof is relatively specific and detailed, but it should not be understood as limiting the scope of the invention patent. The technical features of the above-mentioned examples can be combined arbitrarily. In order to make the description concise, not all possible combinations of the technical features in the above-mentioned examples are described. However, as long as there is no contradiction in these combinations of the technical features, they should be considered to be within the scope of this specification.

## Claims

1. A compound having a structure of formula (I), or a pharmaceutically acceptable salt thereof, or a pharmaceutical composition thereof, or a prodrug molecule thereof: 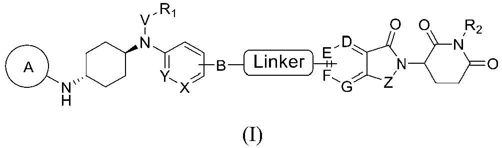
wherein Z is: CH₂ or CO;
V is selected from the group consisting of: 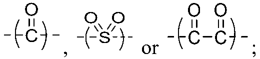
X and Y are each independently selected from the group consisting of: N, CH or CR₃; wherein R₃ is selected from the group consisting of: halogen, cyano, hydroxyl, amino, C₁-C₃ alkyl, halogenated C₁-C₃ alkyl, C₁-C₃ alkoxy, halogenated C₁-C₃ alkoxy, C₃-C₈ cycloalkyl , or 3- to 8-membered heterocyclyl;
B is selected from the group consisting of: 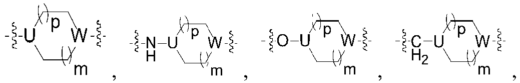 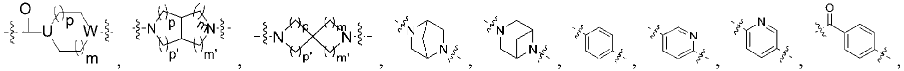 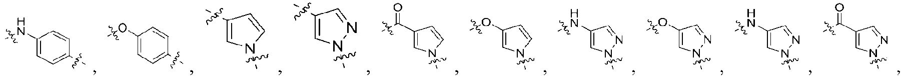 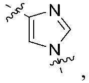 NH, O, CO, or CH₂; wherein each of U and W is independently selected from the group consisting of: N or CH; each of m, p, m' and p' is independently: 0, 1, 2 or 3;
A ring is selected from the group consisting of: 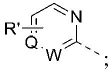
wherein Q and W are each independently selected from: CH or N;
R' is independently selected from: hydrogen, halogen, cyano, hydroxy, substituted hydroxy, amino, substituted amino, C₁-C₅ alkyl, halogenated C₁-C₅ alkyl, C₁-C₅ alkoxy, halogenated C₁-C₅ alkoxy, or C₃-C₈ cycloalkyl, and when Q and W are independently selected from CH, R' may be a substituent on Q or W;
D, E, F, and G are each independently selected from the group consisting of: CH, N, or CR₆; wherein R₆ is selected from the group consisting of: halogen, trifluoromethyl, hydroxy, cyano, amino, methyl, methoxy, or trifluoromethoxy; and when any one of D, E, F, and G is CH, the atom can serve as an attachment site with linker, and in this case, D, E, F, or G is C;
R₁ is selected from the group consisting of: H, -NHR₇, -OR₇, or -(C(R₉)R₈)R₇;
wherein R₇ is -R₁₀, -CH₂R₁₀ or -(CH₂)₂R₁₀;
R₈ and R₉ are each independently selected from the group consisting of: hydrogen, halogen, cyano, methyl, halomethyl, methoxy, halomethoxy, ethyl, haloethyl, ethoxy, haloethoxy, hydroxy, amino, or 3- to 8-membered heterocyclic ring containing 1, 2 or 3 heteroatoms, and the heteroatoms are selected from O, S or N;
or R₈ and R₉, together with the C atom to which they are attached, form a 3- to 7-membered heterocyclic ring;
R₁₀ is selected from the group consisting of:
1) cyano, C₁-C₃alkyl, halogenated C₁-C₄alkyl, C₁-C₄alkoxy, C₃-C₁₀cycloalkyl, substituted or unsubstituted 5- to 12-membered aromatic ring, or substituted or unsubstituted 3- to 12-membered heterocyclic ring; or
2) 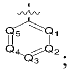 wherein Q₁, Q₂, Q₃, Q₄ and Q₅ are each independently selected from: CH, N or CR₁₁;
each R₁₁ is independently selected from the group consisting of: halogen, cyano, hydroxy, amino, nitro, C₁-C₃ alkyl, halogenated C₁-C₃ alkyl, C₁-C₄ alkoxy, halogenated C₁-C₄ alkoxy, or C₃-C₈ cycloalkyl;
R₂ is selected from the group consisting of: H, C₁-C₃ alkyl, 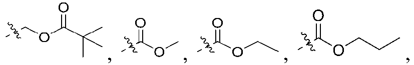 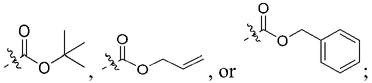
when B is selected from the group consisting of: 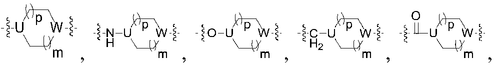 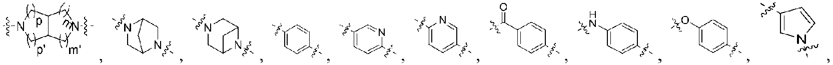 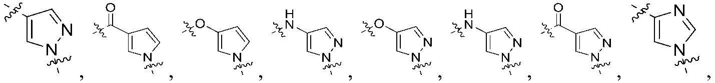 NH, O, CO, or CH₂; wherein each of U and W is independently selected from the group consisting of: N or CH; each of m, p, m' and p' is independently: 0, 1, 2 or 3;
Linker is: 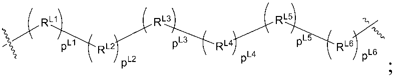
wherein,
R^{L1}, R^{L2}, R^{L3}, R^{L4} and R^{L5} are the same or different and are each independently selected from the substituted or unsubstituted groups in the group consisting of: a chemical bond, CH₂, CHD, CD₂, C=O, O, NH, SO, SO₂, P=O, NHCO, NHSO₂, OCH₂, OCH₂CH₂, CH₂OCH₂, NHCH₂, NMeCH₂, NHCH₂CH₂, NMeCH₂CH₂, CH₂NHCO, NHCOCH₂, 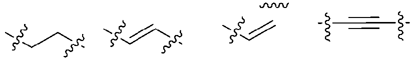 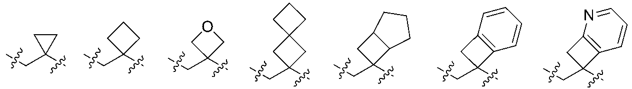 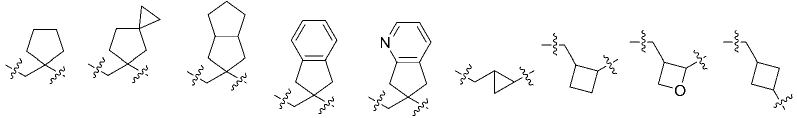 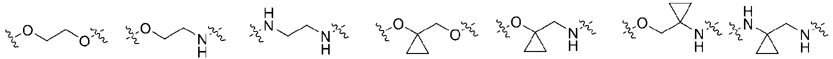 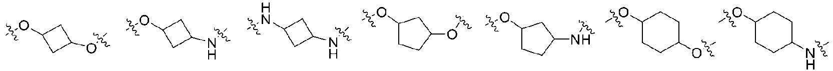 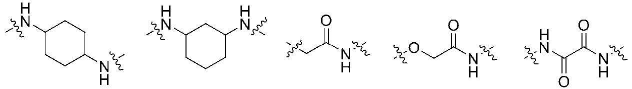
R^{L6} is a ring and optionally selected from the following structure: 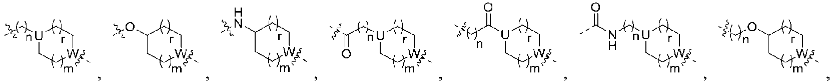 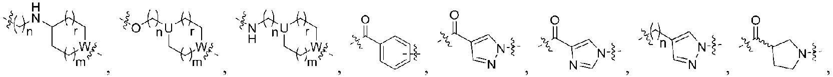 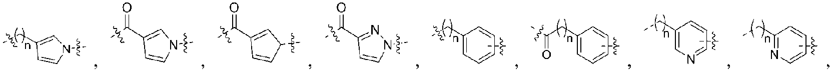 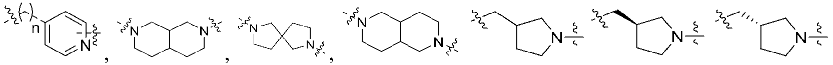 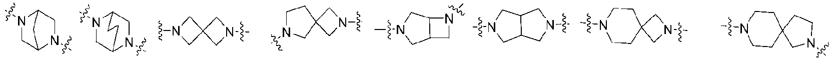 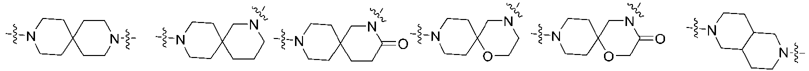 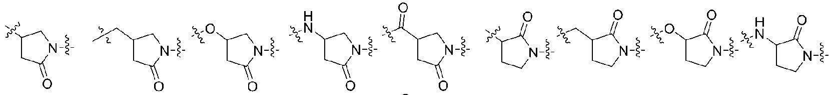 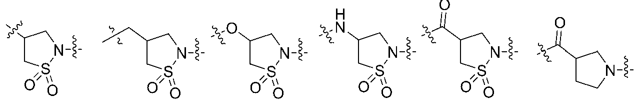 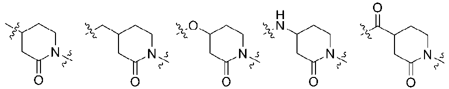 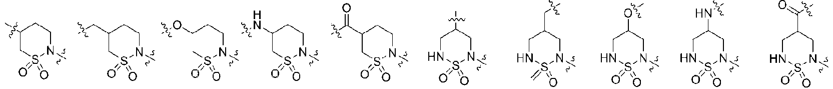 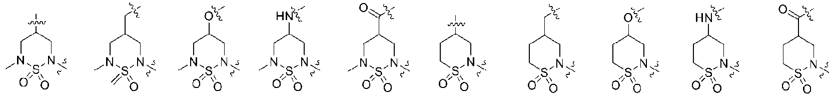 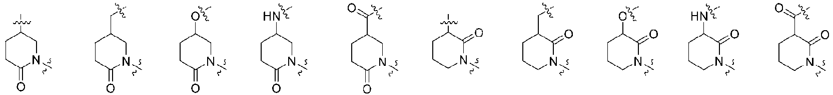 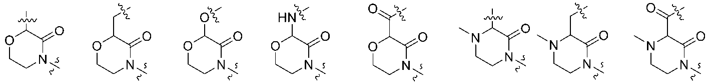 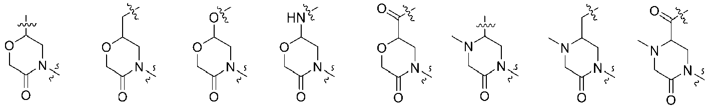 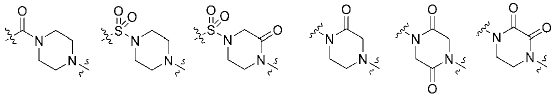 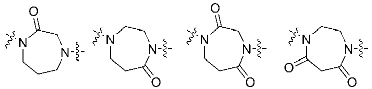 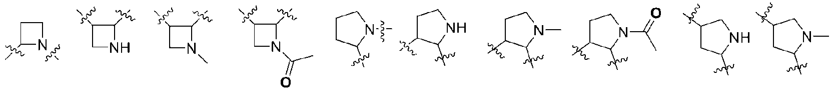 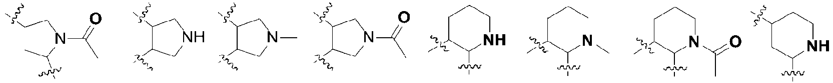 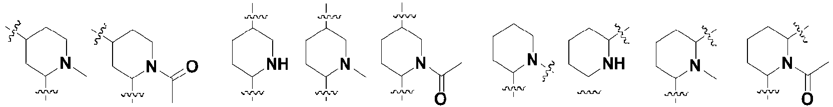 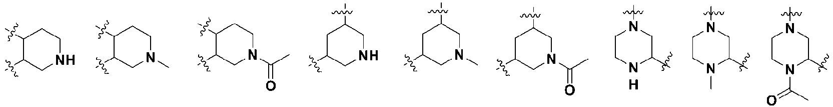 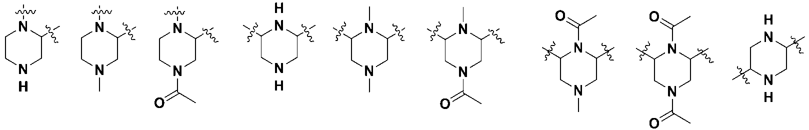 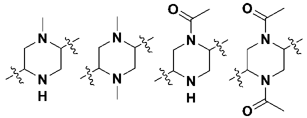 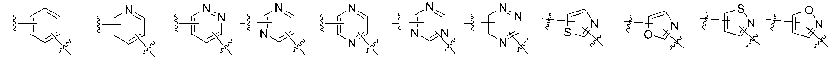 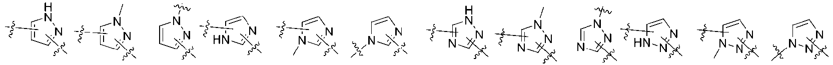 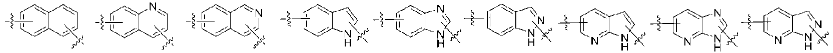 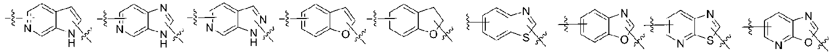 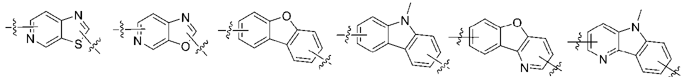 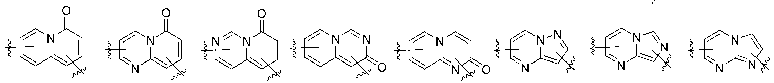 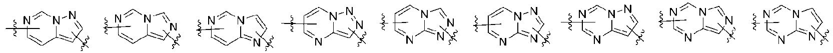 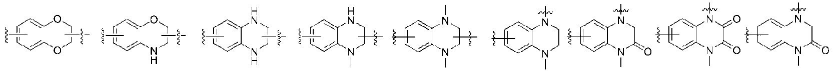 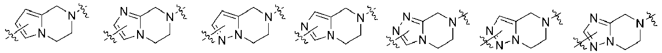 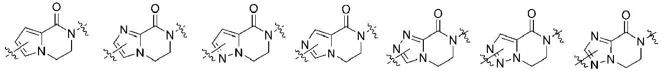 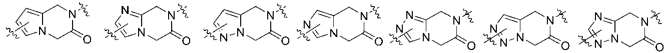
wherein each n is independently 0, 1, 2, 3, 4, 5 or 6; each of r and m is independently 0, 1 or 2; each of U and W is independently selected from the group consisting of: N or CH;
p^{L1}, p^{L2}, p^{L3}, p^{L4}, p^{L5} and p^{L6} are each independently selected from 0, 1, 2, 3, 4, 5 or 6; or
when B is 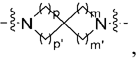 where each of m, p, m' and p' is independently: 0, 1, 2 or 3;
Linker is: 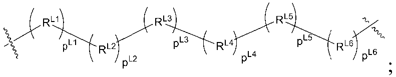
wherein,
R^{L1}, R^{L2}, R^{L3}, R^{L4}, R^{L5} and R^{L6} are the same or different and are each independently selected from the substituted or unsubstituted groups in the group consisting of: a chemical bond, CH₂, CHD, CD₂, C=O, O, NH, SO, SO₂, P=O, NHCO, NHSO₂, OCH₂, OCH₂CH₂, CH₂OCH₂, NHCH₂, NMeCH₂, NHCH₂CH₂, NMeCH₂CH₂, CH₂NHCO, NHCOCH₂, 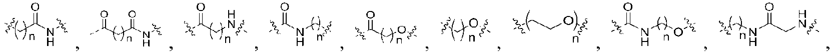 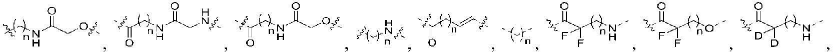 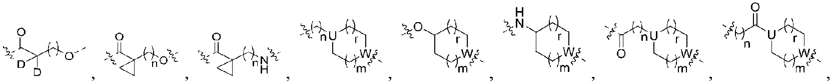 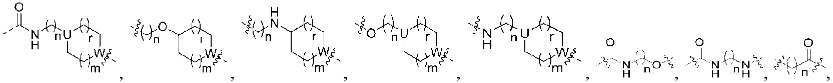 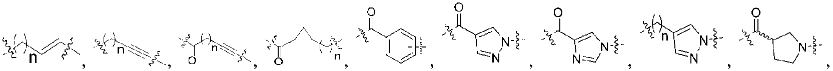 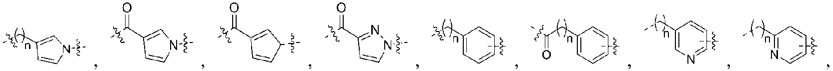 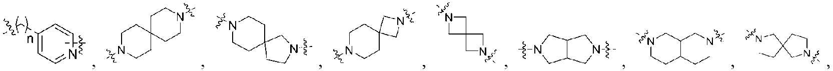 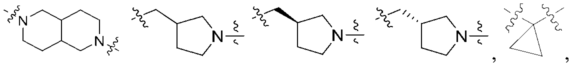 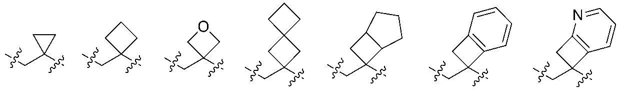 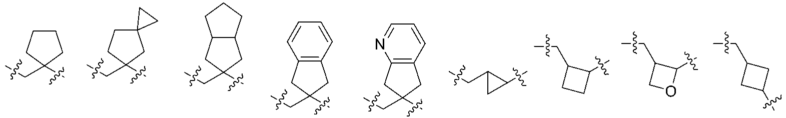 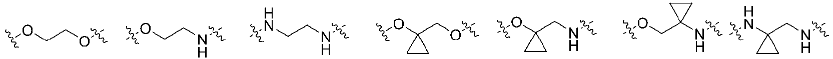 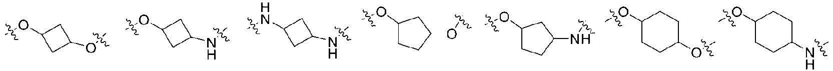 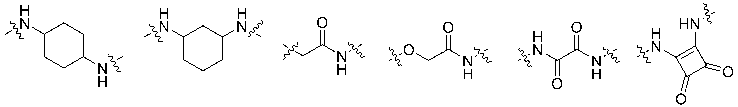 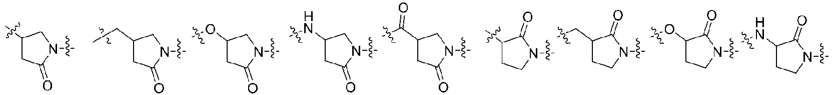 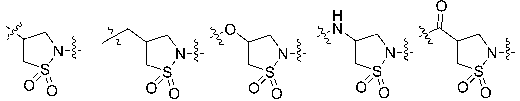 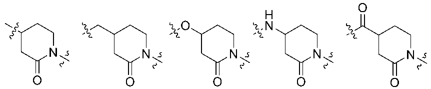 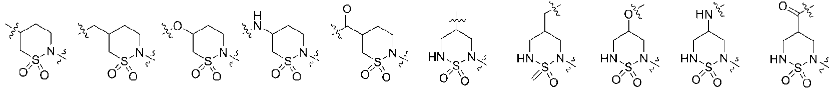 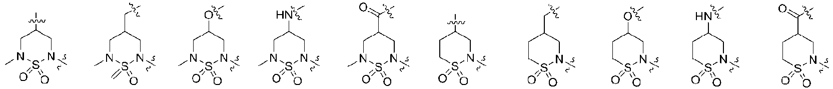 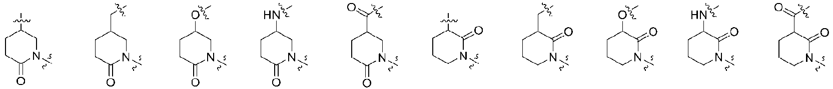 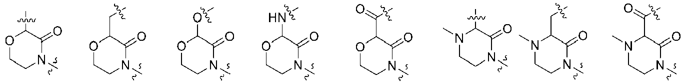 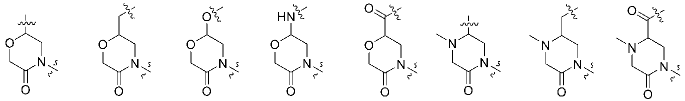 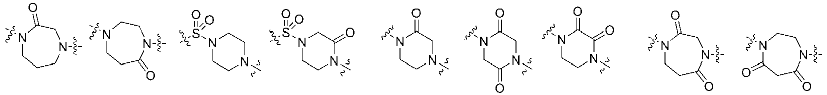 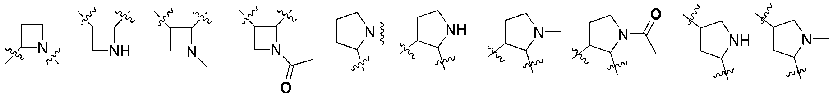 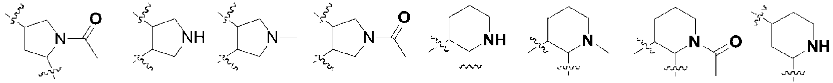 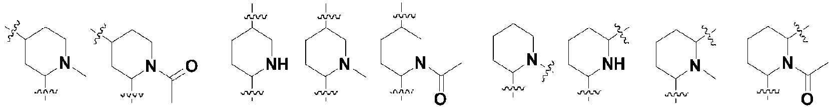 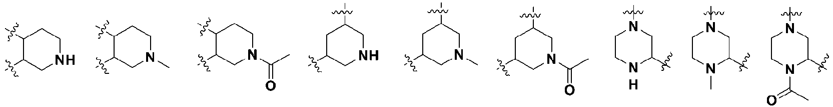 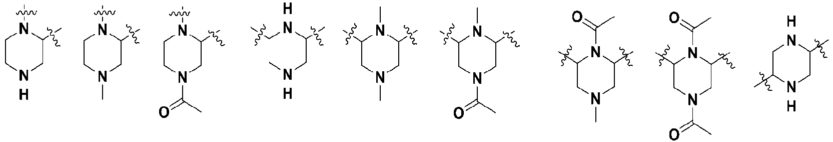 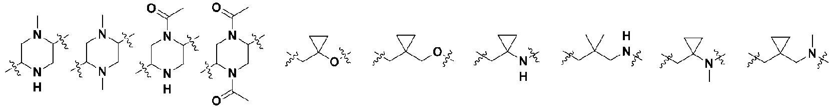 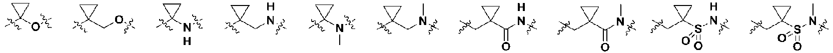 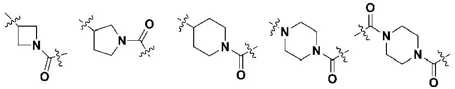 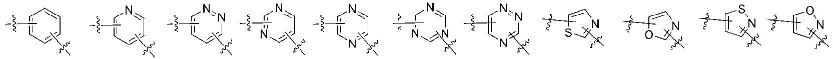 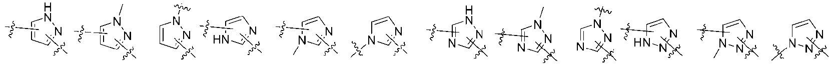 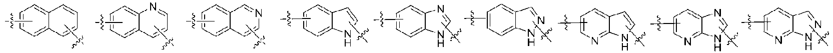 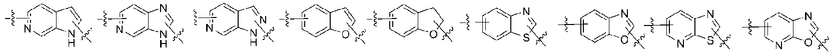 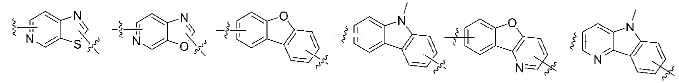 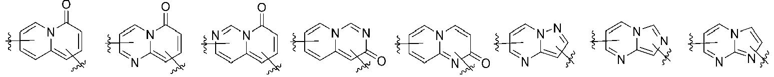 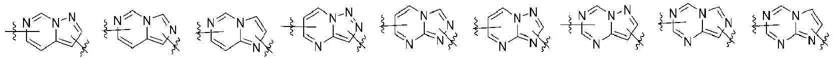 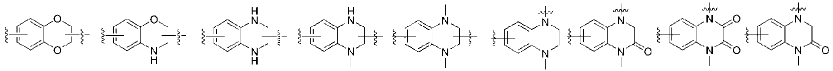 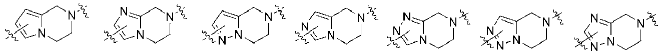 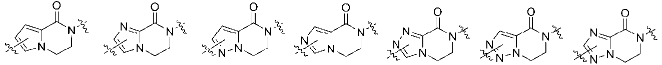 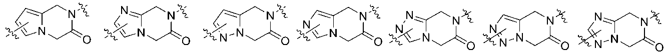
wherein each n is independently 0, 1, 2, 3, 4, 5 or 6; each of r and m is independently 0, 1 or 2; each of U and W is independently selected from the group consisting of: N or CH;
p^{L1}, p^{L2}, p^{L3}, p^{L4}, p^{L5} and p^{L6} are each independently selected from 0, 1, 2, 3, 4, 5 or 6.

2. The compound according to claim 1, or a pharmaceutically acceptable salt thereof, or a stereoisomer thereof, or a prodrug molecule thereof, wherein
B is selected from the group consisting of: 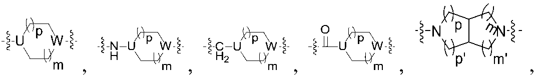 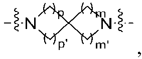 NH, O, or CO;
wherein each of U and W is independently selected from the group consisting of: N or CH; each of m, p, m' and p' is independently 0, 1, 2 or 3.

3. The compound according to claim 1, or a pharmaceutically acceptable salt thereof, or a stereoisomer thereof, or a prodrug molecule thereof, wherein
A ring is selected from the group consisting of: 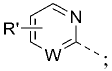
wherein W is selected from the group consisting of: CH or N;
R' is selected from: hydrogen, halogen, cyano, hydroxy, amino, C₁-C₃ alkyl, halogenated C₁-C₃ alkyl, C₁-C₅ alkoxy, halogenated C₁-C₅ alkoxy, or C₃-C₈ cycloalkyl.

4. The compound according to claim 1, or a pharmaceutically acceptable salt thereof, or a stereoisomer thereof, or a prodrug molecule thereof, wherein the D, E, F, and G are CH or CR₆; wherein R₆ is selected from the group consisting of: halogen.

5. The compound according to claim 1, or a pharmaceutically acceptable salt thereof, or a stereoisomer thereof, or a prodrug molecule thereof, wherein
when B is selected from the group consisting of: 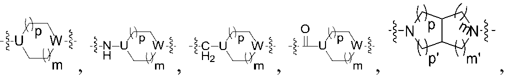 NH, O, or CO; the Linker is selected from the group consisting of: 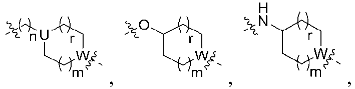 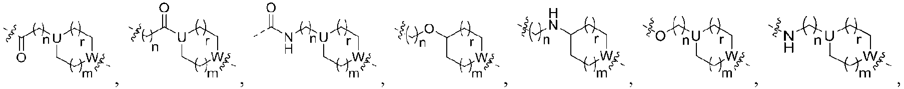 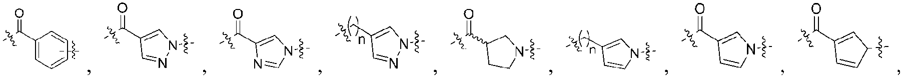 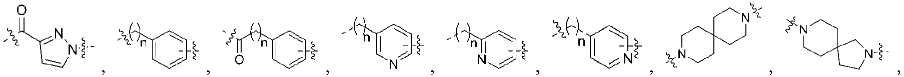 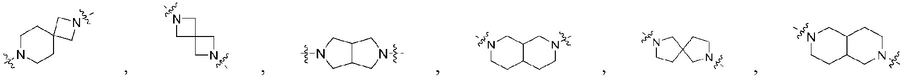 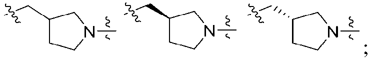
and when B is 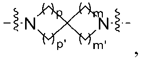 the Linker is selected from the group consisting of: a chemical bond,

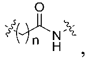

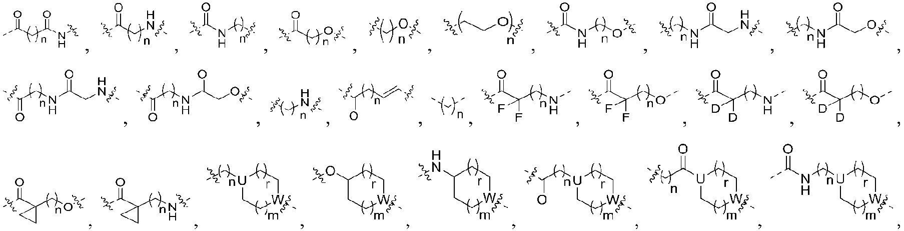

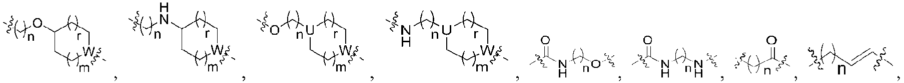

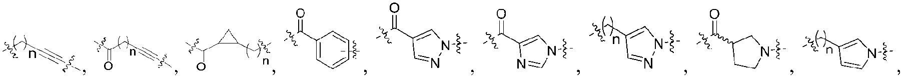

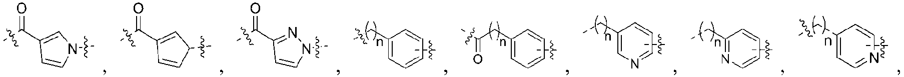

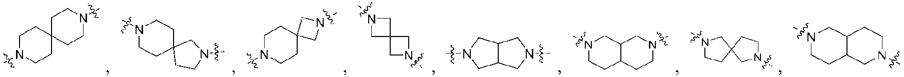

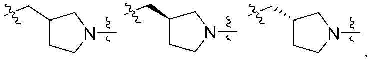

6. The compound according to claim 1, or a pharmaceutically acceptable salt thereof, or a stereoisomer thereof, or a prodrug molecule thereof, having a structure represented by formula (II): 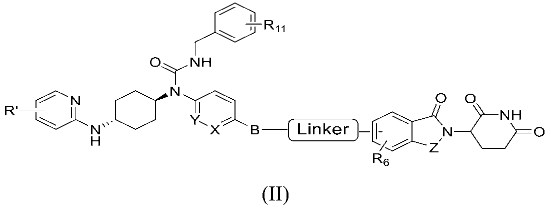 wherein B is selected from: 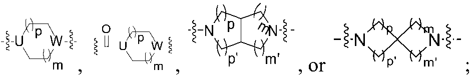
wherein each of U and W is independently selected from the group consisting of: N or CH; each of m, p, m' and p' is independently 0, 1, 2 or 3;
R' is optionally selected from: hydrogen, halogen, cyano, hydroxy or amino;
R₁₁ and R₆ are optionally selected from: hydrogen or halogen;
X and Y are independently selected from: CH, N or CR₃;
wherein R₃ is optionally selected from: halogen, cyano, hydroxy or amino;
Z is optionally selected from: CH₂ or CO.

7. A compound having a structure of formula (I), or a pharmaceutically acceptable salt thereof, or a pharmaceutical composition thereof, or a prodrug molecule thereof: 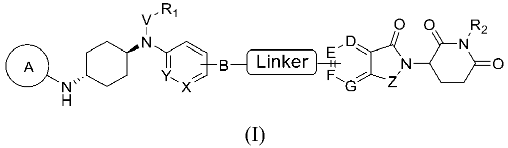
wherein Z is selected from the group consisting of: CH₂ or CO;
V is selected from the group consisting of: 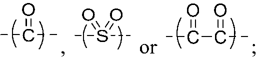
X and Y are selected from the group consisting of: N, CH or CR₃; wherein R₃ is selected from the group consisting of: halogen, cyano, hydroxyl, amino, C₁-C₃ alkyl, halogenated C₁-C₃ alkyl, C₁-C₃ alkoxy, halogenated C₁-C₃ alkoxy, C₃-C₈ cycloalkyl , or 3- to 8-membered heterocyclyl;
B is selected from the group consisting of: 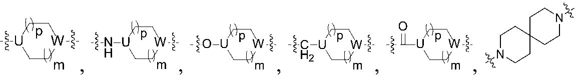 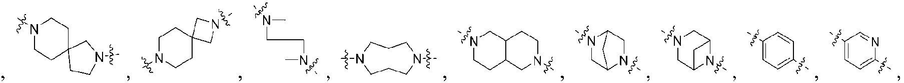 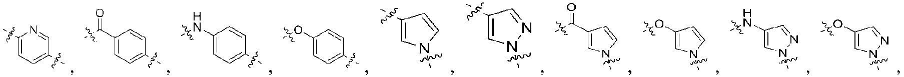 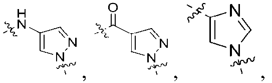
NH, O, CO, or CH₂; wherein each of U and W is independently selected from the group consisting of: N or CH; each of m and p is independently: 0, 1 or 2;
A ring is selected from the group consisting of: 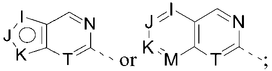
M and T are each independently selected from the group consisting of: N or CR₄;
I, J and K are each independently selected from the group consisting of:N, O, S, CR₄ and NR₄;
R₄ is selected from the group consisting of: hydrogen, halogen, cyano, hydroxy, amino, C₁-C₅ alkyl, halogenated C₁-C₃ alkyl, C₁-C₃ alkoxy, halogenated C₁-C₃ alkoxy, C₃-C₈ cycloalkyl, -CH₂R₅, -(CH₂)₂R₃, -(CH₂)₃R₅, or 3- to 8-membered heterocyclyl;
R₅ is selected from the group consisting of: cyano, hydroxy, amino, C₃-C₈cycloalkyl, or 3- to 8-membered heterocyclyl;
D, E, F, and G are each independently selected from the group consisting of: CH, N, or CR₆; wherein R₆ is selected from the group consisting of: halogen, trifluoromethyl, hydroxy, cyano, amino, methyl, methoxy, or trifluoromethoxy;
R₁ is selected from the group consisting of: H, -NHR₇, -OR₇, or -(C(R₉)R₈)R₇;
wherein R₇ is -R₁₀, -CH₂R₁₀ or -(CH₂)₂R₁₀;
R₈ and R₉ are each independently selected from the group consisting of: hydrogen, halogen, cyano, methyl, halomethyl, methoxy, halomethoxy, ethyl, haloethyl, ethoxy, haloethoxy, hydroxy, amino, or 3- to 8-membered heterocyclic ring containing 1, 2 or 3 heteroatoms, and the heteroatoms are selected from O, S or N;
or R₈ and R₉, together with the C atom to which they are attached, form a 3- to 7-membered heterocyclic ring;
R₁₀ is selected from the group consisting of:
1) cyano, C₁-C₅ alkyl, halogenated C₁-C₄ alkyl, C₁-C₄ alkoxy, C₃-C₁₀ cycloalkyl, substituted or unsubstituted 5-to 12-membered aromatic ring, or substituted or unsubstituted 3- to 12-membered heterocyclic ring; or
2) 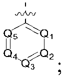 wherein Q₁, Q₂, Q₃, Q₄ and Q₅ are each independently selected from: CH, N or CR₁₁;
each R₁₁ is independently selected from the group consisting of: halogen, cyano, hydroxy, amino, nitro, C₁-C₃ alkyl, halogenated C₁-C₃ alkyl, C₁-C₄ alkoxy, halogenated C₁-C₄ alkoxy, or C₃-C₈ cycloalkyl;
R₂ is selected from the group consisting of: H, C₁-C₃ alkyl, 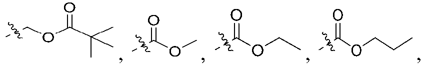 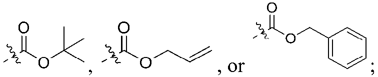
Linker is: 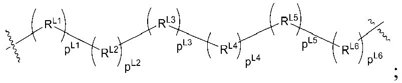
wherein R^{L1} - R^{L6} are the same or different, and are each independently selected from the substituted or unsubstituted groups in the group consisting of: a bond, CH₂, CHD, CD₂, C=O, O, NH, SO, SO₂, P=O, NHCO, NHSO₂, OCH₂, OCH₂CH₂, CH₂OCH₂, NHCH₂, NMeCH₂, NHCH₂CH₂, NMeCH₂CH₂, CH₂NHCO, NHCOCH₂, 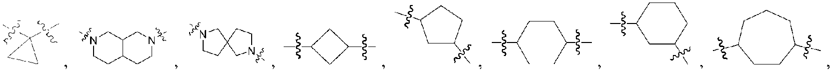 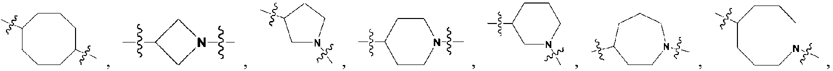 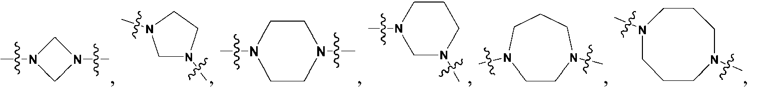 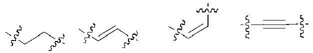 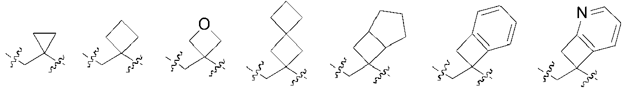 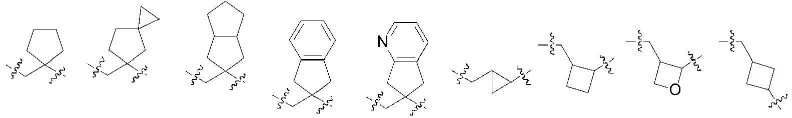 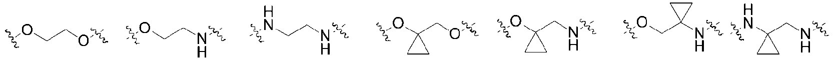 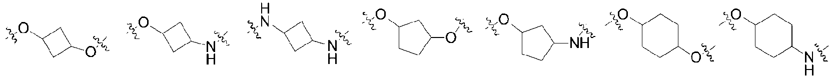 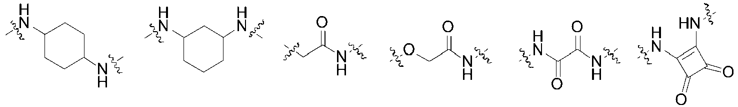 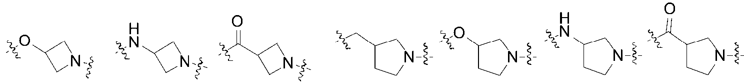 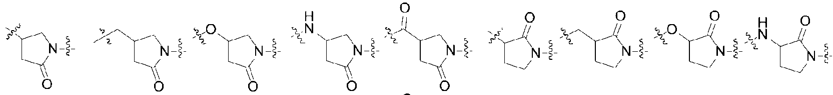 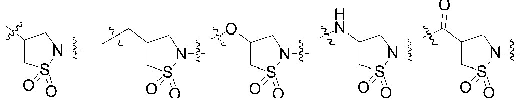 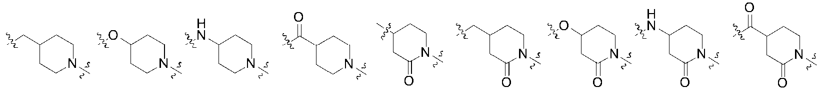 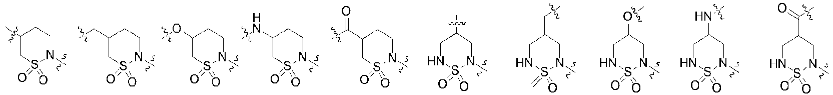 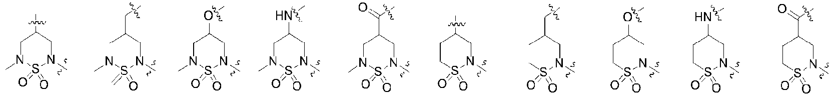 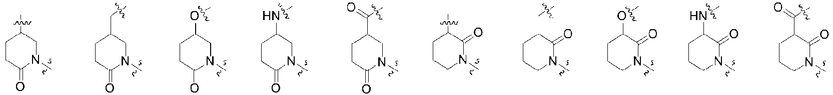 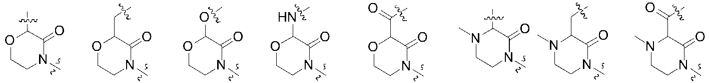 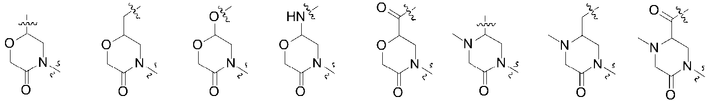 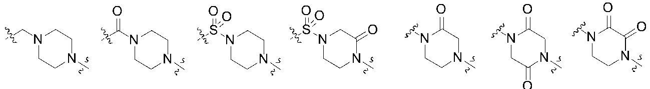 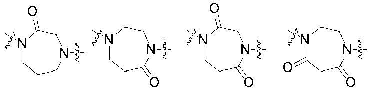 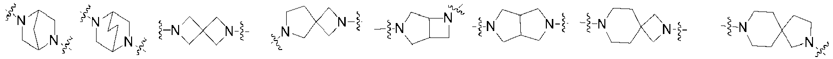 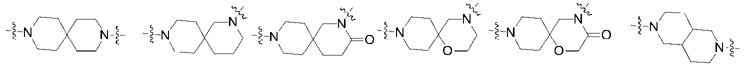 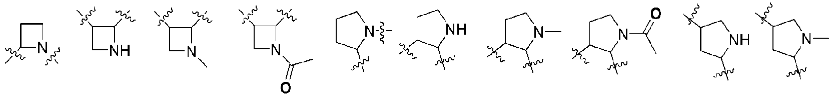 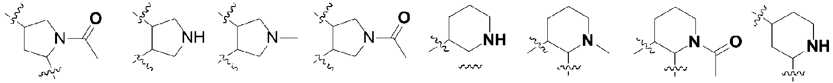 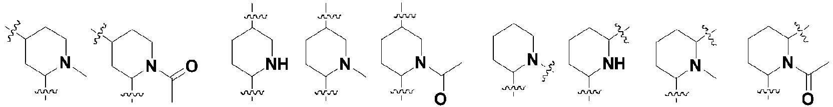 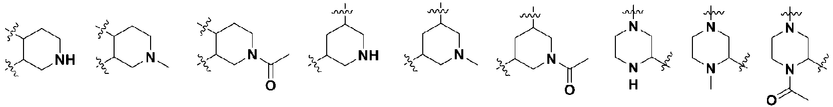 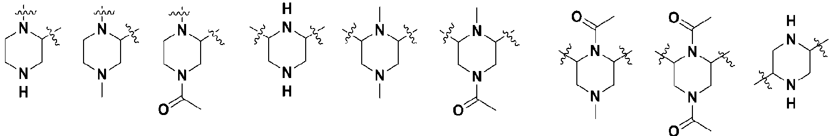 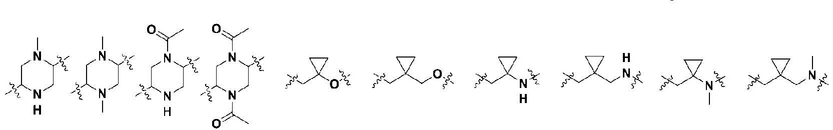 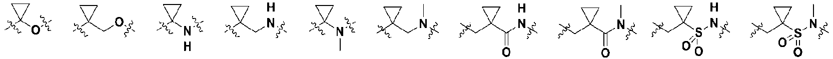 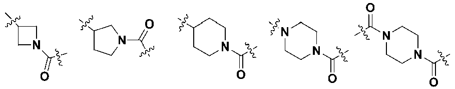 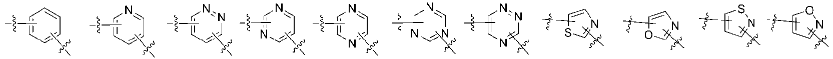 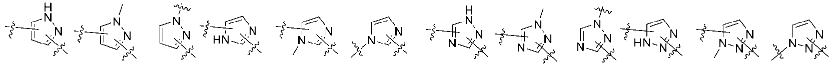 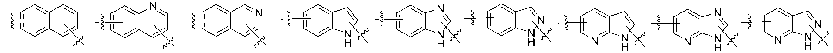 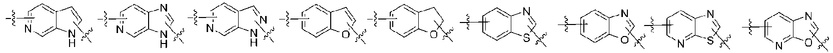 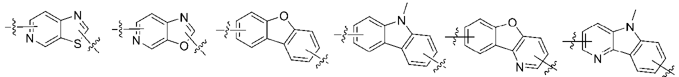 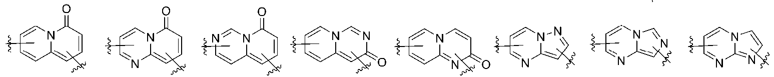 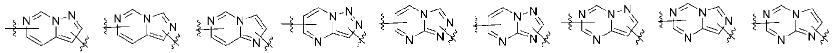 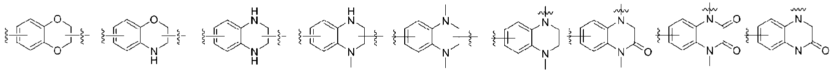 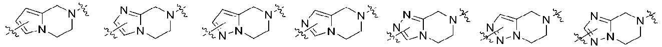 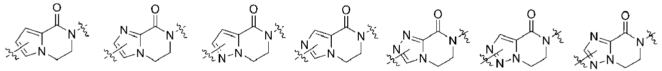 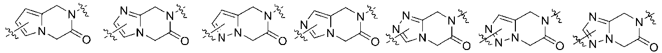
wherein each n is independently 0, 1, 2, 3, 4, 5 or 6; each of r and m is independently 0, 1 or 2; each of U and W is independently selected from the group consisting of: N or CH
the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester, amino, amido, sulfonamido, or ureido;
each of p^{L1} - p^{L6} is independently selected from 0, 1, 2, 3, 4, 5, or 6.

8. The compound according to claim 1 or 7, or a pharmaceutically acceptable salt thereof, or a stereoisomer thereof, or a prodrug molecule thereof, wherein
V is selected from the group consisting of:

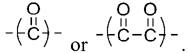

9. The compound according to claim 1 or 7, or a pharmaceutically acceptable salt thereof, or a stereoisomer thereof, or a prodrug molecule thereof, wherein
X and Y are each independently selected from the group consisting of: N, CH or CR₃;
wherein R₃ is selected from the group consisting of: halogen, cyano, hydroxy, amino, C₁-C₃ alkyl, halogenated C₁-C₃ alkyl, C₁-C₃ alkoxy, or halogenated C₁-C₃ alkoxy.

10. The compound according to claim 7, or a pharmaceutically acceptable salt thereof, or a stereoisomer thereof, or a prodrug molecule thereof, wherein
B is selected from the group consisting of: 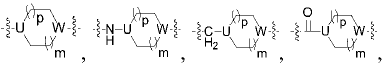 NH, O, or CO;
wherein each of U and W is independently selected from the group consisting of: N or CH; each of m and p is independently 0 or 1.

11. The compound according to claim 7, or a pharmaceutically acceptable salt thereof, or a stereoisomer thereof, or a prodrug molecule thereof, wherein
A ring is selected from the group consisting of: 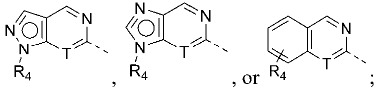
wherein T is selected from the group consisting of: CHor N.

12. The compound according to claim 7, or a pharmaceutically acceptable salt thereof, or a stereoisomer thereof, or a prodrug molecule thereof, wherein
R₄ is selected from the group consisting of: hydrogen, halogen, cyano, hydroxy, amino, C₁-C₅ alkyl, halogenated C₁-C₅ alkyl, C₁-C₅ alkoxy, halogenated C₁-C₅ alkoxy, C₃-C₈ cycloalkyl, -CH₂R₅, -(CH₂)₂R₅, -(CH₂)₃R₅, or 3- to 8-membered heterocyclyl.

13. The compound according to claim 7, or a pharmaceutically acceptable salt thereof, or a stereoisomer thereof, or a prodrug molecule thereof, wherein the D, E, F and G are CH.

14. The compound according to claim 7, or a pharmaceutically acceptable salt thereof, or a stereoisomer thereof, or a prodrug molecule thereof, wherein the R₁ is -NHR₇;
wherein R₇ is -CH₂R₁₀; R₁₀ is defined as described in claim 7.
In another preferred embodiment, the R₁₀ is selected from the group consisting of: cyano, C₁-C₃ alkyl, halogenated C₁-C₄ alkyl, C₁-C₄ alkoxy, C₃-C₁₀ cycloalkyl, 4- to 7-membered heterocyclyl, or C₆-C₁₀ aryl; wherein the aryl is substituted with one or more CR₁₁; R₁₁ is defined as described in claim 7.

15. The compound according to claim 7, or a pharmaceutically acceptable salt thereof, or a stereoisomer thereof, or a prodrug molecule thereof, wherein
the Linker is selected from the group consisting of: 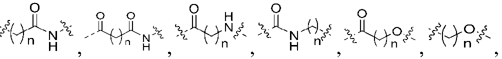 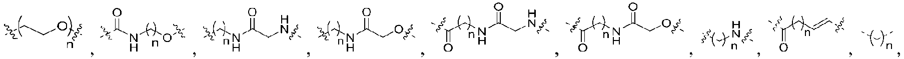 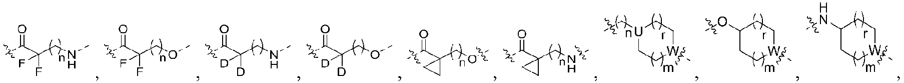 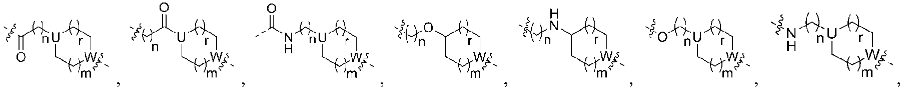 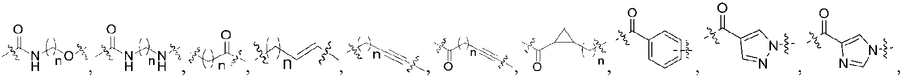 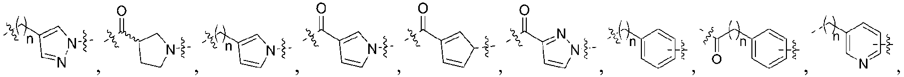 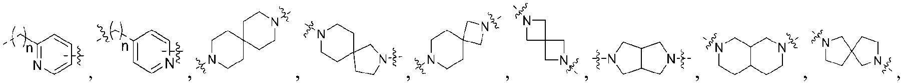 or 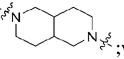 wherein each n is independently 0, 1, 2, 3, 4, 5 or 6; each of r and m is independently 0, 1 or 2; and each of U and W is independently selected from the group consisting of: N or CH.
In another preferred embodiment, the Linker is selected from the group consisting of: 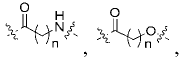 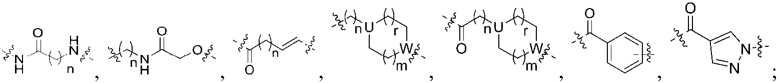
wherein each n is independently 0, 1, 2, 3, 4, 5 or 6; each of r and m is independently 0, 1 or 2; each of U and W is independently selected from the group consisting of: N or CH.

16. The compound according to claim 7, or a pharmaceutically acceptable salt thereof, or a stereoisomer thereof, or a prodrug molecule thereof, having a structure represented by formula (II) or formula (III): 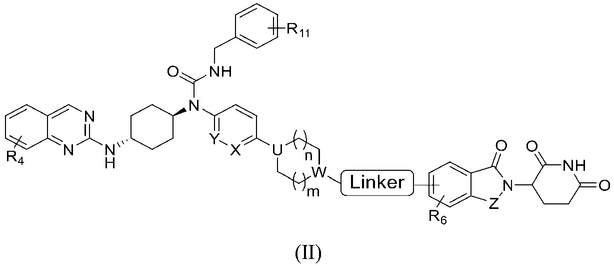 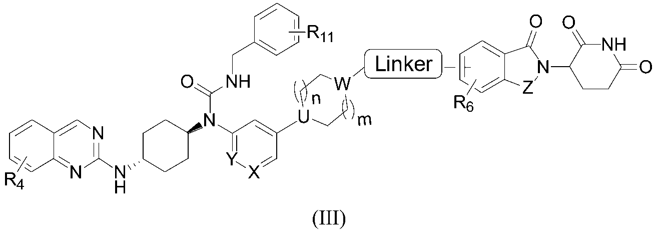 wherein U and W are independently selected from: CH or N;
X and Y are independently selected from: CH, N or CR₃;
Z is optionally selected from: CH₂ or CO;
n and m are independently selected from: 0 or 1.

17. The compound according to claim 1, or a pharmaceutically acceptable salt thereof, or a stereoisomer thereof, or a prodrug molecule thereof, wherein the compound is selected from the group consisting of:

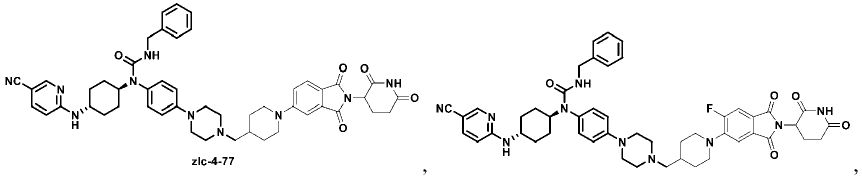

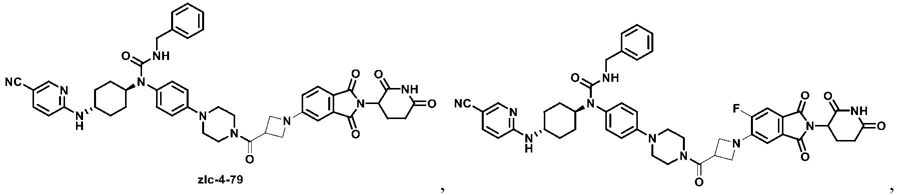

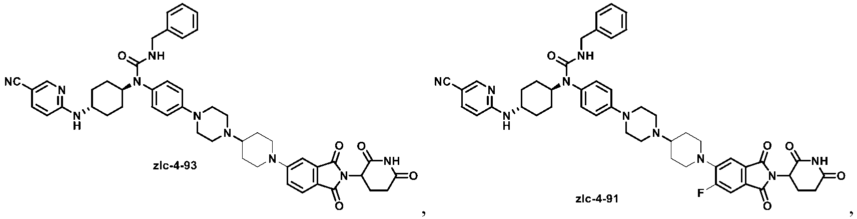

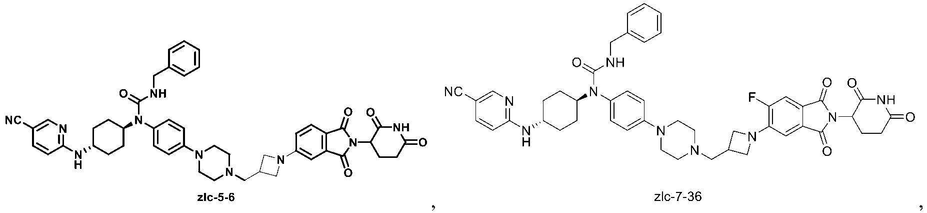

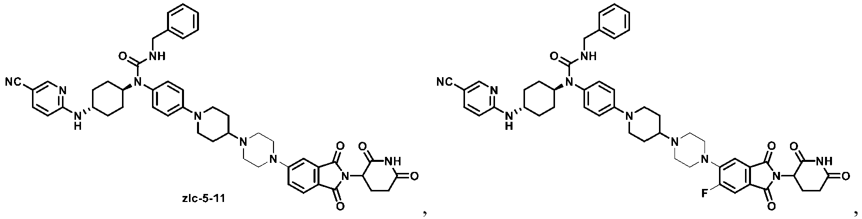

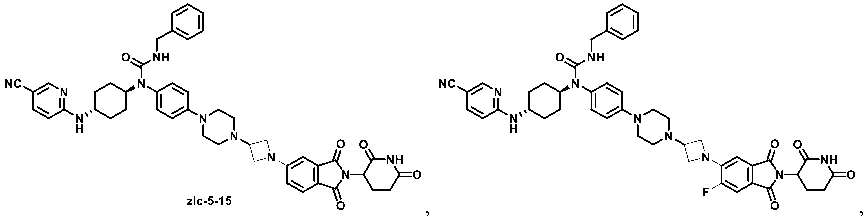

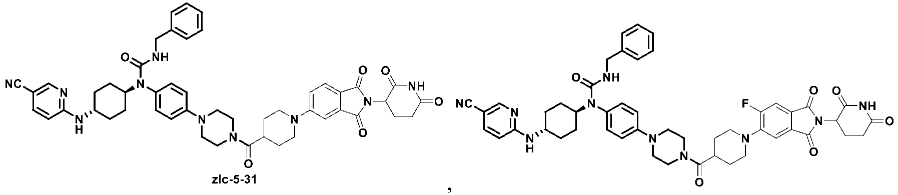

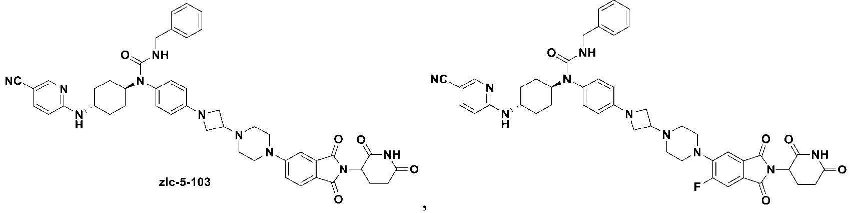

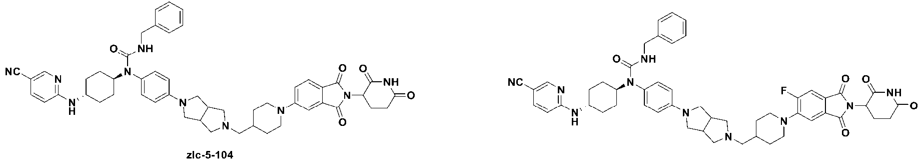

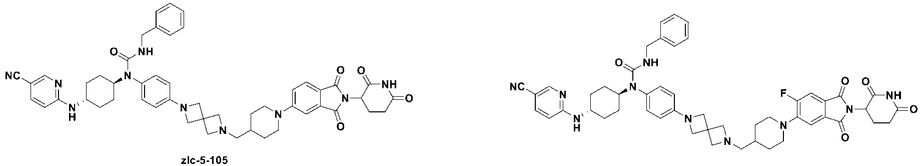

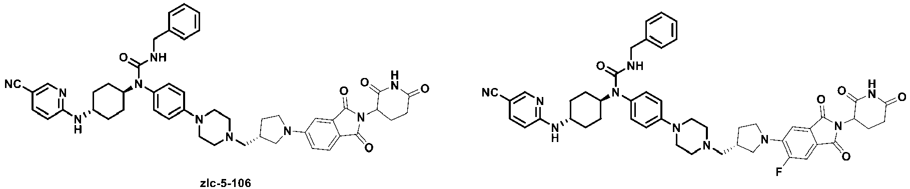

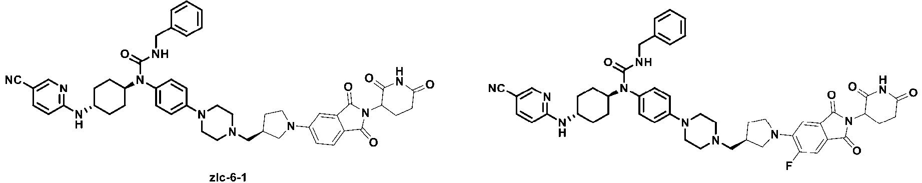

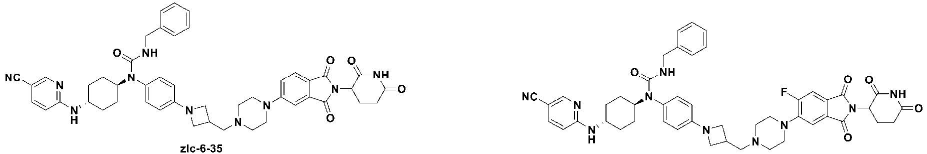

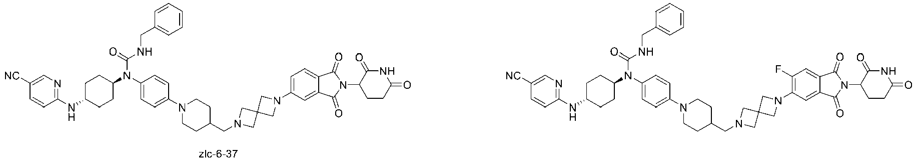

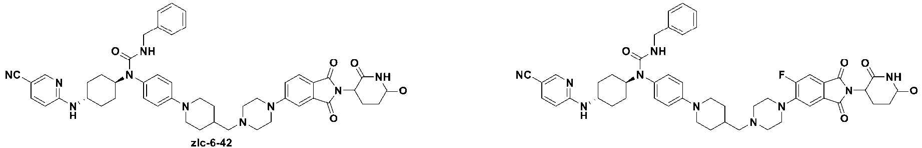

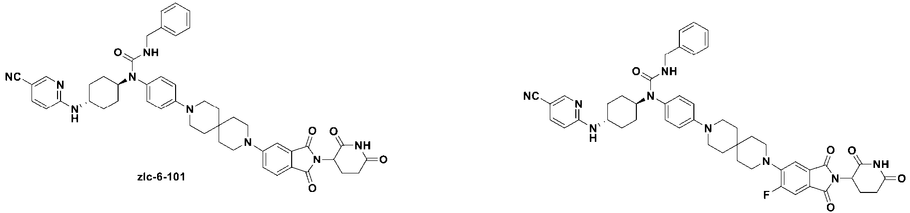

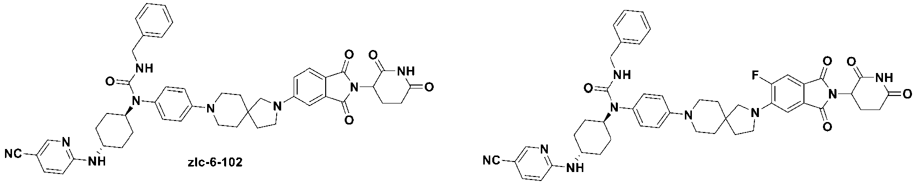

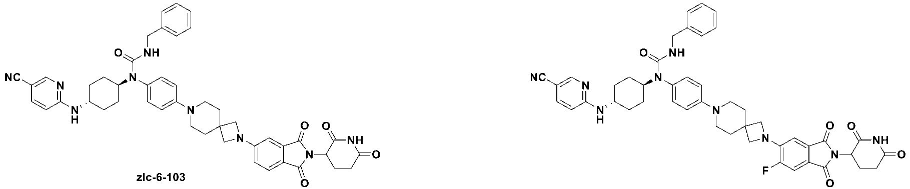

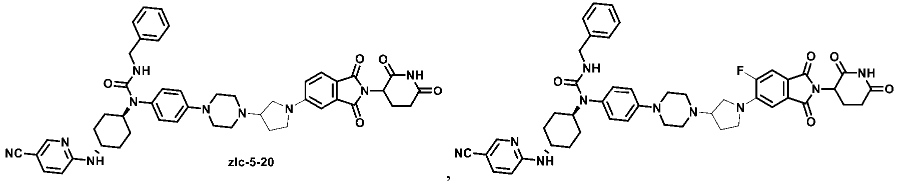

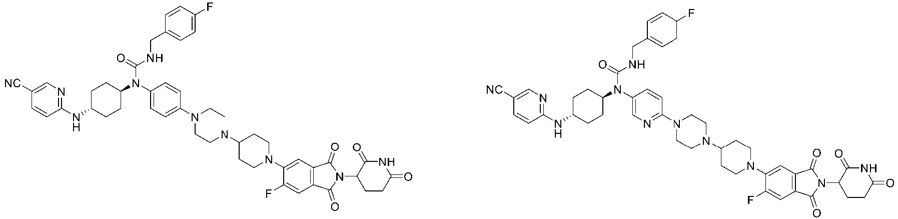

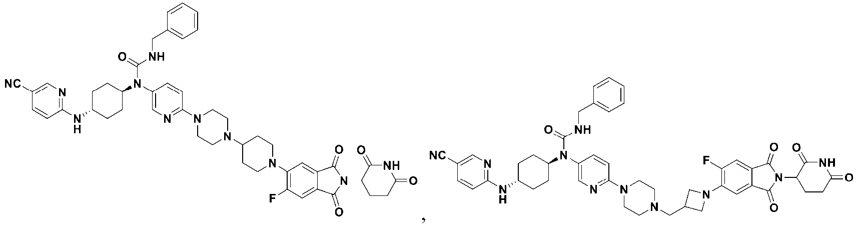

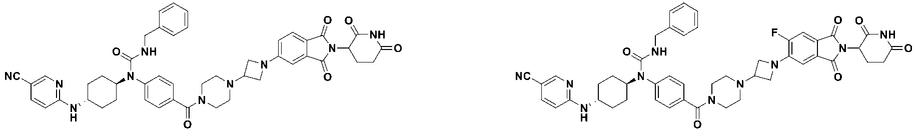

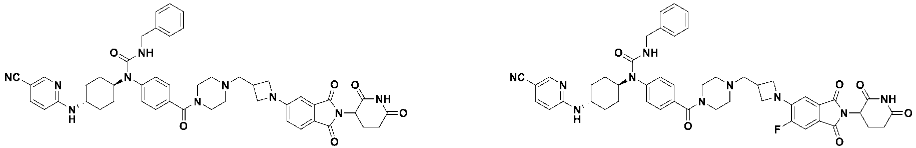

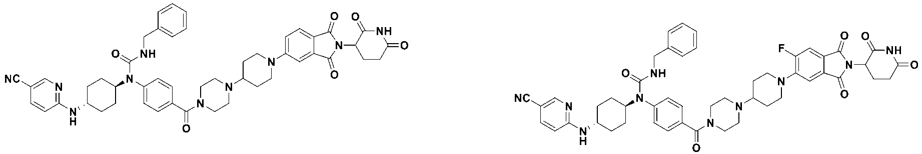

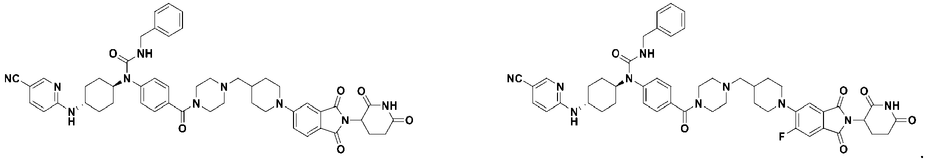

18. The compound according to claim 7, or a pharmaceutically acceptable salt thereof, or a stereoisomer thereof, or a prodrug molecule thereof, wherein the compound is selected from the group consisting of:

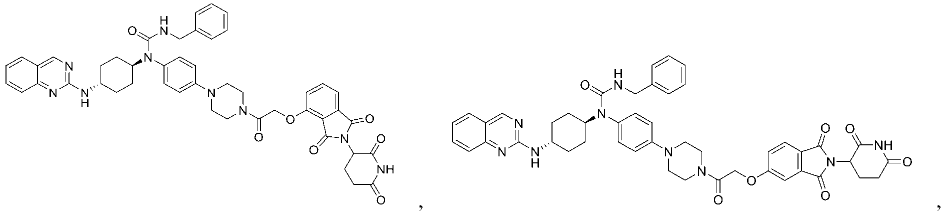

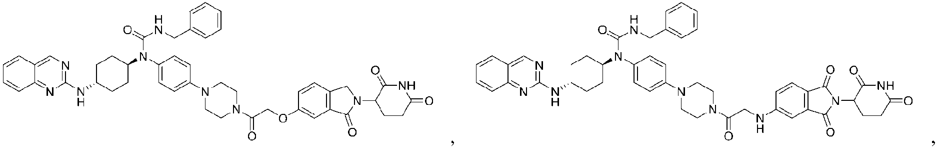

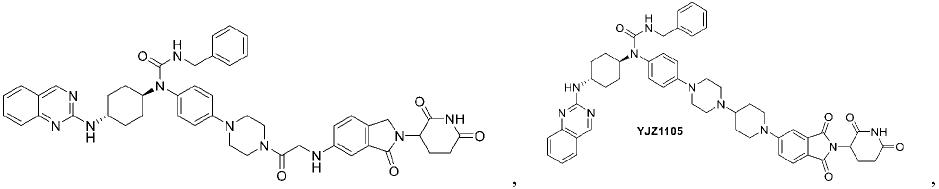

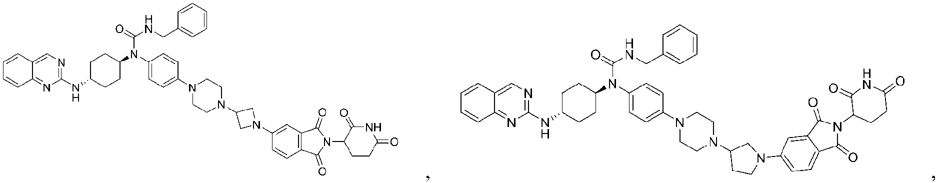

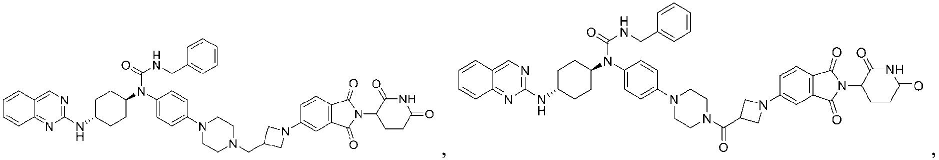

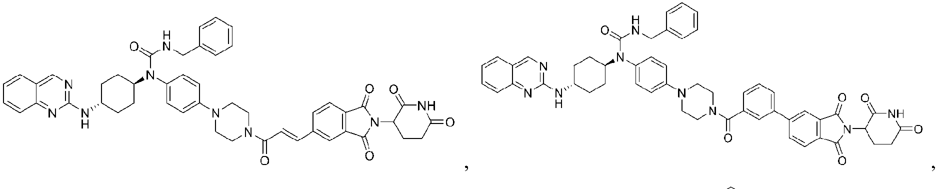

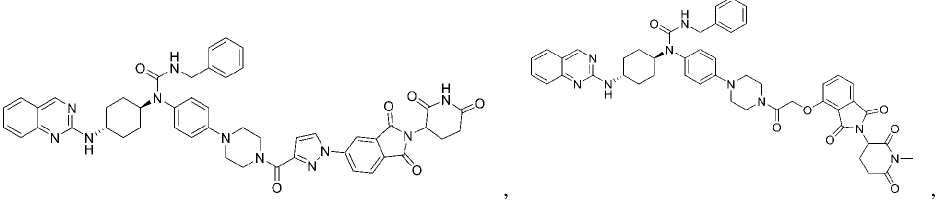

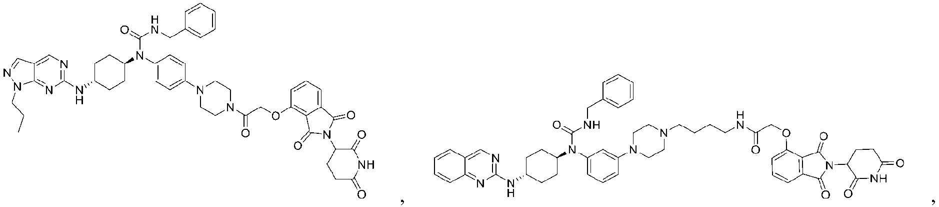

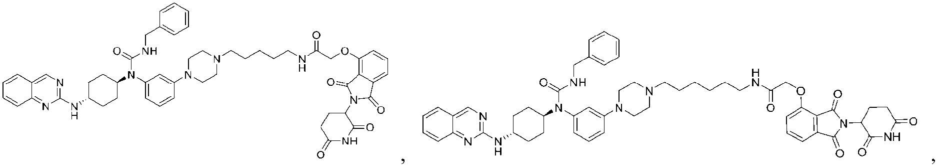

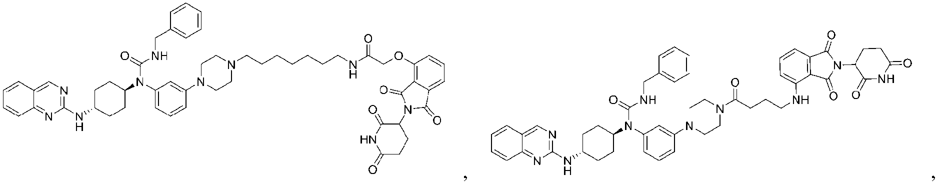

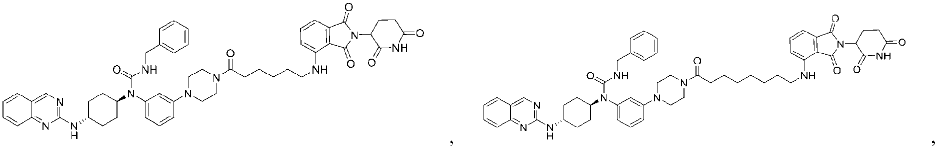

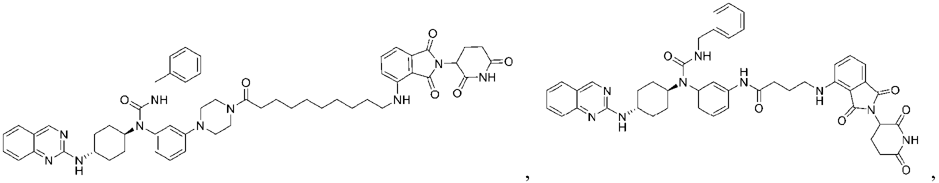

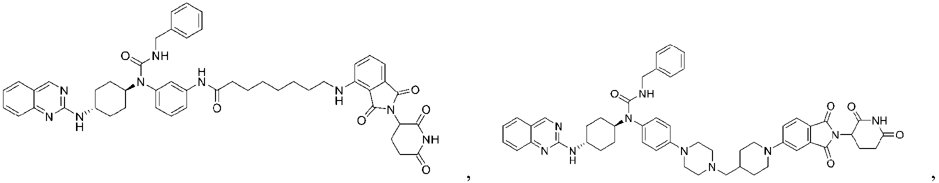

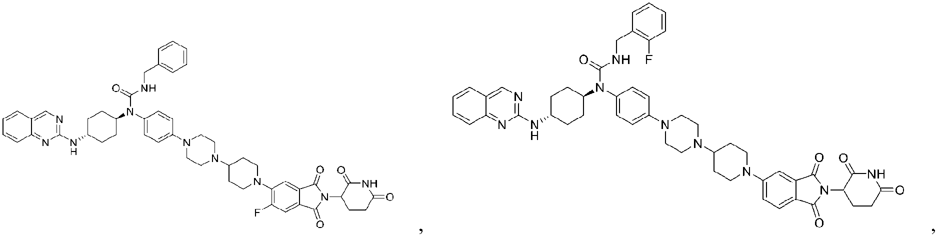

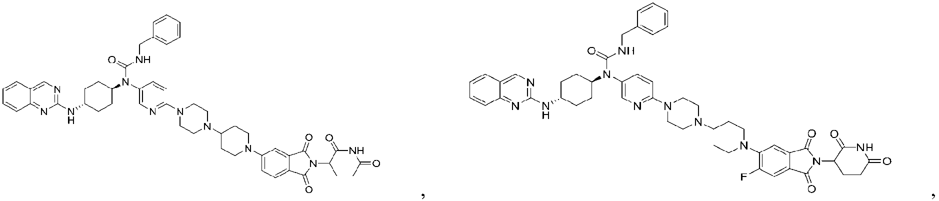

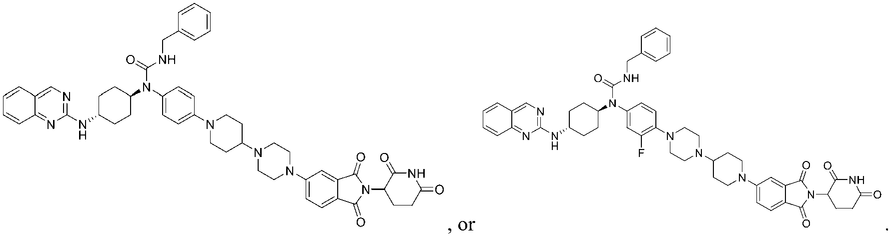

19. A pharmaceutical composition, comprising
(1) as an active ingredient, the compound of any one of claims 1-18, or a pharmaceutically acceptable salt, a stereoisomer thereof, or a prodrug molecule thereof; and
optionally (2) a pharmaceutically acceptable carrier.

20. Use of the compound according to any one of claims 1-18, or the pharmaceutically acceptable salt thereof, or the stereoisomer thereof, or the prodrug molecule thereof, or the pharmaceutical composition according to claim 19, for use in the preparation of a degradation agent for a CDK12/13 protein kinase.

21. Use of the compound according to any one of claims 1-18, or the pharmaceutically acceptable salt thereof, or the stereoisomer thereof, or the prodrug molecule thereof, or the pharmaceutical composition according to claim 19, for use in the preparation of a drug for preventing and/or treating a disease mediated by a CDK12/13 serine/threonine protein kinase.

22. The use according to claim 21, wherein the disease mediated by the CDK12/13 serine/threonine protein kinase is selected from the group consisting of: prostate cancer, breast cancer, uterine cancer, ovarian cancer, non-small cell lung cancer, small cell lung cancer, Ewing sarcoma, lung adenocarcinoma, squamous cell lung carcinoma, pancreatic cancer, liver cancer, skin cancer, epithelial cell carcinoma, gastrointestinal stromal tumor, leukemia, histiocytic lymphoma, nasopharyngeal carcinoma, head and neck tumor, colon cancer, rectal cancer, or glioma.
